# EUROPEAN PATENT SPECIFICATION

(11) **EP 2 279 417 B1**
(45) Date of publication and mention of the grant of the patent: **20.07.2016**
(21) Application number: 09763258.2
(22) Date of filing: 28.05.2009
(51) Int. Cl.: G01N 33/574, G06F 19/00

(54) **METABOLIC BIOMARKERS FOR OVARIAN CANCER AND METHODS OF USE THEREOF**
STOFFWECHSEL-BIOMARKER FÜR EIERSTOCKKREBS UND ANWENDUNGSVERFAHREN DAFÜR
BIOMARQUEURS MÉTABOLIQUES POUR LE CANCER DES OVAIRES ET PROCÉDÉS POUR LEUR UTILISATION

(30) Priority: 05.05.2009 US 175571 P; 28.05.2008 US 56618 P
(43) Date of publication of application: 02.02.2011
(73) Proprietor: Georgia Tech Research Corporation, Atlanta, GA 30332-0415 (US)
(72) Inventor: FERNANDEZ, Facundo, M., Atlanta, GA 30317-1442 (US); ZHOU, Manshui, Atlanta, GA 30309 (US); MCDONALD, John, Arnoldsville, GA 30619 (US); GRAY, Alexander, Atlanta, GA 30309 (US); GUAN, Wei, Foster City, CA 94404 (US)
(74) Representative: Walker, Ross Thomson
(86) International application number: PCT/US2009/045508
(87) International publication number: WO 2009/151967

(56) References cited:
- WO-A2-2005/083440
- WO-A2-2007/030928
- US-A1- 2002 009 740
- US-A1- 2007 065 827
- US-A1- 2007 172 902
- US-A1- 2007 221 835
- US-A1- 2008 064 055
- GUAN WEI ET AL: "Ovarian cancer detection from metabolomic liquid chromatography/mass spectrometry data by support vector machines", BMC BIOINFORMATICS, BIOMED CENTRAL, LONDON, GB, vol. 10, no. 1, 22 August 2009 (2009-08-22), page 259, XP021055704, ISSN: 1471-2105, DOI: 10.1186/1471-2105-10-259

## Description

### FIELD OF THE INVENTION

The present invention generally relates to a computer-implemented method and computer program for selecting a subject for treatment of ovarian cancer.

### BACKGROUND OF THE INVENTION

Epithelial ovarian cancer (EOC) is the eighth most common cancer and the fifth leading cause of cancer deaths in women in the United States. Despite decades of research and an annual investment in the U.S. of more than $2.2 billion (in 2004 dollars) on treatment, ovarian cancer remains the leading cause of deaths from gynecological malignancies (Brown, et al., Med. Care, 40(8 supplement) IV:104-117 (2002)). It is estimated that 21,650 new cases of ovarian cancer were diagnosed in 2008 and 15,520 women died from the disease (http://seer.cancer.gov/statfacts/html/ovary.html).

Most cancer blood tests in current clinical practice monitor changes in levels of a single molecule that has been demonstrated to be elevated (or lowered) in a significant number of diseased patients. While these tests are often not definitive *per se,* they can be of significant predictive value when combined with clinical symptoms and other diagnostic procedures. The challenge with ovarian cancer is that the disease typically arises and progresses initially without well-defined clinical symptoms (Jacobs and Menon, Mol. Cell Proteomics, 3:355-66 (2004)). Due to the asymptomatic nature of the disease, women are frequently undiagnosed until the disease is late in its progression (stage III/IV) when the 5-year survival rate is only 15-20% (Odunsi, et al., Int. J. Cancer, 113(5):782-8 (2005)).

This lack of early clinical symptoms places an elevated burden of accuracy on any potential blood test for ovarian cancer. So far, attempts to identify a single molecule with significant diagnostic potential for ovarian cancer have been uniformly unsuccessful. The assay for CA125 is currently the only FDA-approved test for ovarian cancer detection but the overall predictive value of CAl 25 has been reported to be less than 10% (Petricoin, et al., The Lancet, 359(9306):572-7 (2002)).

For this reason, current interest has focused on the development of tests using panels of biomarkers. For example, a recently developed test having a panel of six serum proteins has been shown to be of significant diagnostic value in high ovarian cancer risk groups (e.g., BRAC 1 positive patients) (Visintin, Clin. Cancer Res., 14:1065-72 (2008)) but not sufficiently accurate for diagnostic screening in the general population (Green, et al., CHn. Cancer Res., 14:7574-75 (2008)).

Efforts to discover potentially more accurate biomarkers of ovarian cancer using mass spectrometry have focused on large biopolymers, such as proteins (Williams, et at, J Proteome Res., 6:2936-62 (2007)). However, finding and validating biomarkers of this kind is hampered by the fact that the serum proteome is extremely complex, comprising -2 x 10 protein species with a dynamic range spanning 10 orders of magnitude (Anderson and Anderson, Mol. Cell Proteomics, 1:845-68 (2002)). This inherent complexity combined with current limitations in the proteomic analytical arsenal can result in the convolution of biomarker variability with non-biological sources of variance.

WO2007/030928 discloses metabolic markers for diagnosis of ovarian cancer.

### SUMMARY OF THE INVENTION

It is an object of the invention to provide methods for detecting changes in serum metabolites that are predictive of ovarian cancer.

In a first aspect of the present invention there is provided a computer-implemented method of selecting a subject for treatment of ovarian cancer comprising:
(i) inputting expression data of a panel of serum metabolic biomarkers in a serum sample obtained from the subject; and
(ii) determining whether expression of the metabolic biomarkers in the serum sample obtained from the subject is indicative of ovarian cancer using a computer system programmed with a trained machine learning classifier for distinguishing subjects with ovarian cancer and without ovarian cancer; wherein the machine learning classifier has been trained using expression data of a panel of serum metabolic biomarkers obtained from patients having ovarian cancer and from control subjects that do not have ovarian cancer; and
(iii) selecting the subject wherein the expression data of the panel of serum metabolic biomarkers in the serum sample obtained from the subject is correlated by the computer system to be indicative of ovarian cancer, and wherein the diagnostic accuracy is at least 80%, and wherein the panel of serum metabolic biomarkers comprises each of D-1-Piperideine-2-carboxylic acid, 2-Phenylacetamide, D-Glyceraldehyde 3-phosphate, 5-Methoxytryptophan, N-(2-hydroxyethyl) icosanamide, Isopentenyladenine-9-N-glucoside, Asp-Val-Thr, LysoSM(d18:0) and His-Tyr-Arg.

In a further aspect of the present invention there is provided a computer program adapted to perform a method according to claim 1 when executed on computer comprising:
(i) a means adapted for receiving expression data of the metabolic biomarkers recited in claim 1;
(ii) a module adapted for performing the determination recited in claim 1;
(iii) a means for indicating whether the subject is selected as recited in claim 1.

### SUMMARY OF THE DISCLOSURE

The term "embodiment" as used throughout the description refers to embodiments of the disclosure. Only those embodiments falling under the scope of the claims constitute embodiments of the invention. Methods and compositions for detecting changes in serum metabolites that correlate with cancer are provided. Panels of serum metabolites have been identified that can be used to diagnose cancer or assess the risk of developing cancer. A preferred cancer is ovarian cancer. The metabolic biomarkers include serum metabolites that are differentially present in the serum of subjects with or at risk of developing cancer as compared to the serum of control subjects that do not have cancer. The serum metabolic biomarkers preferably include serum metabolites that are differentially present in the serum of patients with gynecologic cancers, as compared to the serum of control subjects.

In certain embodiments, profiles of serum metabolites are obtained from subjects with cancer and subjects without cancer. Profiles of statistically significant serum metabolites indicative or predicative of cancer are obtained by comparing the serum metabolite profiles of the two populations. Once the profile of serum metabolites indicative of cancer is obtained, a serum metabolite profile from a sample from a subject can be obtained and compared to the predetermined profile of serum metabolites indicative of cancer. If the profile obtained test sample correlates with the profile indicative of cancer, the subject is diagnosed with cancer.

The disclosed panels of serum metabolic biomarkers include at least 2 or more serum metabolites. In some embodiments, the metabolic biomarker panels include 2, 3, 4, 5, 6, 7, 8, 9, 10, 15, 20, 25, 50, 75, 100, 150, or more metabolites. In preferred embodiments, the metabolic biomarker panels include 10 or more metabolites. Serum metabolic biomarkers may be characterized by their molecular weight, their chemical formula, their mass-to-charge ratio (*m*/*z*), for example as determined by mass spectrometry, or their chemical name.

Methods for using the metabolic biomarker panels to identify a subject for treatment of cancer are provided. The methods generally include the steps of detecting two or more metabolic biomarkers in the serum of a test subject, comparing the levels of the two or more metabolic biomarkers with the levels of the metabolic biomarkers detected in a group of subjects without cancer and to the levels of the metabolic markers detected in a group of cancer patients, and determining whether the levels of the metabolic biomarkers in the test subject are indicative of the presence of cancer.

Metabolic biomarkers can be detected by any suitable method, including, but not limited to, mass spectrometry methods such as liquid chromatography time-of-flight mass spectrometry (LC-TOF MS) and direct analysis in real time time-of-flight mass spectrometry (DART-TOF MS). Serum metabolites can also be detected using specific binding assays, such as an ELISA assay.

In some embodiments, the methods for using the metabolic biomarker panels to identify a subject for treatment of cancer are computer-implemented methods. Supervised classification methods are preferably used to determine whether the levels of metabolic biomarkers in the test subject are indicative or predictive of cancer. Supervised classification methods include, but are not limited to, partial least squares-discriminant analysis (PLSDA), soft independent modeling of class analogy (SIMCA), artificial neural networks (ANNs), classification and regression trees (CART), and machine learning classifiers, such as the single layer perceptron (SLP), the multi-layer perceptron (MLP), decision trees and support vector machines (SVMs). Preferably the classifier is a SVM.

Machine learning classifiers can be trained to discriminate between the expression data of patients with cancer and the expression data of control subjects without cancer by inputting expression data from these two groups. Trained machine learning classifiers can then be used to classify a sample as a cancer sample or a non-cancer sample by classifying expression data from the sample. Trained classifier may optionally be tested using expression data from subjects that are known to have cancer and from subjects that do not have cancer to determine the sensitivity, specificity, and/or accuracy of the trained machine learning classifier. Trained machine learning classifiers preferably allow a diagnosis of cancer with an accuracy, a specificity, and/or a sensitivity of at least 80%, 85%, 90%, 95%, 96%, 97%, 98% or 99%.

In some embodiments, the number of variables (or features) in the expression dataset can be reduced to improve classification by machine learning classifiers. Suitable feature selection methods include, but are not limited to, recursive genetic algorithm (GA), recursive feature elimination (RFE), ANOVA feature selection, and simple sub-sampling. Additionally, SVMs such as L1SVM and SVMRW, which are described below, can simultaneously perform classification as well as feature selection.

Systems for selecting subjects for treatment of cancer are also provided. In one embodiment, the system includes (i) a means for receiving expression data of two or more serum metabolic biomarkers in a sample from a subject, and; (ii) a module for determining whether the data is indicative of cancer or an increased risk for developing cancer. The module can be a trained machine learning classifier capable of distinguishing data from a cancer patient and data from a control subject. The module for determining whether the data is indicative of the presence of cancer can include a machine learning classifier which has been trained to distinguish expression data characteristic of a cancer patient from expression data characteristic of a control subject.

Kits for use in the diagnosis of cancer are also provided. The kit can include means for detecting two or more of the disclosed metabolic biomarkers. The means of detection can include a capture surface, such as an array of specific binding reagents such as antibodies or antibody fragments. The kit can include one or more samples of one or more of the disclosed metabolic biomarkers in a container. The metabolic biomarkers provided in the kit can be used as a control or for calibration.

### BRIEF DESCRIPTION OF THE DRAWINGS

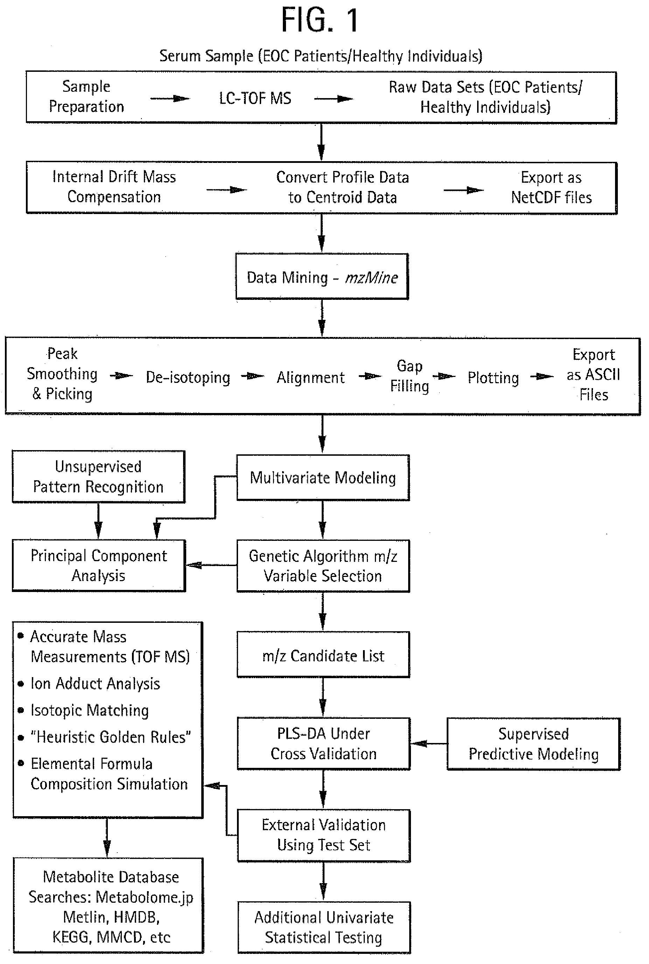

**Figure 1** is a schematic showing the metabolomic workflow followed for ovarian cancer biomarker discovery in Example 1.

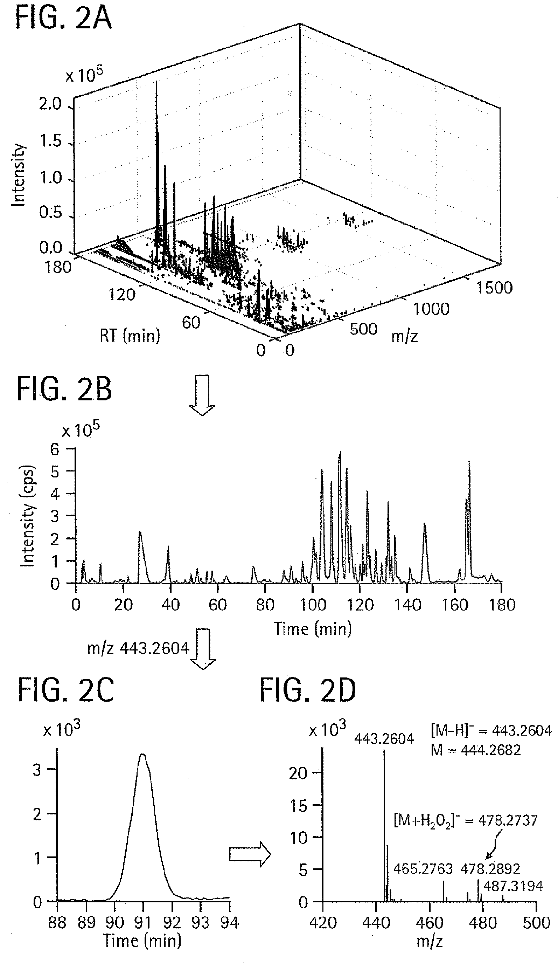

**Figure 2A** is a 3-D mass spectrometry profile of serum metabolites from a typical stage III ovarian cancer serum sample demonstrating the capability of liquid chromatography electrospray ionization time-of-flight mass spectrometry (LC/TOF MS) to resolve hundreds of compounds in a wide mass range within 180 minutes. **Figure 2B** is a total ion chromatogram (TIC) of the data in Figure 2A. Data are plotted as intensity (cps) as a function of retention time (minutes). **Figure 2C** is a selected monoisotopic ion chromatogram for an ion with *m*/*z* 443.26 at a window width of 0.05 Da. **Figure 2D** is the corresponding centroided negative ion mode mass spectrum obtained at a retention time (RT) of 91 minutes.

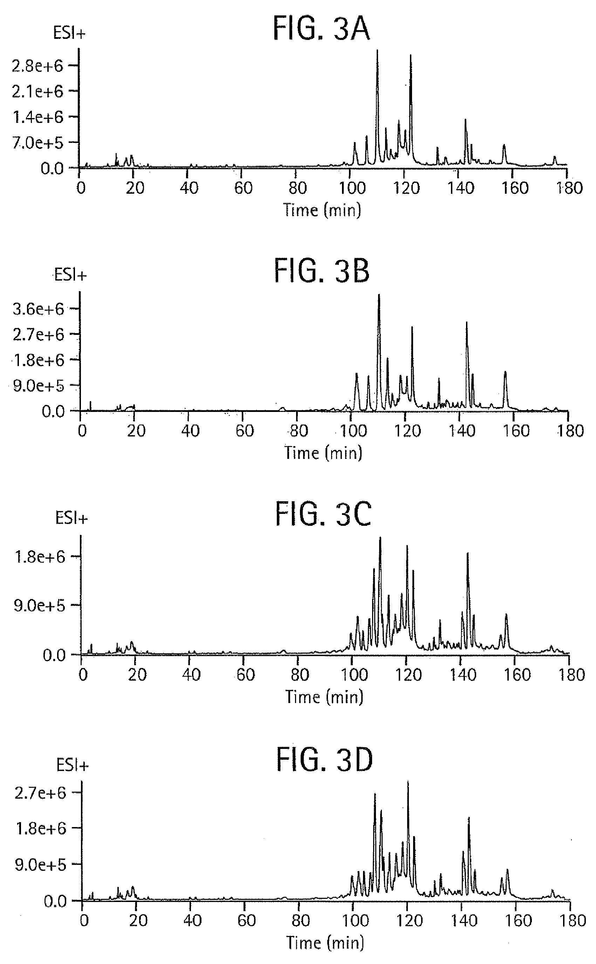

**Figures 3A, 3B, 3C** and **3D** are total ion chromatograms of 4 identical samples prepared in an identical fashion and analyzed by positive ion mode ESI demonstrating good reproducibility at the flow rate of 300 µl min⁻¹.

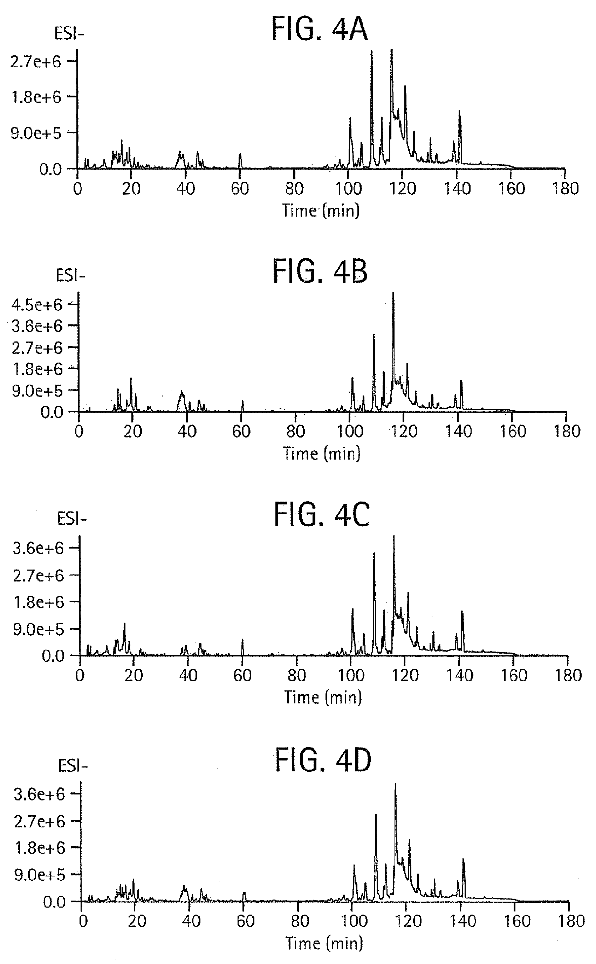

**Figures 4A, 4B, 4C** and **4D** are total ion chromatograms of 4 identical samples prepared in an identical fashion and analyzed by negative ion mode ESI demonstrating good reproducibility at the flow rate of 300 µl min⁻¹.

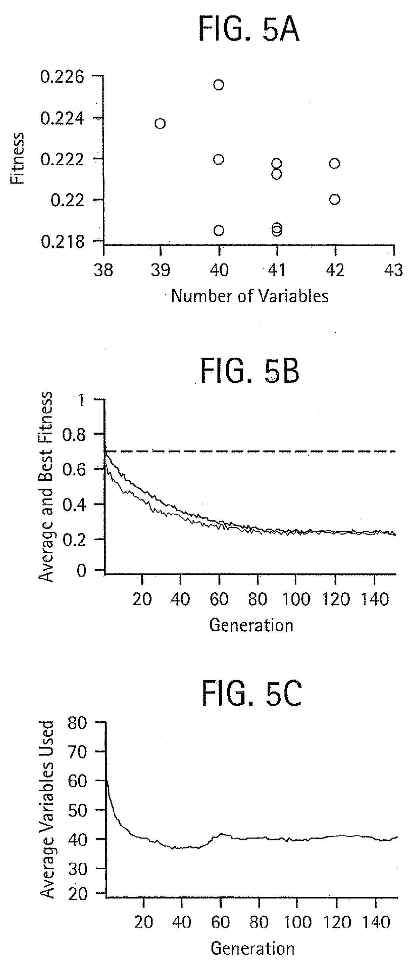

**Figure 5A** is a plot of the fitness observed for a final pool of "chromosomes" selected after 150 generations of a genetic algorithm (GA)-based evolutionary variable selection strategy on multimode ionization data. **Figure 5B** is a line graph showing the improvement in classification accuracy (also termed as the "fitness of the variable subset") as a function of the number of generations of the genetic algorithm. **Figure 5C** shows the evolution in the number of variables used during the genetic algorithm selection process.

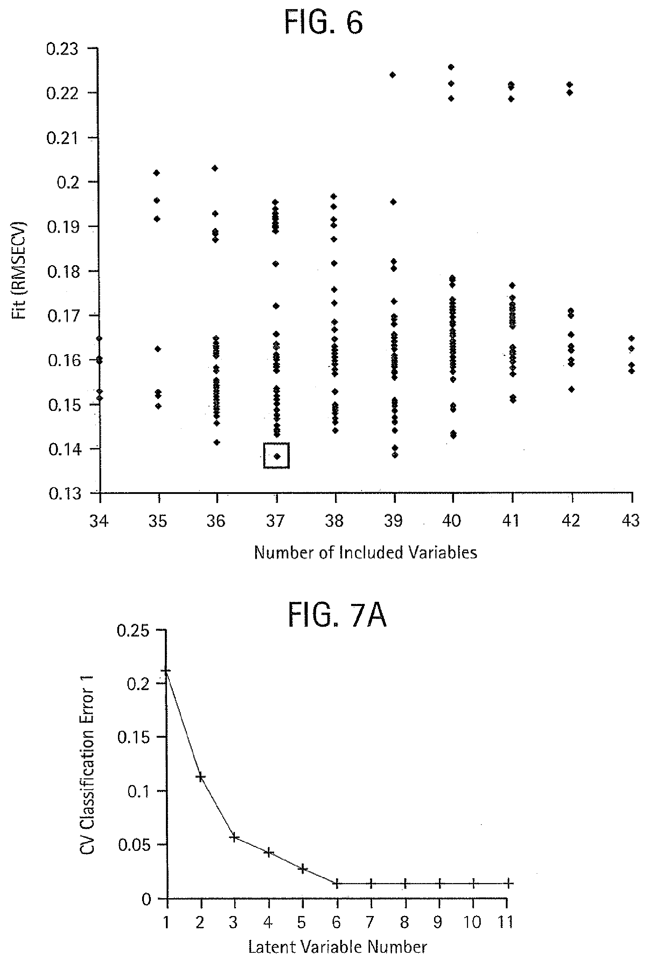

**Figure 6** is a plot showing the fitness of a pool of "chromosomes" resulting from 10 GA iterations of 150 generations each on multimode ionization data.

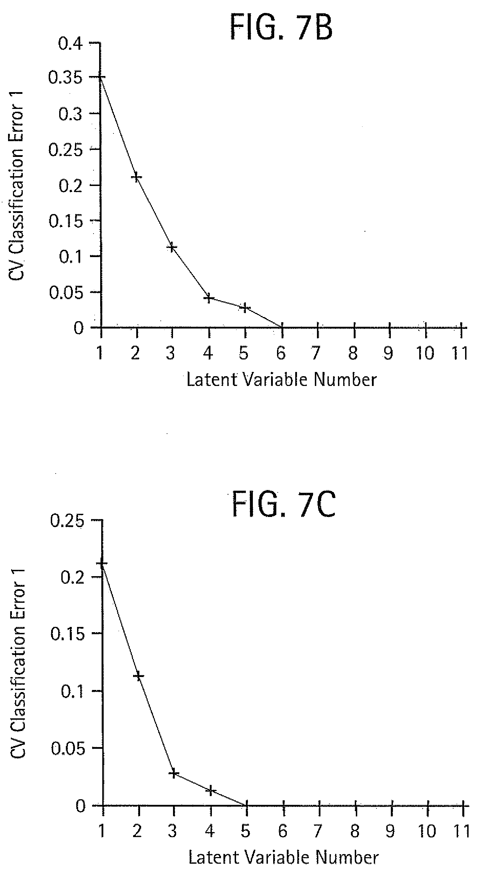

**Figure 7A** is a line graph showing the change in crossvalidation classification error as a function of the number of latent variables used in the construction of partial least squares-discriminant analysis (PLSDA) models using positive ion mode data. **Figure 7B** corresponds to negative ion mode data and **Figure 7C** to combined positive and negative (multimode) data.

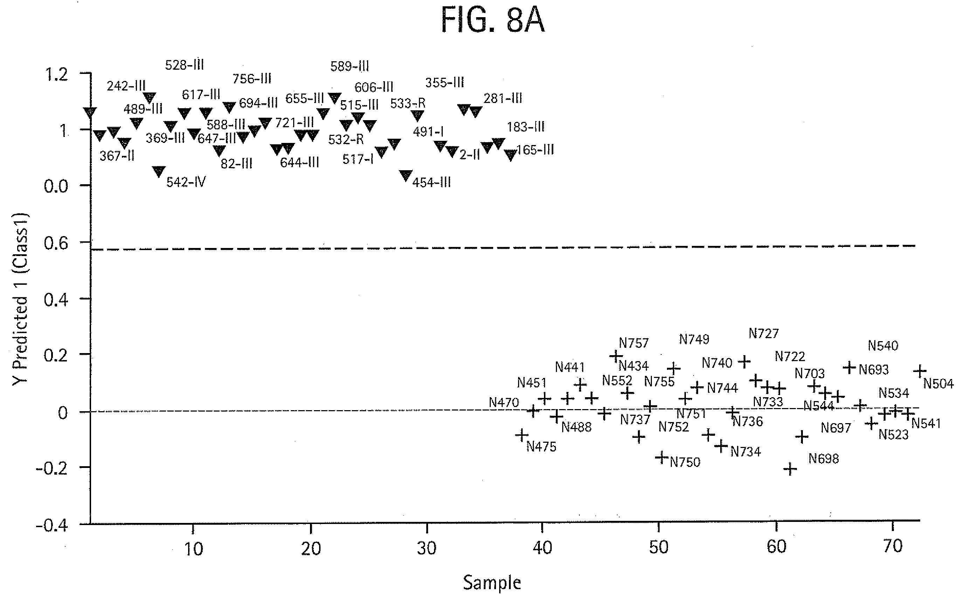

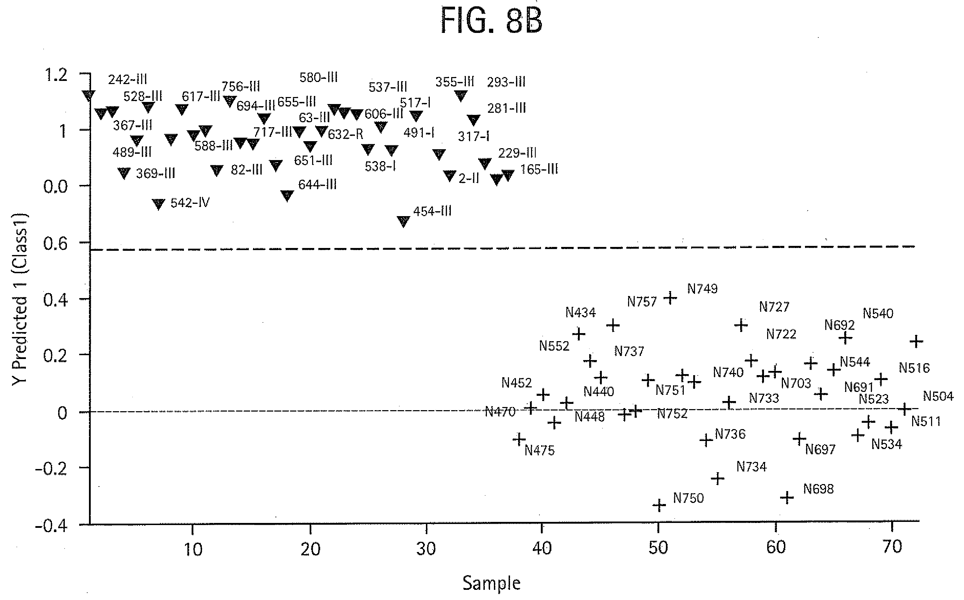

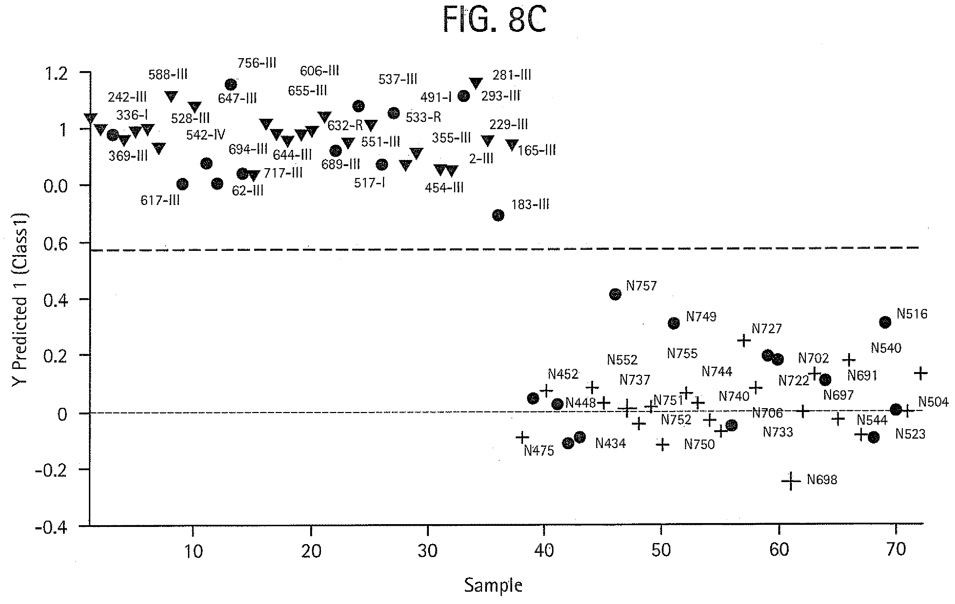

**Figures 8A-8C** are PLSDA plots of predicted Y block class membership values for all serum samples using GA-selected multimode ionization LC/TOF MS data. **Figure 8A** shows predicted Y values during the calibration stage, **Figure 8B** shows predicted Y values during Venetian-blinds crossvalidation. Figure **8C** shows external validation using 24 samples as an unknown test set. The red dashed line in each graph represents the decision threshold.

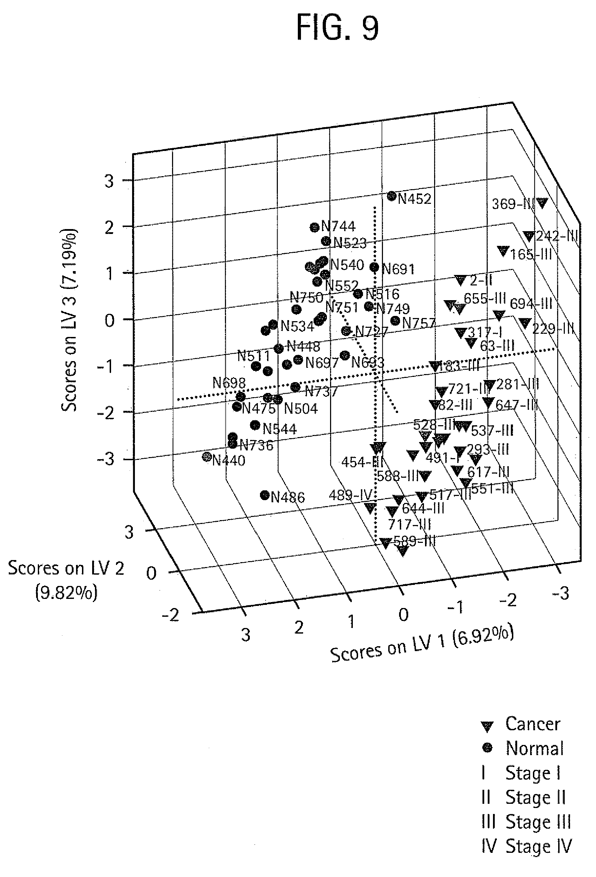

**Figure 9** is a PLSDA score plot of the first three latent variables for all serum samples in different cancer stages after GA.

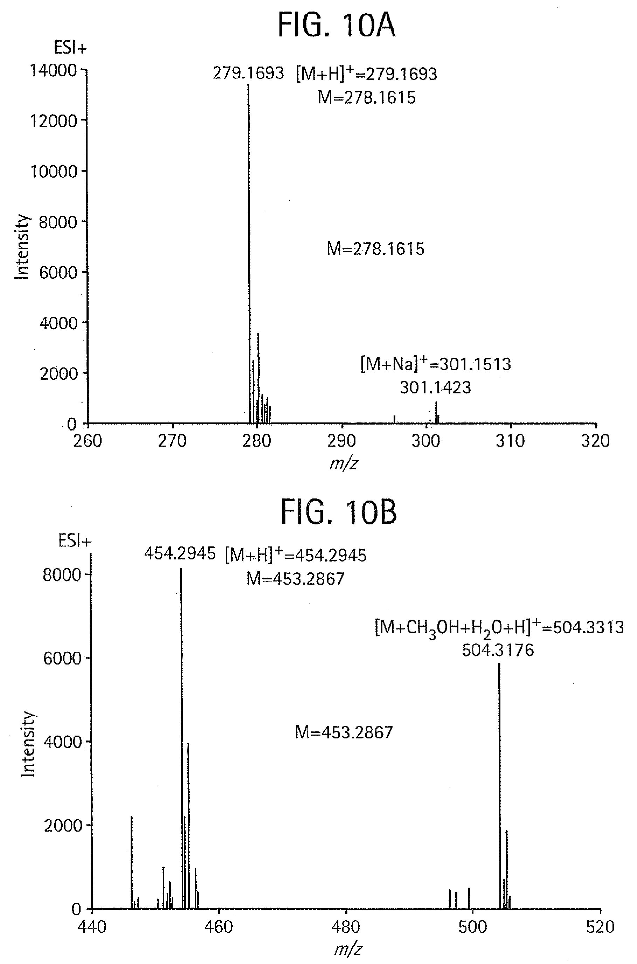

**Figure 10A** through **Figure 10O** are centroided mass spectra corresponding to all annotated variables from Tables 6 and 7.

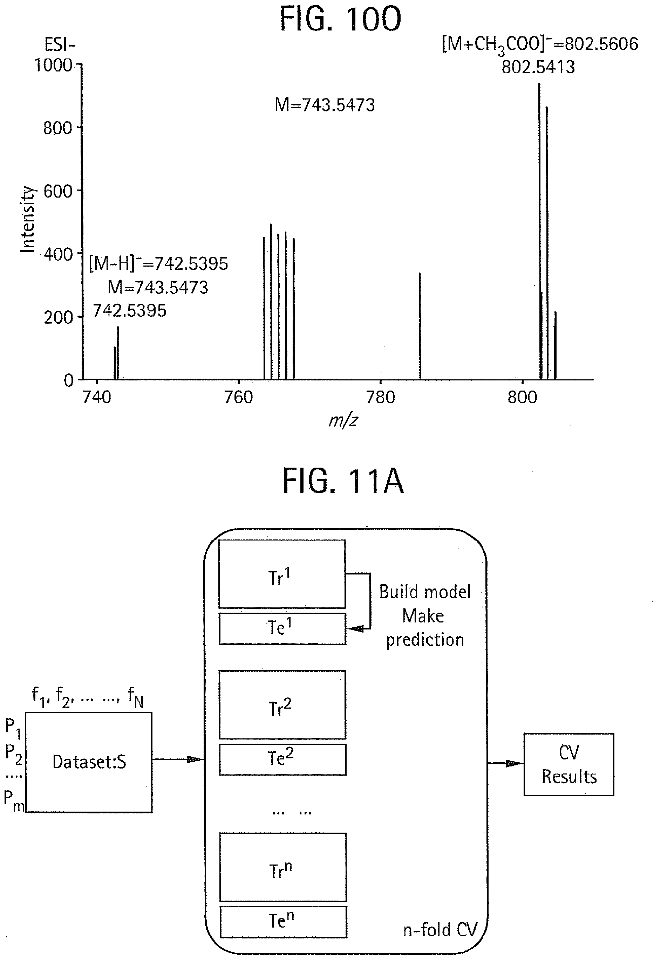

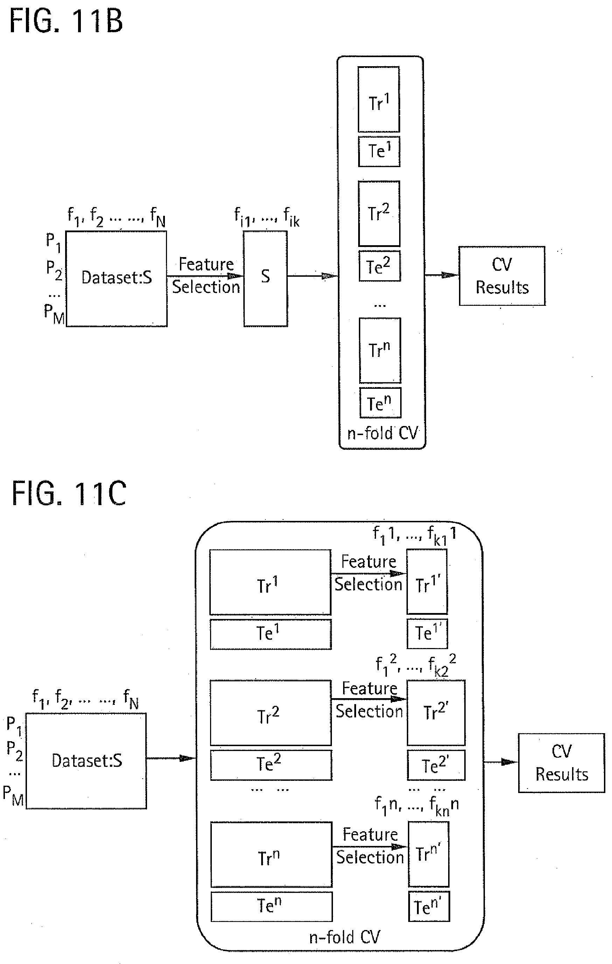

**Figure 11****.A** is a schematic showing a prediction performance evaluation framework without feature selection for mass spectrometry datasets. **Figure 11B** is a schematic showing a prediction performance evaluation framework applying feature selection to the whole dataset. **Figure 11C** is a schematic showing a prediction performance evaluation framework applying feature selection to training subsampling of dataset during each cross-validation.

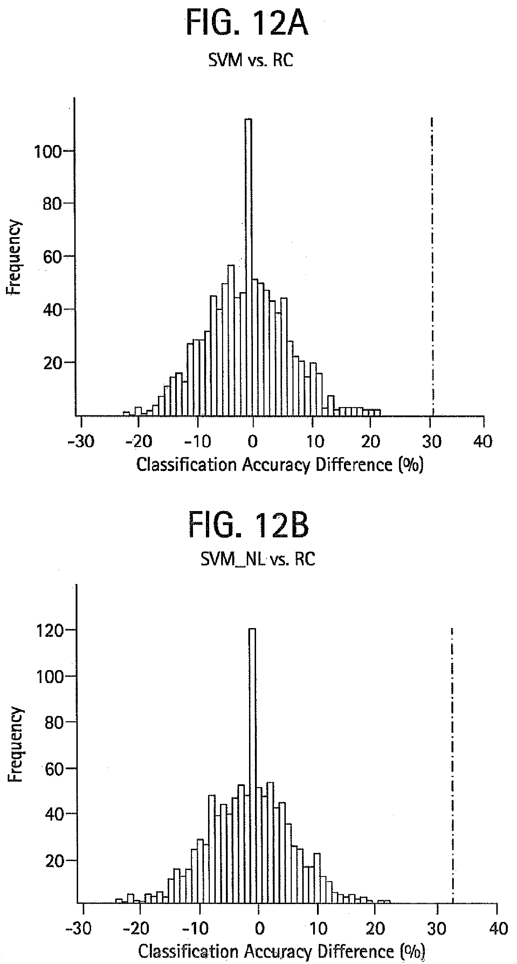

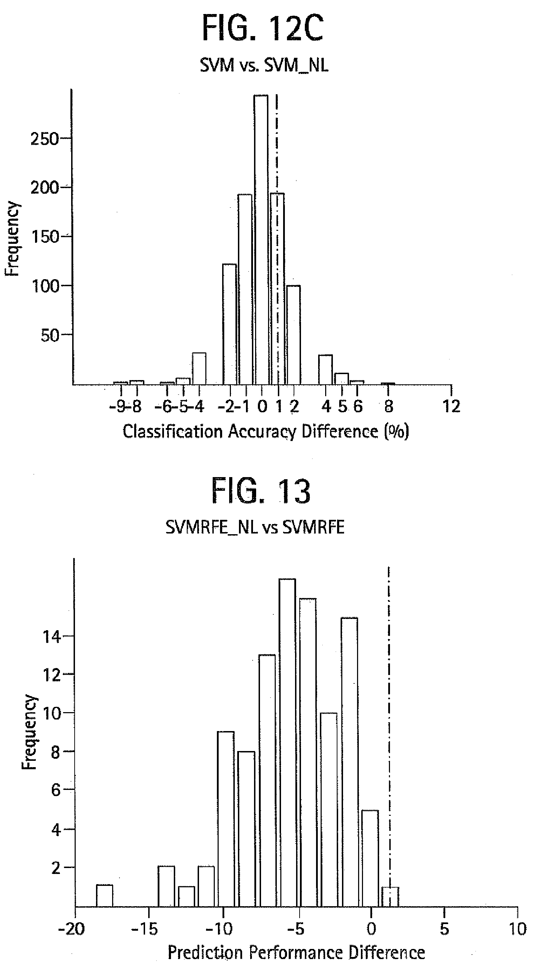

**Figure 12A** is a graph showing a comparison of classification accuracy for a linear support vector machine (SVM) classifier versus a random classifier (RC) for a multimode LC/TOF MS dataset. **Figure 12B** is a graph showing a comparison of classification accuracy for a nonlinear SVM classifier with degree 2 polynomial kernel (SVM_NL) versus RC for a multimode LC/TOF MS dataset. **Figure 12C** is a graph showing a comparison of classification accuracy for SVM versus SVM_NL for a multimode LC/TOF MS dataset. For each graph, the x-axis is the classification accuracy difference, and the y-axis is the frequency of the given classification accuracy difference. The dotted line in each graph represents the classification accuracy difference.
**Figure 13** is a graph showing a comparison of the prediction performance for feature selection results of recursive feature elimination (RFE) feature selection with nonlinear SVM (SVMRFE_NL) versus RFE feature selection with linear SVM (SVMRFE).

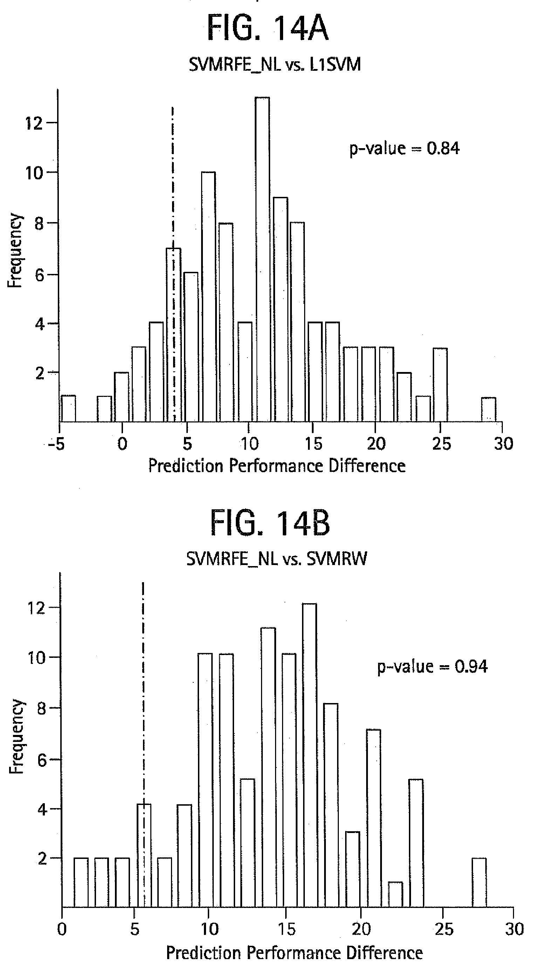

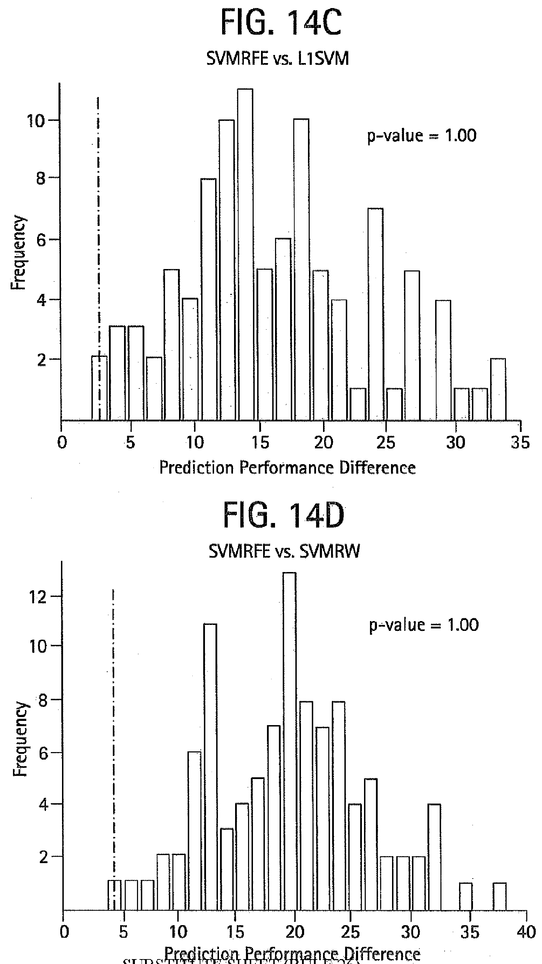

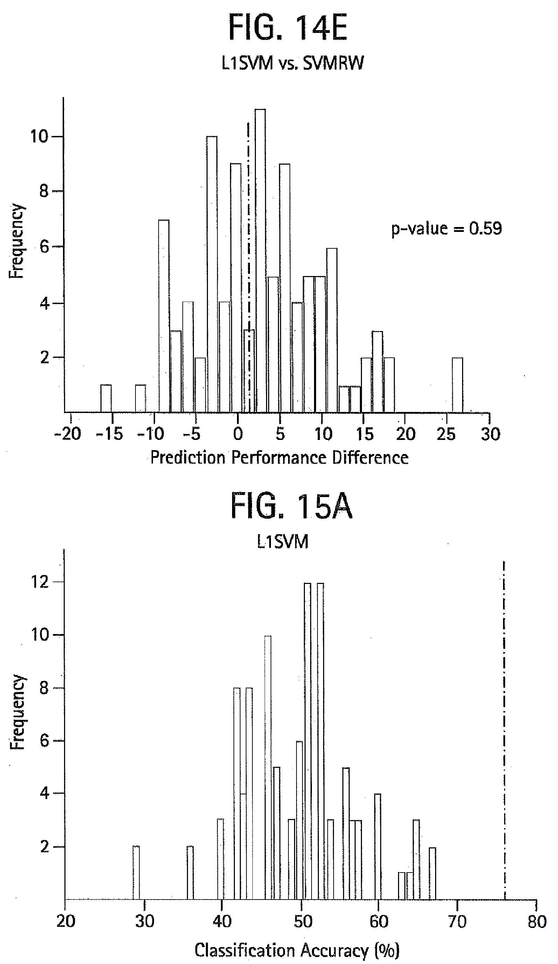

**Figure 14A** is a graph showing a comparison of the prediction performance for feature selection results of SVMRFE_NL versus L1SVM. **Figure 14B** is a graph showing a comparison of the prediction performance for feature selection results of SVMRFE_NL versus Weston's feature selection method with nonlinear SVM (SVMRW). **Figure 14C** is a graph showing a comparison of the prediction performance for feature selection results of SVMRFE versus L1SVM. **Figure 14D** is a graph showing a comparison of the prediction performance for feature selection results of SVMRFE versus SVMRW. **Figure 14E** is a graph showing a comparison of the prediction performance for feature selection results of L1SVM versus SVMRW.

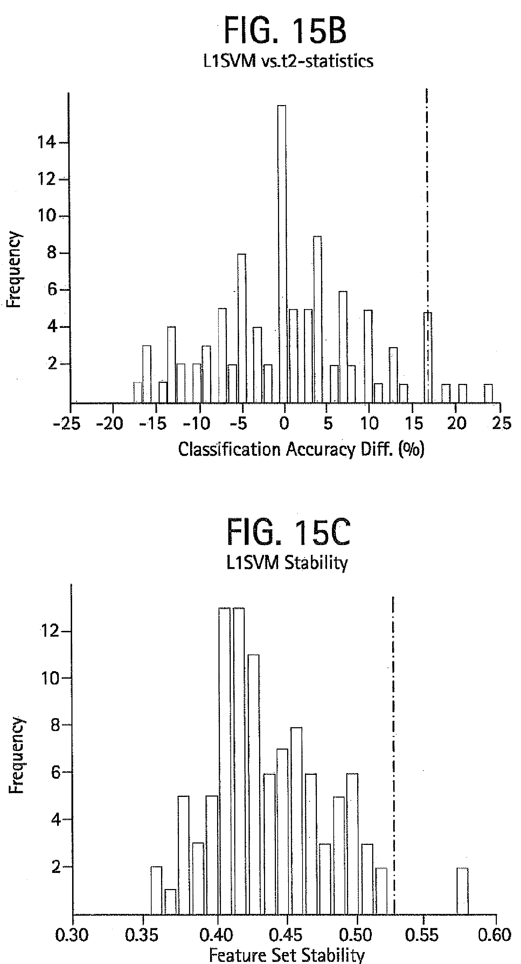

**Figure 15A** is a graph showing the prediction Performance of L1SVM. **Figure 15B** is a graph showing performance difference of L1SVM and t2-statistics. **Figure 15C** is a graph showing the stability of stability of L1SVM.

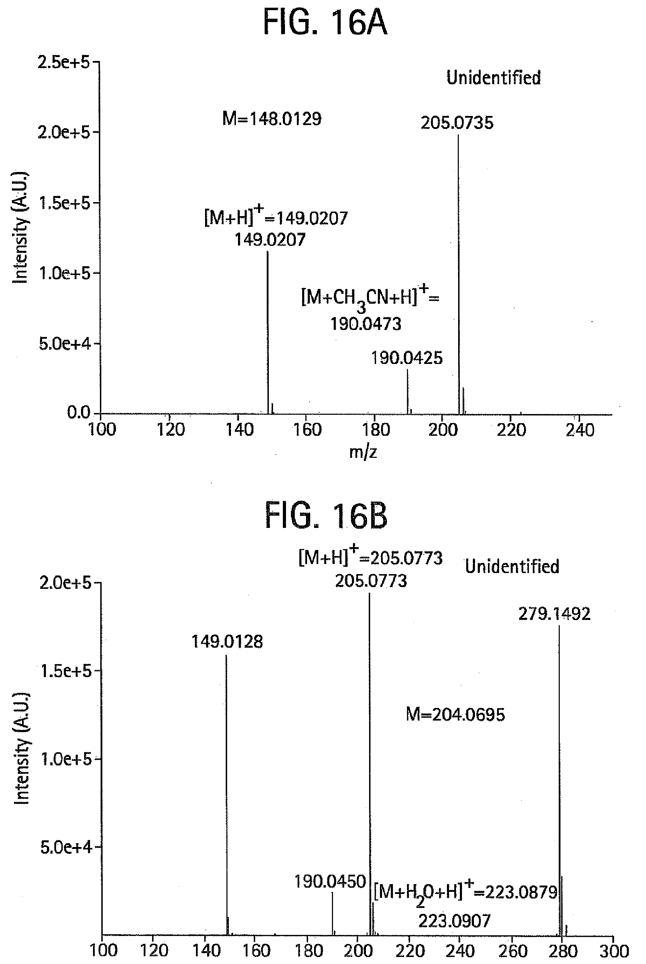

**Figure 16A** through **Figure 16I** are centroided mass spectra corresponding to all variables from Table 18.

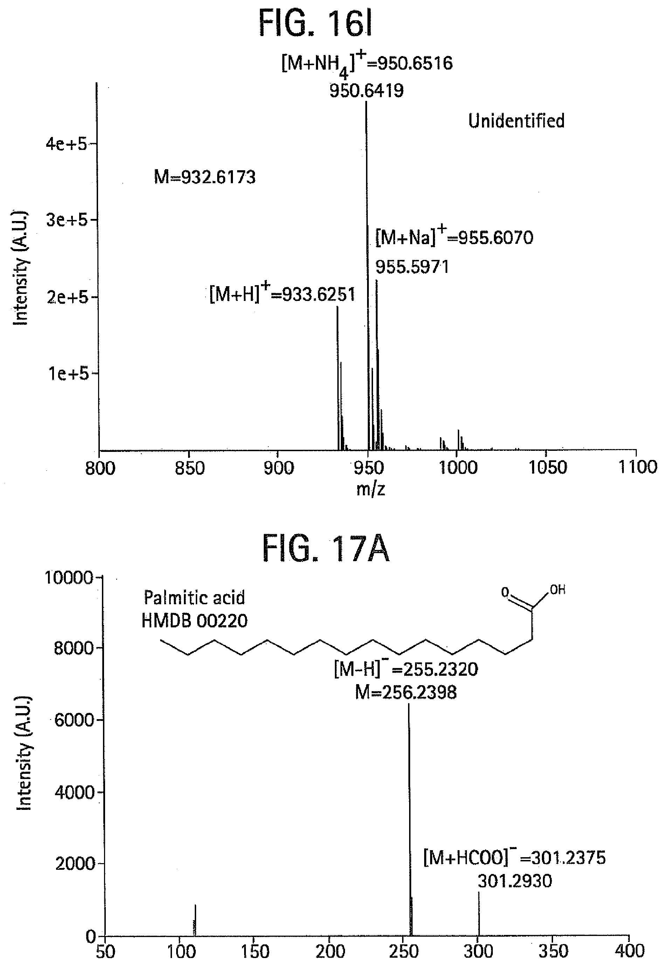

**Figure 17A** through **Figure 17T** are centroided mass spectra corresponding to all variables from Table 19.

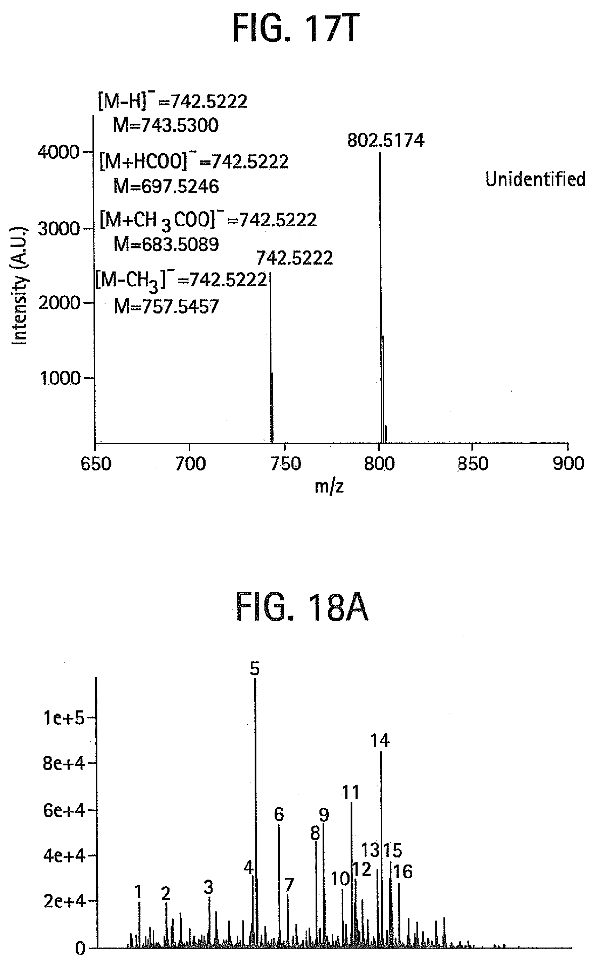

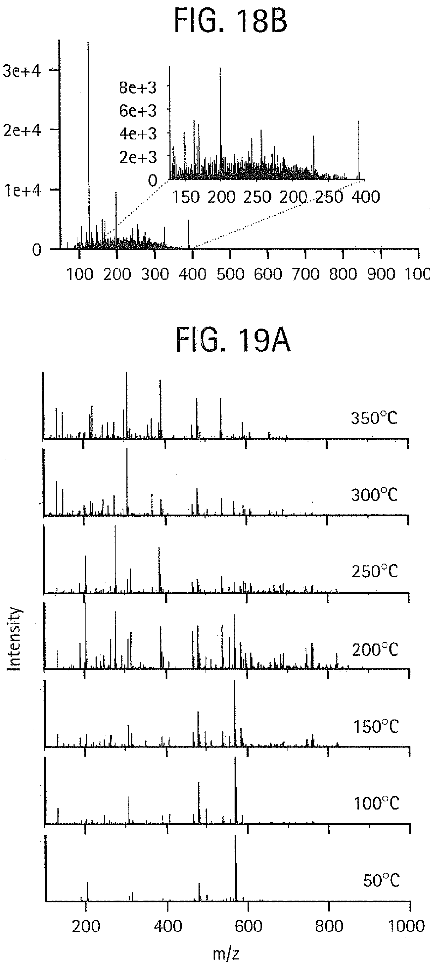

**Figure 18A** is direct analysis in real time (DART) coupled with TOF (DART-TOF) mass spectrum of a sample of healthy human serum derivatized with MSTFA/TMCS. **Figure 18B** is a DART-TOF mass spectrum of an underivatized sample of healthy human serum.

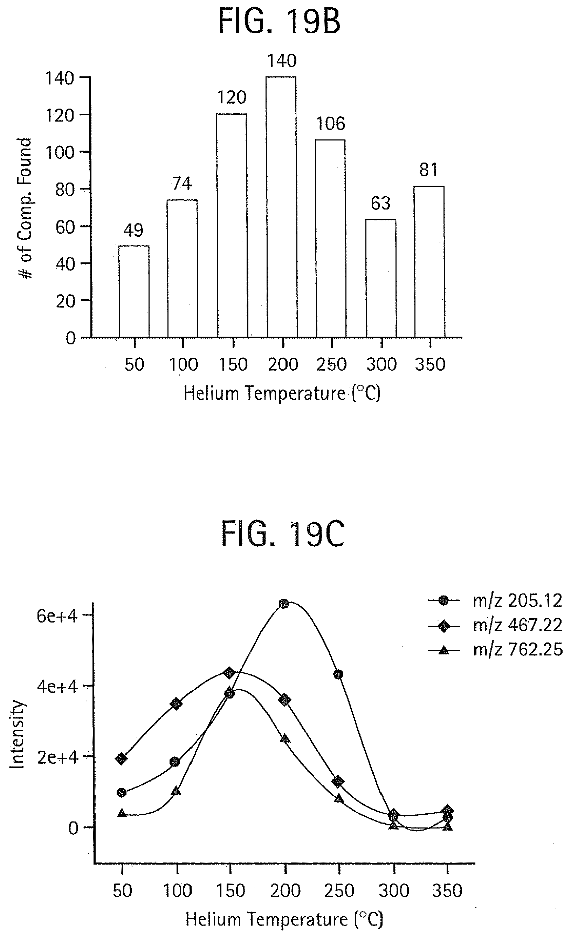

**Figure 19A** is a series of mass spectra of derivatized healthy human serum showing the effect of various helium gas temperatures on DART-TOF MS sensitivity. **Figure 19B** is a bar graph showing the number of metabolites matched to HMDB database for each mass spectrum from Figure 19A. **Figure 19C** is a line graph showing the change in the signal to noise ratio (six) of three mass spectrometric signals at *m*/*z* 205.12, 467.22 and 762.25 as a function of helium temperature.

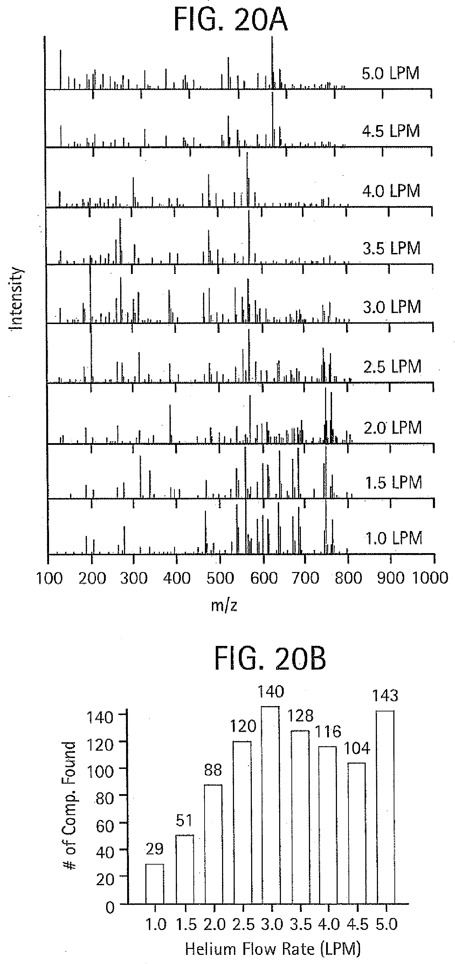

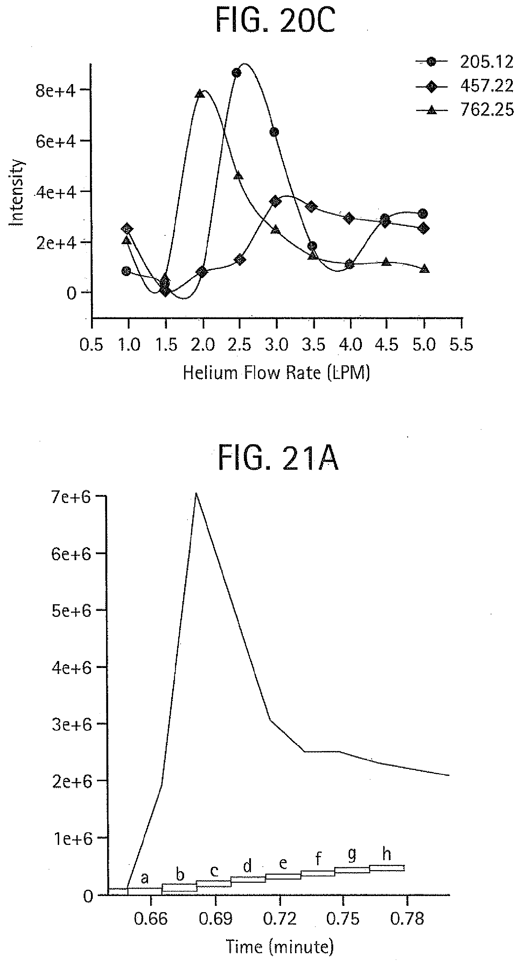

**Figure 20A** is a series of mass spectra of derivatized healthy human serum showing the effect of various helium flow rates on DART-TOF MS sensitivity. **Figure 20B** is a bar graph showing the number of metabolites matched to HMDB database for each mass spectrum from Figure 20A. **Figure 20C** is a line graph showing the change in the signal to noise ratio (S/N) of three mass spectrometric signals at *m*/*z* 205.12,467.22 and 762.25 as a function of helium flow rate.

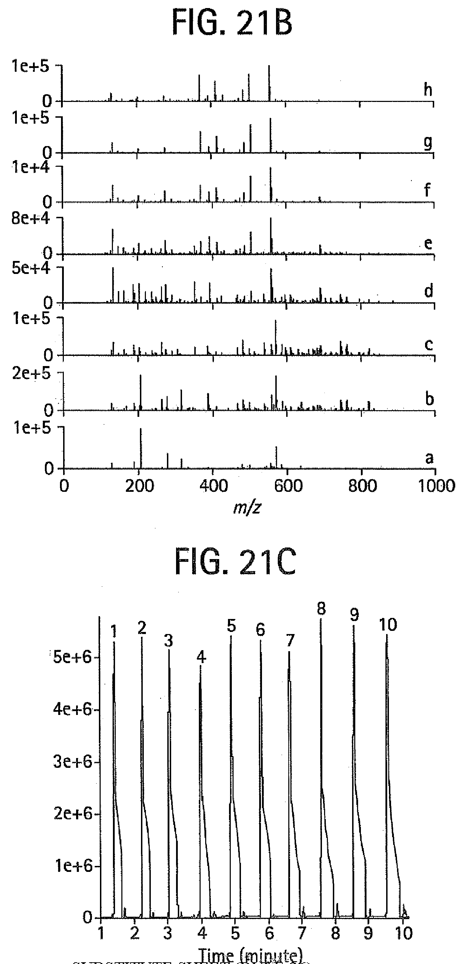

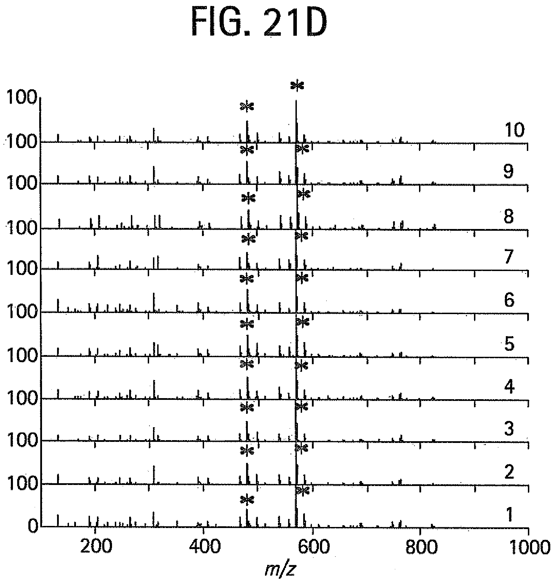

**Figure 21A** is a total ion chronogram (TIC) observed for derivatized serum. Each letter denotes a time interval of 1 second. **Figure 21B** is a series of averaged mass spectra corresponding to each time interval indicated in Figure 21A. **Figure 21C** is a TIC observed for 10 repeat injections of a healthy serum sample analyzed by DART-MS. **Figure 21D** is a series of mass spectra corresponding to TIC peaks shown in Figure 21C. Asterisks denote signals selected for coefficient of variation (CV) calculation.

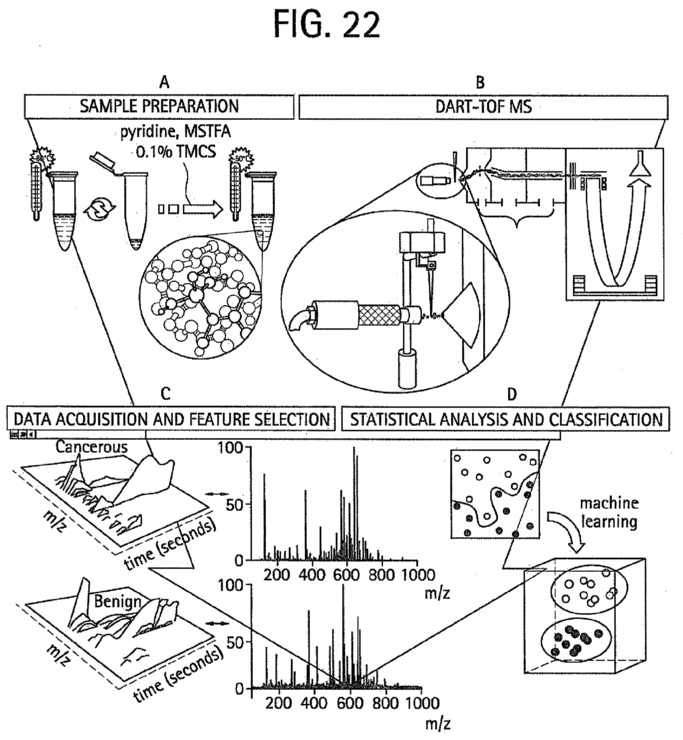

**Figure 22** is a diagram of the study design and workflow used in Example 4 showing metabolomic investigation of serum samples for detection of ovarian cancer by DART-TOF MS. **a.** Serum sample preparation: *i*. protein precipitation, centrifugation and separation of the metabolite containing supernatant followed by *ii*. evaporation of solvent to generate a metabolite-containing pellet. This pellet is then subject to *iii.* derivatization to increase volatility of polar metabolites. **b**, Schematic of the DART-TOF mass spectrometer equipped with a custom-built sample arm (*iv*. glow discharge compartment, *v*. gas heater, *vi*. ionization region where sample-carrying capillary is placed, *vii*. differentially-pumped atmospheric pressure interface to transport ions towards the mass analyzer, *viii*. radiofrequency ion guide where ions are collisionally cooled prior to entering the *ix*. orthogonal TOF mass analyzer. **c**, Typical data is acquired in a time-resolved fashion (*x*. three-dimensional contour plots of single runs corresponding to an ovarian cancer patient (top), and a control (bottom)). The region of the time-resolved signal with best signal-to-noise ratio was averaged yielding *xi.* profile mass spectra reflecting metabolic fingerprints. **d**, Machine learning techniques such as SVMs are used for building a multivariate classifier (*xii.* objects in original variable space, *xiii.* objects in classifier space).

### DETAILED DESCRIPTION OF THE INVENTION

### I. Metabolic biomarker panels

Panels or profiles of metabolic biomarkers for cancer are provided. Metabolites are the end products of cellular regulatory processes, and can be regarded as the ultimate response of biological systems to genetic, pathophysiological or environmental stressors. As used herein, the term "metabolic biomarker" refers to a metabolite that is less than 1,000 Da, and is differentially present in a biological sample from a subject with or at risk of developing cancer as compared to a control subject that does not have cancer or does not have that same type of cancer. The terms "individual", "host", "subject", and "patient" are used interchangeably herein, and refer to a mammal, including, but not limited to, humans, rodents such as mice and rats, and other laboratory animals.

The disclosed metabolic markers can be detected in any biological fluid from a subject, including, but not limited to, serum, blood, plasma, saliva, lymph, cerebrospinal fluid, synovial fluid, urine, or sputum. In preferred embodiments, the disclosed panels of metabolic markers include serum metabolites that are detected in the serum of a subject.

Efforts to discover serum protein biomarkers has been hampered by the fact that the serum proteome is extremely complex, comprising ~2 x 10⁶ protein species with a dynamic range spanning 10 orders of magnitude (Anderson and Anderson, Mol. Cell Proteomics, 1:845-58 (2002)). In comparison, the serum metabolome is relatively less complex, including about 2,500 molecules. As used herein, the term "metabolome", refers to the complete set of small-molecule metabolites (such as metabolic intermediates, hormones and other signaling molecules, and secondary metabolites) that are found within a biological sample, such as a single organism or tissue. The term "serum metabolome" is used herein to refer to the complete set of small-molecule metabolites that are found within the serum of an organism.

The disclosed panels of serum metabolic biomarkers include metabolites that are differentially present in the serum of subjects with or at risk of developing cancer as compared to the serum of control subjects that do not have cancer. A metabolic biomarker is present differentially in samples taken from cancer patients and samples taken from control subjects if it is present at an increased level or a decreased level in serum samples from subjects with cancer as compared to serum samples from control subjects that do not have cancer. Preferably, the increase or decrease in the amount of a metabolic biomarker is a statistically significant difference.

In some embodiments, the metabolic biomarker panels include serum metabolites that are differentially present in subjects with or at risk of developing a gynecologic cancer as compared to control subjects that do not have a gynecologic cancer. In a preferred embodiment, the gynecologic cancer is ovarian cancer.

The disclosed panels of serum metabolic biomarkers include at least 2 or more serum metabolites. In some embodiments, the metabolic biomarker panels include 2, 3, 4, 5, 6, 7, 8, 9, 10, 15, 20, 25, 50, 75, 100, 150, or more metabolites. In preferred embodiments, the metabolic biomarker panels include 10 or more metabolites. Serum metabolic biomarkers may be characterized by their molecular weight, their chemical formula, their mass-to-charge ratio (*m*/*z*), for example as determined by mass spectrometry, or their chemical name.

There may be some variation in *m*/*z* value or molecular weight. For example, there may be variation that is dependent on the resolution of the machine used to determine *m*/*z* value or molecular weight, or on chemical modification of the metabolic biomarker. Accordingly, the metabolic biomarkers listed disclosed herein may have the specified *m*/*z* value or molecular weight plus or minus about 10%, about 5%, about 1%, about 0.5% or about 0.2%.

In one embodiment, the panel of serum metabolic biomarkers includes at least 2, 3, 4, 5, 6, 7, 8, 9, 10, 15, 20, 25, 30, 35 or 40 of the serum metabolites with molecular weights (in Daltons) of about: 187.0614, 256.2398,278.1434,278.1615, 306.3145, 308.1377, 308.2881, 322.1534, 354.1682, 368.1588, 369.2999, 428.3340, 453.2861, 453.2867, 456.2856, 467.2955, 470.2904, 481.2914, 484.3061, 485.3773, 490.3327, 495.3206, 495.3380, 495.3394, 499.9355, 505.2842, 507.3592, 517.3238, 519.3070, 521.3220, 523.3690, 525.2924, 530.3115, 553.3424, 304.2407, 304.2512, 632.2342, 635.4104, 640.4429, 654.4586, 700.4640, 743.5473, 757.5572, and 759.5895. In another embodiment, the panel of serum metabolic biomarkers includes all of the above-listed serum metabolites.

In another embodiment, the panel of serum metabolic biomarkers includes at least 2, 3, 4, 5, 6, 7, 8, 9 or 10 of the serum metabolites with the following chemical names: Phe-Ile, PE(16:0/0:0), PC(14:0/0:0), PC(16:0/0:0), PC(18:3(9Z, 12Z, 15Z)/0:0[U]), 3-sialyllactosamine, PE-NMe(18:1(9E)/18:1(9E)), palmitic acid, arachidonic acid, Gln-His-Ala, 4a-Carboxy-4b-methyl-5a-cholesta-8,24-dien-3b-olercalcitriol, PE(16:0/0:0), PC(O-16:0/2:0) platelet activating factor, and PE(18:1(9E)/18:1(9E)). The term "PE" refers to phosphatidylethanolamine. The term "PC" refers to phosphatidylcholine. In another embodiment, the panel of serum metabolic biomarkers includes all of the above-listed serum metabolites.

In another embodiment, the panel of serum metabolic biomarkers includes at least 2, 3, 4, 5, 6, 7, 8, 9, 10, 15, 20, 25, 30, 35 or 40, or all of the serum metabolites with the properties indicated in Tables 6 and 7.

In another embodiment, the panel of serum metabolic biomarkers includes at least 2, 3, 4, 5, 6, 7, 8, 9, 10, 15, 20, 25, 30 or 35 of the serum metabolites with molecular weights (in Daltons) of about: 148.0129, 204.0695, 256.2398, 274.1710, 278.1434, 280.2446, 280.2460, 282.2154, 284.2701, 340.2489, 354.1676, 368.1652, 384.2831, 398.2982, 433.3256, 444.3037, 479.3310, 481.2835, 481.3047, 495.3210, 499.9613, 505.2842, 505.3308, 507.3131, 509.3156, 519.3330, 519.3459, 529.2699, 563.3363, 683.5089, 697.5246, 743.5300, 757.5457, 757.5678, 759.5775, 781.5595, 787.6000, and 932.6173. In another embodiment, the panel of serum metabolic biomarkers includes all of the above-listed serum metabolites.

In another embodiment, the panel of serum metabolic biomarkers includes at least 2, 3, 4, 5, 6, 7, 8, 9 or 10 of the serum metabolites with the following chemical names: LysoPC(18:2(9Z,12Z) or isomers thereof, PE-NMe(18:1(19E)118:1(9E)) or isomers thereof, PC(14:0/20:1(11Z)) or isomers thereof, PC(14:0/22:4(7Z,10Z,13Z,16Z)) or isomers thereof, PC(14:0/22:1(13Z)) or isomers thereof, palmitic acid or isomers thereof, 12-hydroxy-8E,10Eheptadecadienoic acid, stearic acid or isomers thereof, Gln-His-Ala or isomers thereof, DHEA Sulfate or isomers thereof, Lithocholic acid glycine conjugate, PC(P-16:0/0:0) or isomers thereof, PC(10:0/4:0) or isomers thereof, PE(9:0/10:0) or isomers thereof, and glycoursodeoxycholic acid 3-sulfate or isomers thereof. In another embodiment, the panel of serum metabolic biomarkers includes all of the above-listed serum metabolites.

In another embodiment, the panel of serum metabolic biomarkers includes at least 2, 3, 4, 5, 6, 7, 8, 9, 10, 15, 20, 25, 30 or 35, or all of the serum metabolites with the properties indicated in Tables 18 and 19.

In another embodiment, the panel of serum metabolic biomarkers includes at least 2, 3, 4, 5, 6, 7, 8, 9, 10, 15, 20, 25, 50, 75, 100, 150, 200 or 250 of the serum metabolites with *m*/*z* values of about: 108.1764, 109.1530, 110.1295, 11.1061, 112.0826, 113.0592, 114.0357, 115.0123, 116.3143, 119.2440, 123.1502, 124.1267, 125.1033, 126.0798, 127.0564, 128.0329, 132.2646, 133.2412, 139.1005, 140.0770, 141.0536, 142.0301, 144.3087, 146.2618, 147.2384, 150.1680, 151.1446, 152.1211, 156.0273, 158.3059, 161.2356, 162.2121, 167.0949, 168.0714, 170.0245, 172.3031, 174.2562, 175.2328, 176.2093, 178.1624, 180.1155, 181.0921, 183.0452, 184.0217, 185.3238, 186.3003, 187.2769, 188.2534, 193.1362, 194.1127, 198.0189, 200.2975, 202.2506, 204.2037, 208.1099, 209.0865, 210.0630, 211.0396, 212.0161, 214.2947, 216.2478, 222.1071, 225.0368, 228.2919, 229.2685, 230.2450, 232.1981, 235.1278, 238.0574, 241.3126, 242.2891, 243.2657, 244.2422, 246.1953, 248.1484, 250.1015, 252.0546, 254.0077, 257.2629, 258.2394, 259.2160, 260.1925, 263.1222, 264.0987, 266.0518, 268.0284, 268.0049, 269.3070, 270.2835, 271.2601, 272.2366, 274.1897, 278.0959, 279.0725, 280.0490, 281.0256, 282.0021, 283.3042, 284.2807, 285.2573, 288.1869, 292.0931, 293.0697, 294.0462, 295.0228, 296.3248, 298.2779, 299.2545, 300.2310, 301.2076, 302.1841, 303.1607, 304.1372, 306.0903, 308.0434, 309.0200, 313.2517, 315.2048, 318.1344, 320.0875, 323.0172, 324.3192, 325.2958, 326.2723,327.2489, 329.2020, 331.1551, 332.1316, 336.0378, 338.3164, 344.1757, 341.2461, 345.1523, 346.1288, 347.1054, 352.3136, 353.2902, 355.2433, 357.1964, 359.1495, 360.1260, 361.1026, 364.0322, 366.3108, 369.2405, 371.1936, 374.1232, 376.0763, 378.0294, 379.0060, 383.2377, 385.1908, 387.1439, 388.1204, 390.0735, 391.0501, 392.0266, 394.3052, 396.2583, 397.2349, 399.1880, 400.1645, 401.1411, 402.1176, 403.0942, 404.0707, 406.0238, 408.3024, 410.2555, 413.1852, 416.1148, 418.0679, 419.0445, 422.2996, 423.2762, 424.2527, 425.2293, 428.1589, 429.1355, 431.0886, 435.3203, 437.2734, 439.5520, 443.1327, 445.0858, 447.0389, 448.0154, 450.2940, 451.2706, 460.0595, 464.2912, 468.1974, 471.1271, 473.0802, 475.0333, 478.2884, 482.1946, 485.1243, 487.0774, 490.0070, 492.2856, 494.2387, 496.1918, 500.0980, 502.0511, 503.0277, 507.2594, 508.2359, 510.1890, 516.0483, 517.0249, 518.0014, 520.2800, 522.2331, 526.1393, 530.0455, 531.0221, 532.3241, 534.2772, 540.1365, 548.2744, 550.2275, 559.0165, 566.1778, 568.1309, 576.2688, 578.2219, 582.1281, 586.0343, 592.2191, 598.0784, 602.3101, 603.2867, 604.2632, 610.1225, 612.0756, 619.237, 620.2135, 628.0259, 630.3045, 632.2576, 636.1638, 638.1169, 640.07, 648.2079, 650.161, 654.0672, 660.252, 664.1582, 670.0175, 674.2492, 686.2933, 688.2464,691.1761,699.314,700.2905, 702.2436 and 714.2877. In another embodiment, the panel of serum metabolic biomarkers includes all of the above-listed serum metabolites.

In another embodiment, the panel of serum metabolic biomarkers includes at least 2, 3, 4, 5, 6, 7, 8, 9, 10, 15, 20, 25, 50, 75, 100, 150 or 200 of the serum metabolites: Histamine, D-Proline, Ethanol, Guanidine, Urea, beta-Aminopropionitrile, 3-aminopropanal, Pyridine, L-Alanine, 2-Piperidinone, L-a-aminobutyric, acid, L-Serine, p-Cresol, Imidazole-4-acetaldehyde, trans-Hex-2-enoic acid, L-Proline, Benzamide, 1-Methylhistamine, D-1-Piperideine-2-carboxylic acid, Pyroglutamic acid, L-Isoleucine, 2-Phenylacetamide, Tetrahydropteridine, Tyramine, L-Histidinol, Proline betaine, 6-Methyladenine, D-Arabitol, 2-Methyl- butyrylglycine, 7-Methylguanine, Pyridoxamine, 1-Methylhistidine, N-butanoyl-lhomoserine lactone, Hexanoylglycine, Citrulline, 5-Hydroxytryptophol, 2(N)-Methyl-norsalsolinol, 6-methyl- tetrahydropterin, 11-dodecen-1-ol, Ala Pro, Proline, (R)-N-Methylsalsolinol, 1-Methylhistamine, Thymine, Pyroglutamic acid, Deoxyribose, 2-Phenylacetamide, Histidinal, 2-amino-8-oxo-9,14-epoxy-decanoic acid, Glycine, Mevalonic acid, 10-pentadecenal, Dopamine, 5-Tetradecenoic acid, L-Histidine, L-isoleucyl-L-proline, 3-Methyl-crotonylglycine, 2-Methyl- butyrylglycine, Beta-Alanine, L-Methionine, 3-Methyldioxyindole, S-aminomethyl-dihydrolipoamide 9-hexadecen-1-ol, D-Glyceraldehyde 3-phosphate, Hexanoylglycine, Citrulline, Deoxyadenosine, 5-Hydroxy-kynurenamine, L-Tyrosine, Hypogeic acid, Palmitic acid, 2-hydroxy-pentadecanoic acid, Ser-Pro-Gly, Estradiol, Gly Pro Thr, Dimethyl-L-arginine, Bovinic acid, Vaccenic acid, Stearic acid, C 17 Sphinganine, S-(3-Methylbutanoyl)-dihydrolipoamide-E, 11Z-eicosen-1-ol, Sphinganine, Gamma-Aminobutyryl-lysine, Aminoadipic acid, L-beta-aspartyl-L-threonine, 14Z-eicosenoic acid, 10-oxo-nonadecanoic acid, 5-HEPE, Argininic acid, 5-Hydroxytryptophol, Fructosamine, D-Glucose, 19-oxo-eicosanoic acid, 2-hydroxy-eicosanoic acid, MG(0:0/16:0/0:0), Ser-Pro-Gly, Ser-Gly-Val, Kyotorphin, 2-oxo-heneicosanoic acid, 2-(3-Carboxy-3-(methylammonio)propyl)-L-histidine, N-propyl arachidonoyl amine, Dimethyl-L-arginine, Queuine, 8-iso-15-keto-PGE2, Dihydrolipoamide, MG(0:0/18:3(6Z,9Z,12Z)/0:0), N-(2-hydroxyethyl)icosanamide, 2-hydroxy behenic, MG(18:0/0:0/0:0), 5beta-Cholane-3alpha,24-diol, 3b,17b-Dihydroxyetiocholane, Pro-His-Asn, Val-Arg-Pro, Prolylhydroxyproline, MG(0:0/14:0/0:0), Dihydroxycoprostanoic acid, 5-Methoxytryptophan, 25-Azacholesterol, Lys-Thr, Deoxyadenosine, 4a-Methylzymosterol, 7-Ketocholesterol, MG(0:0/16:0/0:0), Ser-Gly-Val, Kyotorphin, Lys-Met-His, Val-Glu-Val, Epsilon-(gamma-Glutamyl)-lysine, Queuine, Val-Tyr-Ala, N-(2-hydroxyethyl) icosanamide, 1α-hydroxy-25-methoxyvitamin D3, Ala-Thr-Thr, Ser-Phe-Ile, Pro-Ser-Val, Gln-Arg-Phe, Tyr-Gly-Ala, 3'-O-Aminopropyl-25-hydroxyvitamin D3, 3-Sulfodeoxycholic acid, Arg-Arg-Glu, Tyr-Ala-Ala, Trp-Asp-Arg, Asp-Val-Thr, Lys-Met-His, Glu-Thr-Thr, Trp-Lys-Tyr, 2-hexacosanamido- ethanesulfonic acid, Ser-Phe-Ile, Sulfolithocholylglycine, Phe-Ser-Glu, N-[(3a,5b,7b)-7-hydroxy-24-oxo-3-(sulfooxy)cholan-24-yl]-Glycine, Arg-Phe-His, Arg-Arg-Glu, Ile-Val-Tyr, Thr-Glu-Phe, Arg-Trp-Trp, Asn-Arg-Asp, Leucine Enkephalin, Ile-Arg-Gln, Trp-Ser-Lys, Gln-Phe-Gln, Tyr-Ile-Glu, Gln-Glu-Arg, Arg-Cys-Arg, Tyr-Lys-Gln, Taurocholic Acid, N-[(3a,5b,7b)-7-hydroxy-24-oxo-3-(sulfooxy)cholan-24-yl]-Glycine, Lys-His-Trp, His-Tyr-Arg, 11-beta-hydroxy-androsterone-3-glucuronide, and Arg-His-Trp. In another embodiment, the panel of serum metabolic biomarkers includes all of the above-listed serum metabolites.

In another embodiment, the panel of serum metabolic biomarkers includes at least 2, 3, 4, 5, 6, 7, 8, 9, 10, 15, 20, 25, 50, 75, 100, 150, 200 or 250, or all of the serum metabolites with the properties indicated in Table 24.

In another embodiment, the panel of serum metabolic biomarkers includes at least 2, 3, 4, 5, 6, 7, 8, 9 or 10 of the serum metabolites with *m*/*z* values of about: 199.9720, 208.6214, 317.8554, 452.3401, 500.6095, 509.8635, 553.4827, 621.8411, 683.5962, 691.0366, 726.5643, 787.2499, 787.2964 and 787.3429. In another embodiment, the panel of serum metabolic biomarkers includes all of the above-listed serum metabolites.

In another embodiment, the panel of serum metabolic biomarkers includes at least 2, 3, 4, 5,6, 7, 8,9 or 10 of the serum metabolites with the following chemical names: D-1-Piperideine-2-carboxylic acid, 2-Phenylacetamide, D-Glyceraldehyde 3-phosphate, 5-Methoxytryptophan, N-(2-hydroxyethyl) icosanamide, Isopentenyladenine-9-N-glucoside, Asp-Val-Thr. LysoSM(d18:0) and His-Tyr-Arg. In accordance with the invention, the panel of serum metabolic biomarkers includes all of the above-listed serum metabolites.

In another embodiment, the panel of serum metabolic biomarkers includes at least 2, 3, 4, 5, 6, 7, 8, 9 or 10, or all of the serum metabolites with the properties indicated in Table 25.

In another embodiment, the panel of serum metabolic biomarkers includes at least 2, 3, 4, 5, 6, 7, 8,9 or 10 of the serum metabolites with *m*/*z* values of about: 317.8554, 452.3401, 509.8635, 553.4827, 553.5292, 636.0243, 636.0708, 667.6924, 691.0366, 787.2499, 787.2964 and 787.3429. In another embodiment, the panel of serum metabolic biomarkers includes all of the above-listed serum metabolites.

In another embodiment, the panel of serum metabolic biomarkers includes at least 2, 3,4, 5,6,7, 8,9 or 10 of the serum metabolites with the following chemical names: D-Glyceraldehyde 3-phosphate, 5-Methoxytryptophan, Isopentenyladenine-9-N-glucoside, Asp-Val-Thr, Asn-Met-Arg, Ceramide, (d18:1/9Z-18:1) and His-Tyr-Arg. In another embodiment, the panel of serum metabolic biomarkers includes all of the above-listed serum metabolites.

In another embodiment, the panel of serum metabolic biomarkers includes at least 2, 3, 4, 5, 6, 7, 8, 9 or 10, or all of the serum metabolites with the properties indicated in Table 26.

### II. Methods for using metabolic biomarkers,

### A. Detecting subjects for cancer treatment

Methods for using the disclosed metabolic biomarker panels and methods to identify, or assist in the identification of, subjects for treatment of cancer are provided. The subjects selected for treatment of cancer may have cancer, or may have an increased risk for developing cancer relative to the general population. The methods include the steps of obtaining a serum sample containing metabolites from the subject, detecting the amounts of two or more metabolic biomarkers selected from one of the disclosed metabolic biomarker panels in the serum sample, and determining whether or not the amounts of the metabolic markers in the sample are indicative of cancer or the propensity to develop cancer. The detected amount of one or more metabolites in a sample is referred to herein as "expression data". Determining whether or not the metabolic biomarker expression data is indicative of cancer or the propensity to develop cancer includes the step of comparing the metabolic biomarker expression data from the test subject to the expression data of the metabolic biomarkers from a group of control subjects that do not have cancer and a group of subjects that do have cancer.

The examples below demonstrate that, when used with the disclosed diagnostic methods, these metabolic biomarker panels can diagnose ovarian cancer in subjects with a high degree of accuracy, sensitivity and specificity. The performance of the disclosed diagnostic methods may be assessed by considering the number of subjects correctly diagnosed (true positives (TP) and true negatives (TN)) and incorrectly diagnosed (false positives (FP) and false negatives (FN)). The term "accuracy" is used herein to refer to the proportion of correct classifications (accuracy = (TP+TN)/(TP+FP+TN+FN)). The term "sensitivity" is used herein to refer to the conditional probability of true positive (sensitivity = TP/(TP+FN)). The term "specificity" is used herein to refer to the conditional probability of true negative (specificity = TN/(TN+FP)).

Use of expression data from two or more metabolic biomarkers enhances the accuracy of the diagnosis. Using combinations of more than two metabolic biomarkers, such as three or more metabolic biomarkers, may further enhance the accuracy of diagnosis. Accordingly, expression data from two or more markers, preferably three or more markers, for example four or more markers, such as five, six, seven, eight, nine, ten, fifteen, twenty or more markers, are used in the disclosed diagnostic methods.

In preferred embodiments, the disclosed methods allow a diagnosis of cancer with an accuracy, a specificity, and/or a sensitivity of at least 80%, 85%, 90%, 95%, 96%, 97%, 98% or 99%. Serum metabolic biomarkers may be selected from the disclosed biomarker panels to provide the desired diagnostic accuracy, specificity, and/or sensitivity.

One embodiment provides a method for selecting a subject for treatment of cancer by detecting *in vitro* the levels of two or more metabolic biomarkers in a serum sample obtained from the subject, wherein the metabolic biomarkers are selected from the group consisting of serum metabolites with *m*/*z* values of about: 199.9720, 208.6214, 317.8554, 452.3401, 500.6095, 509.8635, 553.4827, 621.8411, 683.5962, 691.0366, 726.5643, 787.2499, 787.2964 and 787.3429. The method further includes comparing the levels of the two or more metabolic biomarkers detected in the serum sample to predetermined levels of the metabolic biomarkers detected in a group of subjects without cancer and to the predetermined levels of the biomarkers detected in a group of subjects with cancer, and selecting the subject for treatment wherein the levels of the two or more metabolic biomarkers in the serum sample obtained from the subject correlate with the predetermined levels of the metabolic biomarkers in the group of subjects with cancer. The method has greater than 80% predictability, preferably greater than 95% predictability.

### 1. Cancers to be diagnosed

The metabolic biomarker panels disclosed herein can be used to diagnose any cancer, including, but not limited to, the following: bladder, brain, breast, colo-rectal, esophageal, kidney, liver, lung, nasopharangeal, pancreatic, prostate, skin and stomach. In some embodiments, the metabolic biomarker panels are used to diagnose gynecologic cancers, including ovarian, cervical, uterine, vulvar and vaginal cancer. In accordance with the invention, the metabolic biomarkers panels are used to diagnose a subject as having ovarian cancer or as having an increased risk for developing ovarian cancer as compared to a control.

### 2. Secondary indicators

The metabolic biomarkers can be used in combination with one or more other symptoms or diagnostic markers of cancer. Additional methods for diagnosing cancer include, but are not limited to, physical examination, imaging methods such as X-rays, CT scanning, PET scanning and MRI imaging, and detection of additional biomarkers, such as alpha-fetoprotein (AFP), beta human chorionic gonadotropin (β-HCG), calcitonin, carcinoembryonic antigen (CEA) and prostate-specific antigen (PSA). For example, diagnosis of ovarian cancer can include performing ovarian palpation, transvaginal ultrasound, or screening for additional markers, such as CA-125.

### B. Monitoring efficacy of cancer treatment

Methods for using the disclosed metabolic biomarker panels and methods to monitor the efficacy of a cancer treatment are provided. The methods include the steps of obtaining a serum sample containing metabolites from a subject prior to administration of a cancer therapy, obtaining one or more serum samples from the same subject at one or more time points during and/or following the cancer therapy, detecting the amounts of two or more metabolic biomarkers selected from one of the disclosed metabolic biomarker panels in the serum samples, and determining whether or not the levels of the biomarkers changed in the serum samples during and/or following administration of the cancer therapy. In one embodiment, the metabolic biomarker expression data from each serum sample is compared to expression data of the metabolic biomarkers from a group of control subjects that do not have cancer and a group of subjects that do have cancer. Differences in metabolic biomarker expression data during and/or following cancer treatment as compared to metabolic biomarker expression data prior to treatment, such that the expression data during and/or following cancer treatment is less closely correlated with expression data from the group of subjects that have cancer is indicative of an efficacious treatment. No change in metabolic biomarker expression data during and/or following treatment, or a change in metabolic biomarker expression data, such that the expression data during and/or following cancer treatment is more closely correlated with expression data from the group of subjects that have cancer is indicative of the treatment having a low or no efficacy.

### C. Methods for detecting levels of metabolic biomarkers

The disclosed metabolic biomarkers can be detected in serum samples using any suitable method. Exemplary methods include mass spectrometry and specific binding assays. Prior to detection using one of these methods, the serum is treated to remove polypeptides, proteins, and other large biomolecules. For example, the serum sample can be treated with acetonitrile or a 2:1 (v/v) acetone:isopropanol mixture to precipitate proteins which can then be removed from the serum sample by centrifugation. The samples can also be treated to derivatize the serum metabolites for improved detection. For example, the serum sample can be treated with N-trimethylsilyl-N-methyltrifluoroacetamide (MSTFA) to result in TMS derivatization of amide, amine and hydroxyl groups for improved detection by mass spectrometry.

### 1. Mass spectrometry methods

Gas phase ion spectrometry requires a gas phase ion spectrometer to detect gas phase ions. Gas phase ion spectrometers include an ion source that supplies gas phase ions and include mass spectrometers, ion mobility spectrometers and total ion current measuring devices. Since metabolites have vastly-differing chemical properties, and occur in a wide range of concentrations, mass spectrometry (MS) is a preferred method for obtaining metabolic expression data. In preferred embodiments, the disclosed metabolic biomarkers are detected using mass spectrometry methods.

A mass spectrometer is a gas phase ion spectrometer that measures a parameter which can be translated into mass-to-charge ratios (*m*/*z*) of gas phase ions. Mass spectrometers typically include an ion source and a mass analyser. Examples of mass spectrometers are time-of-flight (ToF), magnetic sector, quadrupole filter, ion trap, ion cyclotron resonance, electrostatic sector analyser and hybrids of these. A laser desorption mass spectrometer is a mass spectrometer which uses laser as a means to desorb, volatilize and ionize an analyte. A tandem mass spectrometer is mass spectrometer that is capable of performing two successive stages of *m*/*z-*based discrimination or separation of ions, including ions in an ion mixture. Methods for performing mass spectrometry on a sample are generally known in the art.

### a. Liquid chromatography-mass spectrometry (LC-MS)

Mass spectrometry can be combined with chromagraphic separation techniques to detect metabolites in complex mixtures such as serum. In one embodiment, metabolites are detected using liquid chromatography-mass spectrometry (LC-MS) which combines the physical separation capabilities of liquid chromatography with the mass analysis capabilities of mass spectrometry. Suitable mass analyzers for use in LC-MS include single quadrupole, triple quadrupole, ion trap, time-of-flight (TOF) and quadrupole-time-of-flight (Q-TOF). The TOF analyzer uses an electric field to give all ions the same kinetic energy, and then measures the time they take to reach the detector. If the particles all have the same charge, the kinetic energies are identical, and their velocities depend only on their masses with lighter ions reaching the detector first. In one embodiment, the metabolites are detected using LC-TOF mass spectrometry.

### b. Direct analysis in real time mass spectrometry (DART MS)

In some embodiments, the mass spectrometry method used to detect serum metabolites does not include an initial chromatographic separation step. In a preferred embodiment, direct analysis in real time (DART) mass spectrometry is used. DART MS is a technique where a stream of excited metastables is used to desorb and chemically ionize a dried drop of solution containing analytes, such as a mixture of metabolites extracted from serum. A mass spectrometer is then used to evaluate the relative abundances of these metabolites. The method displays no memory effects, as it is performed in a non-contact fashion. This increases the reproducibility of the metabolic fingerprints, enabling the detection of differences between disease states. Moreover, DART is able to ionize a broad range of metabolites with varying polarities, enabling the simultaneous interrogation of multiple species.

### 2. Specific binding assays

In some embodiments, specific binding assays can be used for detecting the presence and/or measuring a level of metabolic biomarker in a serum sample, using binding reagents that specifically bind to the metabolites to be detected. A binding reagent "specifically binds" to a metabolite when it binds with preferential or high affinity to the metabolite for which it is specific, but does not bind, does not substantially bind or binds with only low affinity to other substances.

The specific binding agent may be an antibody or antibody fragment specific for the metabolic biomarker. The antibody may be a monoclonal or polyclonal antibody. Monoclonal antibodies are preferred. Antibodies also include antibody fragments, such as Fv, F(ab') and F(ab')₂ fragments as well as single chain antibodies. Suitable antibodies are available in the art. Antibodies and antibody fragments may also be generated using standard procedures known in the art. Aptamers and interacting fusion proteins may also be used as specific binding agents. Specific binding agents also include molecularly imprinted polymers (MIPs). MIPs, or "plastic antibodies", are polymers that are formed in the presence of a molecule that is extracted afterwards, thus leaving complementary cavities behind. The specific binding agent may recognize one or more form of the metabolic biomarker of interest.

Methods for using specific binding agents to detect metabolites generally include the steps of:
a) contacting the sample with binding agents specific for a metabolite to be detected; and
b) detecting binding between the binding agents and molecules of the sample.

Detection of specific binding of the antibody, when compared to a suitable control, is an indication that the metabolite being tested is present in the sample. Suitable controls include a sample known not to contain the metabolite, and a sample contacted with a binding agent (i.e., an antibody) not specific for the metabolite, e.g., an anti-idiotype antibody. A variety of methods to detect specific molecular interactions are known in the art and can be used in the method, including, but not limited to, immunoprecipitation, an enzyme immunoassay (i.e. an ELISA assay), and a radioimmunoassay. In general, the specific binding agent will be detectably labeled, either directly or indirectly. Direct labels include radioisotopes; enzymes whose products are detectable (e.g., luciferase, β-galactosidase, and the like); fluorescent labels (e.g., fluorescein isothiocyanate, rhodamine, phycoerythrin, and the like); fluorescence emitting metals, e.g., ¹⁵²Eu, or others of the lanthanide series, attached to the antibody through metal chelating groups such as EDTA; chemiluminescent compounds, e.g., luminol, isoluminol, acridinium salts, and the like; bioluminescent compounds, e.g., luciferin, aequorin (green fluorescent protein), and the like. The specific binding agent may be attached (coupled) to an insoluble support, such as a polystyrene plate or a bead. Indirect labels include secondary antibodies specific for metabolite-specific antibodies, wherein the secondary antibody is labeled as described above; and optionally contain members of specific binding pairs, e.g., biotin-avidin, etc. The biological sample may be brought into contact with and immobilized on a solid support or carrier. The support may then be washed with suitable buffers, followed by contacting with a detectably-labeled metabolite-specific binding agent.

### D. Methods for determining if levels of detected metabolic biomarkers are indicative of cancer or the propensity to develop cancer

The expression pattern of the metabolic biomarkers of interest is examined to determine whether expression of the metabolic biomarkers is indicative of the patient having cancer. Any suitable method of analysis may be used. Typically, the analysis method used includes comparing the expression data obtained from a subject to be diagnosed with expression data obtained from patients known to have cancer and control subjects who do not have cancer. It can then be determined whether or not the expression of the markers in the subject is more similar to the expression pattern observed in known cancer patients or to the expression pattern observed in control subjects. The method of analysis typically measures the likelihood of a subject having cancer.

### a. Classifiers

Supervised classification methods can be used to determine whether or not the expression patter of metabolic biomarkers in a subject is more similar to the expression pattern observed in known cancer patients or to the expression pattern observed in control subjects. Suitable supervised classification methods include, but are not limited to, partial least squares-discriminant analysis (PLSDA), soft independent modeling of class analogy (SIMCA), artificial neural networks (ANNs), or classification and regression trees (CART). These approaches allow the identification of robust spectral features that may be obscured by biological variability not related to disease.

The method by which it is determined whether the expression data is indicative of cancer, or not, is typically implemented using a computer. The computer may be physically separate from or may be coupled to the reader used to generate expression data, for example to the mass spectrometer.

### 1. Machine learning classifiers

Supervised machine learning classification methods may be used to discriminate the expression data of patients with cancer from expression data of control subjects. The machine learning classifier is first trained using training expression data from cancer patients and training control data from the control subjects.

Methods of training a machine learning classifier to distinguish expression data from a cancer patient from expression data from a subject who does not have cancer include the steps of inputting training data from cancer patients and control subjects where the training data is expression data relating to two or more of the disclosed metabolic biomarkers. The computer maps these input variables (such as *m*/*z* values) to feature space using a kernel and the classifier learns to discriminate between cancer data and control data thus producing a training classifier to discriminate between cancer data and control data.

The trained classifier may then optionally be tested using expression data from further cancer patients and further control subjects to determine the sensitivity, specificity, and/or accuracy of the trained machine learning classifier. Independent training and testing sets may be used, with similar numbers of cancer cases and controls and similar representation of age and sex in each set. The testing data from cancer patients and/or control subjects is mapped by the computer to feature space using a kernel and the trained classifier is used to assign the class of the input variables as being cancer data or non-cancer data. It can then be determined whether the test data has been classified correctly or mis-classified.

A trained machine learning classifier may be used to determine whether expression data from a subject whom it is wished to diagnose as having, or not having, cancer is indicative of the patient having, or not having, cancer. The trained machine learning classifier used in such a method of diagnosis may have been tested as described above, but this testing step is not essential. The diagnostic steps include imputing expression data for two or more of the disclosed metabolic biomarkers into the trained machine learning classifier, which the computer maps to feature space using a kernel. The trained machine learning classifier then classifies the sample as being a cancer sample or non-cancer sample. Hence, the test subject is diagnosed as having or not having cancer and can be selected or nor for treatment of cancer.

Suitable machine learning classifiers include the single layer perceptron (SLP), the multi-layer perceptron (MLP), decision trees and support vector machines (SVMs). Preferably the classifier is an SVM. In machine learning, SVMs are widely considered to represent the state of the art in classification accuracy. SVMs have been successfully applied to various scientific problems as they generally achieve classification performance superior to that of many older methods, particularly in high-dimensional settings (Li, et al., Artificial Intelligence Med., 32(2):71-83 (2004); Rajapakse, et al., Am. J., Pharmacogenomics, 5(5):281 (2005); Yu, et al., Bioinformatics, 21(10):2200-2209 (2005); Shen, et al., Cancer Informatics, 3:339-349 (2007); Wu, et al., Bioinformatics, 19(13):1636-43 (2003); Pham, et al., Stat. Appl. Genetics. Mol. Biol., 7(2):11 (2008)).

Given a dataset $S = {\left\{{x}_{1}, {y}_{1}\right\}}_{i = 1}^{M} ( {x}_{i} ∈ {R}^{N}$ is the feature vector of *i*^{th} instance and *yᵢ* is the corresponding label), for two-class classification problems, the standard linear SVM solves the following convex optimization: ${min}_{w , ξ} \frac{1}{2} {‖ w ‖}^{2} + C {\sum }_{i = 1}^{M} {ξ}_{i} s . t . { y}_{i} \left(w⋅{x}_{i}+b\right) + {ξ}_{i} \geq 1 , {ξ}_{i} \geq 0 , i = 1 , ⋯ , M$

In the case of nonlinear SVMs, the feature vectors *xᵢ* ∈ *R^{N}* are mapped into high dimensional Euclidean space, H, through a mapping function Φ(·) : *R^{N}* → *H*. The optimization problem becomes: ${min}_{w , ξ} \frac{1}{2} {‖ w ‖}^{2} + C {\sum }_{i = 1}^{M} {ξ}_{i} s . t . { y}_{i} \left(w⋅Φ\left({x}_{i}\right) + b\right) + {ξ}_{i} \geq 1 , {ξ}_{i} \geq 0 , i = 1 , ⋯ , M$

The kernel function is defined as *K*(*xᵢ*,*xⱼ*) = Φ(*xᵢ*)·Φ(*xⱼ*) -- for example, for a polynomial kernel of degree 2, *K*(*xᵢ*,*xⱼ*) = (*gxᵢ*·*xⱼ* + *r*)², where g, r are kernel parameters. The linear kernel function is defined as: $K \left({x}_{i}, {x}_{j}\right) = {x}_{i} ⋅ {x}_{j} .$

Tools such as libSVM (http://www.csie.ntu.edu.tw/cjlin/libsvm) can efficiently solve the dual formation of the following problem: ${min}_{α} \frac{1}{2} {\sum }_{i = 1}^{M} {y}_{i} {y}_{j} {α}_{i} {α}_{j} K \left({x}_{i}, {x}_{j}\right) - {\sum }_{i = 1}^{M} {α}_{i} s . t . {\sum }_{i = 1}^{M} {y}_{i} {α}_{i} = 0 , 0 \leq {α}_{i} \leq C , i = 1 , ⋯ , M$ where α*ᵢ* is the Lagrange multiplier corresponding to the *i*^{th} inequality in the primal form. The solution is $w = {\sum }_{i = 1}^{M} {α}_{i} {y}_{i} Φ \left({x}_{i}\right)$ (in the case of linear SVM, $w = {\sum }_{i = 1}^{M} {α}_{i} {y}_{i} {x}_{i} ) .$ The optimal decision function for an input vector x is *f*(*x*) = *w* • Φ(*x*) + *b*, that is, $f \left(x\right) = {\sum }_{i = 1}^{M} {α}_{i} {y}_{i} K \left({x}_{i}, x\right) ,$ where the predicted class is +1 if *f*(*x*) > 0 and -1 otherwise.

In functional classification problems, the input data instances *Xᵢ* are random variables that take values in an infinite dimensional Hilbert space H, the space of functions. The goal of classification (Biau, et al., IEEE Transactions on Information Theory, 51:2163-2172 (2005)) is to predict the label y of an observation X given training data $\left(S={\left\{{X}_{i}, {y}_{i}\right\}}_{i = 1}^{M} , { X}_{i} ∈ H\right) .$

In practice, the functions that describe the input data instances *X*₁,···, *X_{M}* are never perfectly known. Often, n discretization points have been chosen in *t*₁,···, *t_{N}* ∈ *R*, and each functional data instance *X* is described by a vector in *R^{N}*, (*Xᵢ* (*t*₁),···, *Xᵢ*(*t_{N}*)). Sometimes, the functional data instances are badly sampled and the number and the location of discretization points are different between different functional data instances. A usual solution under this context is to construct an approximation (such as B-spline interpolation) for each input functional data instance *Xᵢ* based on its observation values, and then apply sampling uniformly to the reconstructed functional data (Visintin, et al., Clin. Cancer Res., 14:1065-1072 (2008); Greene, et al., Clin. Cancer Res., 14: 7574-7575 (2008)). Therefore, a simple solution would be to apply the standard SVM to the vector representation of the functional data.

However, in some application domains such as chemometrics, it is well known that the shape of a spectrum is sometimes more important than its actual mean value. Therefore, it is beneficial to design SVMs specifically for functional classification, by introducing functional transformations and function kernels (Williams, et al., J. Proteome Res., 6:2936-2962 (2007); Anderson, and Anderson, Mol. Cell Proteomics, 1:845-867 (2002).
1. Apply functional transformation, projection *P*_{γ*N*}, on each observation *Xᵢ* as *P_{V^{N}}* (*Xᵢ*) = *xᵢ* = (*x*_{*i*1},···,*x_{iN}*) with *Xᵢ* approximated by ${\sum }_{k = 1}^{N} {x}_{ik} {Ψ}_{k} ,$ where {Ψₖ}_{k≥1} is a complete orthonormal basis of the functional space H
2. Build a standard SVM on the coefficients *xᵢ* ∈ *R^{N}* for all *i* = 1,···, *M*.

This procedure is equivalent to working with a functional kernel, *K_{N}*(*xᵢ*,*xⱼ*) defined as *K*(*P_{V^{N}}*(*Xᵢ*),*P_{V^{N}}*(*Xⱼ*)), where *P_{V^{N}}* denotes the projection onto the N-dimensional subspace *V^{N} ∈ H* spanned by {Ψ*ₖ*}_{*k*=1,···,*N*}, and *K* denotes any standard SVM kernel.

Good candidates for the basis functions include the Fourier basis and wavelet bases. If the functional data are known to be nonstationary, a wavelet basis might yield better results than the Fourier basis. Other good choices include B-spline bases, which generally perform well in practice (Rossi and Villa, Neurocomputing, 69:730-742 (2006).

### b. Feature selection

In preferred embodiments, feature selection is applied to the dataset used for classification. It has been shown that reducing the number of variables used for supervised multivariate model building is beneficial for eliminating non-informative data, reducing prediction errors, and simplifying the interpretability of the data analysis results. For example, PLSDA has been successfully combined with variable selection tools such as genetic algorithms (GA) to improve classification results in ¹H-NMR-based metabolomic studies.

Suitable feature selection methods include, but are not limited to, recursive genetic algorithm (GA), recursive feature elimination (RFE), ANOVA feature selection, and simple sub-sampling. Additionally, SVMs such as L1SVM and SVMRW, which are described below, can simultaneously perform classification as well as feature selection.

t2-statistics (Baldi and Long, Bioinformatics, 17(6):509-19 (2001)) is a widely used filter-based feature selection method in bioinformatics, $\frac{{µ}_{+} - {µ}_{-}}{\sqrt{\frac{{δ}_{+}}{{n}_{+}} + \frac{{δ}_{-}}{{n}_{-}}}}$ with degree of freedom $df = \frac{{\left[\left({δ}_{-}^{2}/{n}_{-}\right) + \left({δ}_{+}^{2}/{n}_{+}\right)\right]}^{2}}{\frac{{δ}_{-}^{2} / {n}_{-}}{{n}_{-} - 1} + \frac{{δ}_{-}^{2} / {n}_{-}}{{n}_{-} - 1}}$ Where µ₊, µ₋ are the mean of the feature values of cancer patients and controls, respectively. δ₊, δ₋ are the corresponding standard deviations and *n*₊, *n*₋ are the corresponding patient numbers. Though computationally efficient, filter-based feature selection methods generally achieve inferior prediction performance compared to the wrapper based feature selection methods. Therefore, several feature selection methods based on SVMs, such as the commonly used recursive feature elimination (RFE) method (Guyon, et al., Machine Learning, 46:389-422 (2002)), were applied.

At each RFE iteration, first, an SVM is trained with the currently selected feature set; next, the importance of a feature is measured according to the sensitivity of the cost function $J = \frac{1}{2} {\sum }_{i , j = 1}^{M} {y}_{i} {y}_{j} {α}_{i} {α}_{j} K \left({x}_{i}, {x}_{j}\right) - {{\sum }_{i = 1}^{M} α}_{i}$ with respect to the feature; then, less important features are dropped successively from the remaining feature set. Typically the bottom 10% features are removed at each iteration for efficiency, but empirical experiments suggest removing the bottom feature one at a time for highest accuracy. This procedure is repeated iteratively to study the prediction accuracy as a function of the number of remaining features and the smallest feature set that achieved the highest training accuracy is selected as the final output.
The cost function can be rewritten as $J = \frac{1}{2} {α}^{T} Hα - {α}^{T} {1}_{n}$ and the sensitivity of the cost function to a feature is $dJ \left(k\right) = \frac{1}{2} {α}^{T} Hα - \frac{1}{2} {α}^{T} H \left(-k\right) α$ where *Hand H*(-*k*) are *M* × *M* matrices with ${H}_{ij} = {y}_{i} {y}_{j} K \left({x}_{i}, {x}_{j}\right) and H {\left(-k\right)}_{ij} = {y}_{i} {y}_{j} K \left({x}_{i}\left(-k\right) , {x}_{j} \left(-k\right)\right)$ where *x*(-*k*) means the *k*th feature has been removed from the input vectors. In the case of linear SVM, $dJ \left(k\right) = \frac{1}{2} {\sum }_{i , j = 1}^{M} {α}_{i} {α}_{j} {x}_{ik} {x}_{jk} = \frac{1}{2} {ω}_{k}^{2} .$ The feature whose removal leads to a smaller increase to the cost function, *dJ*(*i*), is marked as less important.

Bradley et al. (Bradley, et al., Machine Learning Proc. Of the 15th International Conference (ICML98), 82-90 (1998)) proposed L1SVM, which minimizes the L1-norm: ${‖ ω ‖}_{L 1} = {\sum }_{k = 1}^{N} \left|{ω}_{k}\right|$ rather than minimizing the L2-norm of the weight vector (or normal of the separating hyperplane) ${‖ ω ‖}_{L 2} = {\sum }_{k = 1}^{N} {ω}_{k}^{2} .$ Thus, the optimization problem becomes: ${min}_{ω , b , ξ} \frac{1}{2} {\sum }_{k = 1}^{N} \left|{ω}_{k}\right| + C {\sum }_{i = 1}^{M} {ξ}_{i} s . t . { y}_{i} \left(ω⋅{x}_{i}+b\right) + {ξ}_{i} \geq 1 , {ξ}_{i} \geq 0 i = 1 , … , M .$ Since the L1-norm is used, the optimal weight vector w is often very sparse, thus L1SVM can simultaneously perform classification as well as feature selection. However, this is only applicable in the case of the linear kernel. Although L1SVM performs well in feature selection, its classification results can be improved by applying the standard L2-norm SVM classifier on the selected feature subset (Weston, et al., J. Machine Leaning Res., 3:1439-61 (2003)). Fast algorithms for solving the L1SVM optimization problem were proposed by Fung & Mangasarian in 2004 (Fung and Mangasarian, Comp. Opt. Appl., 28(2):185-202 (2004)) and Mangasarian in 2007 (Mangasarian, et al., J. Machine Learning Res., 7(2):1517-30 (2007)).

Weston et al. (Weston, et al., Adv. Neural Info. Proc. Sys., (NIPS01), 668-74 (2001)) proposed another SVM related feature selection method that minimizes a generalization error bound, namely the radius to margin distance ratio *R²W².* R² is the radius of the smallest sphere, centered at the origin that contains all $Φ \left({x}_{i}\right) , i = 1 , ⋯ , M ;$ *W²* is the L2 norm of the normal vector to the optimal separating hyperplane.
*R²* and *W²* can be formulated as follows with the introduction of kernel ${K}_{δ} \left({x}_{i}, {x}_{j}\right) = K \left({δx}_{i}, {δx}_{j}\right)$ where matrix $δ = diag \left({δ}_{1},⋯,{δ}_{n}\right) , {δ}_{k} ∈ \left\{0, 1\right\} , k = 1 , ⋯ , n :$ ${R}^{2} \left(β, δ\right) = {max}_{β} {\sum }_{i} {β}_{i} {K}_{δ} \left({x}_{i}, {x}_{i}\right) - {\sum }_{i , j} {β}_{i} {β}_{j} {K}_{δ} \left({x}_{i}, {x}_{j}\right) s . t . {\sum }_{i} {β}_{i} = 1 , {β}_{i} \geq 0 , i = 1 , ⋯ , M$ ${W}^{2} \left(α, δ\right) = {max}_{α} {\sum }_{i} {α}_{i} - \frac{1}{2} {\sum }_{i , j = 1}^{M} {α}_{i} {α}_{j} {y}_{i} {y}_{j} {K}_{δ} \left({x}_{i}, {x}_{j}\right) s . t . {\sum }_{i} {α}_{i} {y}_{i} = 0 , {α}_{i} \geq 0 , i = 1 , ⋯ , M$ The above optimization problem is approximated using gradient descent. At each iteration, the algorithm firstly optimizes *R²*(β,δ) with respect to β, *W²*(α,δ) with respect to α (denoting the optimal solution as α⁰ and β⁰, respectively); next, it minimizes *R²*(α,δ)*W²*(β,δ) with α fixed to α⁰ and β fixed to β⁰ using steepest descent; then, it sets the smallest δ*ₖ* to zero, i.e. removes the corresponding *k*th feature from the feature set. The algorithm repeats the above procedure until only d nonzero elements, δ₁,···,δ*_{d}* are left.

### c. Cross validation

Cross validation (CV) may be applied to test the efficacy of the classifier. Suitable cross validation methods are known in the art and include, but are not limited to, venetian blinds CV, leave-one-out CV (LOOCV), k-fold CV and 52-20 split validation. In k-fold CV the training set is randomly split in k groups of equally distributed positive and negative cases. A classifier is trained on k-1 of the groups and its generalization performance is validated on the remaining group. This process is repeated k times, each time holding out a different validation subset and the average represents the overall generalization. In the second scheme, k-fold cross-validation with test, the data is first randomly split into training and testing sets. A k-fold cross-validation is performed on the training set and the generalization is obtained on the unseen testing set.

### d. Metabolite identification

Metabolites represented by selected features used by the classifier to discriminate between cancer and non-cancer samples can be identified using any known technique. For example, when mass spectrometry data is used as the expression data input into the classifier, metabolites can be identified by finding the closest mass spectral peak matching the selected model feature and the mass can be matched against known metabolites in computer databases, such as the HMDB database. Alternative strategies include the use of accurate mass measurements and accurate tandem mass spectrometry experiments coupled to isotope profile matching.

### IV. Systems and kits

Another embodiment provides a system arranged to determine if levels of detected metabolic biomarkers are indicative of cancer or an increased risk of developing cancer. In one embodiment, the system includes (i) a means for receiving expression data of two or more serum metabolic biomarkers in a sample from a subject, and; (ii) a module for determining whether the data is indicative of cancer or an increased risk for developing cancer. The module can be a trained machine learning classifier capable of distinguishing data from a cancer patient and data from a control subject. The apparatus can also include a means for indicating the results of the determination.

The means for receiving expression data may be a keyboard into which data may be entered manually. Alternatively, the expression data may be received directly from the computer analyzing the expression data, such as the mass spectrometry data miner. The expression data may be received by a wire, or by a wireless connection. The expression data may also be recorded on a storage medium in a form readable by the apparatus. The storage medium can be placed in a suitable reader comprised within the apparatus.

The training, testing and/or expression data from a subject being tested for cancer may be raw data or may be processed prior to being inputted into the computer system. The computer system may comprise a means for converting raw data into a form suitable for further analysis.

The module for determining whether the data is indicative of the presence of cancer can include a machine learning classifier which has been trained by a method disclosed herein such that it is able to distinguish expression data characteristic of a cancer patient from expression data characteristic of a control subject.

The means for indicating the results of the determination may be a visual screen, audio output or printout. The results typically indicate the classification of the expression data and may optionally indicate a degree of certainty that the classification is correct.

The system can include a personal computer. The personal computer can be a laptop or a hand held computer, for example a specifically designed hand held computer, which has the advantage of being readily transportable in the field.

The system includes a computer program. The computer program is capable, on execution by the computer system, of causing the system to perform a method of diagnosis as disclosed herein. The computer program generally includes a machine learning classifier, preferably a support vector machine, which has been trained as disclosed herein, such that it is able to distinguish expression data characteristic of a cancer patient from expression data characteristic of a control subject.

Another embodiment provides a storage medium storing in a form readable by a computer system a computer program disclosed herein. Any suitable storage medium may be used such as a CD-ROM or floppy disk.

Kits for use in the diagnosis of cancer are also provided. The kit can include means for detecting two or more of the disclosed metabolic biomarkers. The means of detection can include a capture surface, such as an array of specific binding reagents such as antibodies or antibody fragments. The kit can include one or more samples of one or more of the disclosed metabolic biomarkers in a container. The metabolic biomarkers provided in the kit can be used as a control or for calibration.

The kit can include instructions for operation in the form of a label or separate insert. For example, the instructions may inform a consumer how to collect a serum sample and how to incubate the sample with the capture surface, or how to prepare he sample for mass spectrometry. The kit may include instructions for inputting expression data of the markers into an apparatus, as disclosed above. The kit can include a storage medium.

### V. Methods for treating cancer

Cancers detected in a subject using the disclosed methods and systems can be treated using any appropriate known method. Exemplary methods for treating cancer include, but are not limited to, surgery, chemotherapy, hormone therapy, radiotherapy and immunotherapy. Standard treatments for ovarian cancer include, but not limited to, surgery, administration of paclitaxel, cisplatin and carboplatin, and radiation treatment.

### Examples

### Example 1. Differential serum metabolomics of human ovarian cancer by liquid chromatography time-of-flight mass spectrometry and genetic algorithm variable selection coupled to partial least squares-discriminant analysis.

### Materials and Methods:

### Materials

Serum samples for LC/TOF MS metabolomic analysis were obtained from 37 patients with ovarian cancer (mean age 60 years, range 43-79 with different cancer stages I-IV) and 35 normal within limit (NWL) controls (mean age 54 years, range 32-84). The patients' information is detailed in Table 1.

**Table 1. Population characteristics of ovarian cancer patients and controls.**

| **Characteristics** | **Ovarian Cancer Patients (n = 37)** | | | **Controls (n = 35)** |
|---|---|---|---|---|
| | Stages I/II/Recurr. (n=8) | Stages III/IV (n = 29) | Percentage (n = 37) | |
| Age (y), mean (range) | 60 (43-74) | 61 (44-79) | | 54 (32-84) |
| ***Stages*** | | | | |
| I | 4 | - | 10.8 | |
| II | 2 | - | 5.4 | |
| III | - | 27 | 73.0 | |
| IV | - | 2 | 5.4 | |
| Recurr. | 2 | - | 5.4 | |
| ***Grades*** | | | | |
| 1 | 0 | 3 | 8.1 | |
| 2 | 1 | 7 | 21.6 | |
| 3 | 5 | 16 | 56.8 | |
| Ungraded | 2 | 3 | 13.5 | |
| ***Histological Types*** | | | | |
| Papillary Serious | 4 | 19 | 62.2 | |
| Endometrioid | 1 | 1 | 5.4 | |
| Others (Mixed, Transitional) | 0 | 6 | 16.2 | |
| Mucinous | 0 | 1 | 2.7 | |
| Clear Cell | 0 | 1 | 2.7 | |
| Serious Cyst | 0 | 1 | 2.7 | |
| Primary Peritoneal | 3 | 0 | 8.1 | |

All serum samples were obtained from the Ovarian Cancer Institute (OCI, Atlanta, GA) after approval by the Institutional Review Board (IRB). All donors were required to fast and to avoid medicine and alcohol for 12 hours prior to sampling, except for certain allowable medications, for instance, diabetics were allowed insulin. Following informed consent by donors, 5 mL of whole blood were collected at Northside Hospital (Atlanta, GA) by venipuncture from each donor into evacuated blood collection tubes that contained no anticoagulant. Serum was obtained by centrifugation at 5000 rpm for 5 minutes at 4° C. Two hundred and fifty µL aliquots of serum samples were frozen with dry ice immediately after centrifugation, and stored at -80° C for further use. The sample collection and storage procedures for both ovarian cancer patients and healthy individuals were identical. All chemicals were obtained from Sigma-Aldrich (St. Louis, MO) and used without further purification. All aqueous solutions were prepared with nanopure water (dH₂O) from a Nanopure Diamond laboratory water system (Barnstead International, Dubuque, Iowa).

### Serum sample pretreatment for LC/TOF MS analysis

The metabolomic investigation strategy followed in this study is depicted in Figure 1. Serum samples were thawed, and proteins precipitated by addition of acetonitrile to the serum sample in a 5:1 ratio (1000 µL acetonitrile + 200 µL serum), the mixture was vortexed for 1 minute and incubated at room temperature for 40 minutes, then the sample was centrifuged at 13,000g for 15 minutes and the supernatant retained. This supernatant solution was vacuum evaporated and the residue reconstituted in 80% acetonitrile/0.1% TFA immediately prior to LC/TOF MS analysis. Every ovarian cancer serum sample was randomly paired with a normal sample and run on the same day to ensure that no temporal bias was introduced in the way samples were analyzed. Sample pairs were run in random order and in duplicate.

### Liquid chromatography electrospray ionization time-of-flight mass spectrometric analysis

LC/TOF MS analyses were performed on a JEOL AccuTOF (Tokyo, Japan) mass spectrometer coupled via a single-sprayer ESI ion source to an Agilent 1100 Series LC system (Santa Clara, CA). The TOF resolving power measured at FWHM was 6000, and the observed mass accuracies ranged from 5-15 ppm, depending on signal-to-noise ratios (S/N) of the particular ion investigated. The LC system was equipped with a solvent degasser, a binary pump, a thermostatic column compartment (held at 25° C), and an autosampler. The injection volume was 15 µL in all cases. Reverse phase separation of preoperative serum samples was performed using a Symmetry^{®} C₁₈ column (3.5 µm, 2.1 × 150 mm, pore size 100 Å; Waters, Milford, MA) at a flow rate of 150 µL min⁻¹, the analytical column was preceded by a Zorbax^{®} RX-C₁₈ guard column (5.0 µm, 4.6 × 12.5 mm, pore size 2 µm; Agilent). The LC solvent mixtures used were: A = 0.1% formic acid in water and B = 0.1% formic acid in acetonitrile. After a pre-run equilibration with 5% B for 5 minutes, data acquisition was started and the solvent composition was varied according to the solvent program described in Table 2.

**Table 2. LC solvent gradient used in metabolomic experiments.**

| | Time (min) | %B (acetonitrile/ 0.1 % formic acid) | Flow Rate (µL min⁻¹) |
|---|---|---|---|
| **Pre-Run** | | | |
| | 0.0 | 100 | 300 |
| | 10.0 | 5 | 150 |
| | 15.0 | 5 | 150 |
| **Run** | | | |
| | 0.0 | 5 | 150 |
| | 5.0 | 5 | 150 |
| | 10.0 | 20 | 150 |
| | 20.0 | 25 | 150 |
| | 28.0 | 30 | 150 |
| | 38.0 | 35 | 150 |
| | 50.0 | 40 | 150 |
| | 90.0 | 45 | 150 |
| | 100.0 | 50 | 150 |
| | 110.0 | 60 | 150 |
| | 120.0 | 75 | 150 |
| | 130.0 | 85 | 150 |
| | 160.0 | 95 | 150 |
| | 180.0 | 100 | 150 |
| **Post-Run** | | | |
| | 0.0 | 100 | 300 |
| | 30.0 | 100 | 300 |

After analysis of a given serum specimen, a 0.20 mM sodium trifluoroacetate standard (NaTFA) (Moini, et al., J. Am. Soc. Mass Spectrum., 9:977-980 (1998)) was run for mass drift compensation purposes. For NaTFA analysis, 100% B at a flow rate of 300 µL min⁻¹ was used as the LC solvent, and data was acquired for only 10 minutes, sufficient for collecting a reference spectrum. After injection of the drift correction standard, the column was washed with 100% B for 30 minutes. To ensure maximum reproducibility in metabolomic experiments, all serum specimens were run consecutively within a 2.5 month period.

Spectral data was collected in the 100-1750 *m*/*z* range, with a spectral recording interval of 1.5 s, and a data sampling interval of 0.5 ns for both positive and negative ion ESI modes. The settings for the TOF mass spectrometer for positive or negative ion mode were as follows: needle voltage: +/- 2000 V, ring lens: + 8 V or - 9 V, orifice 1: + 30 V or - 69 V, orifice 2: + 6 V or - 8 V, desolvation chamber temperature: 250° C, orifice 1 temperature: 80° C, nebulizing gas flow rate: 1.0 L min⁻¹, desolvation gas flow rate 2.5 L min⁻¹, and detector voltage +/- 2800 V. eTOF analyzer pressure was ~4.8 × 10⁻⁶ Pa during analysis. The RF ion guide voltage amplitude was swept to ensure adequate transmission of analytes in a wide range of *m*/*z* values. The sweep parameters were as follows: initial peaks voltage: 700 V, initial time: 20%, sweep time: 50%, final peaks voltage: 2500 V. After LC/TOF MS data was collected, it was centroided, mass drift corrected using the NaTFA reference spectrum, and exported in NetCDF format for further mining.

### LC/TOF MS data mining

All data were mined identically and simultaneously. Data mining was performed by loading NetCDF files into mzMine (v0.60, http://mzming.sourceforge.net). Data were smoothed by chromatographic median filtering with a tolerance in *m*/*z* of 0.1, and one-sided scan window length of 3 s. Peaks were picked with a *m*/*z* bin size of 0.15, chromatographic threshold level of 0%, absolute noise level of 200, absolute minimum peak height of 250, minimum peak duration of 5 s, tolerance for *m*/*z* variation of 0.06, and tolerance for intensity variation of 50%. The method for de-isotoping was to assume +1 charge states, and monotonic isotopic patterns. The retention time tolerance (RT) for de-isotoping was 65 s and the *m*/*z* tolerance 0.07. The chromatographic peak alignment *m*/*z* tolerance was 0.2, and the RT tolerance was 12%, with a balance coefficient between *m*/*z* and RT of 30. The minimum number of detections for rare peak filtering in the alignment results was set to 41. Spectral features not initially detected by the peak detection algorithm were subsequently added by a gap filling method using an intensity tolerance of 30%, *m*/*z* tolerance size of 0.2, and RT tolerance size of 12%. Systematic drift in intensity levels between different data files was corrected for by linear intensity normalization using the total raw signal. After the normalized alignment file containing all peak intensities was created, peak areas were exported to Excel and peaks of contaminants, dimers, redundant adducts, and isotopes not adequately detected were removed. Approximately 37% of the peaks from positive mode and 18% of the peaks from negative mode were eliminated after this filtering. Peak areas from duplicate runs were then averaged, and positive and negative mode ESI data were exported as ASCII files into Matlab (R2007a, The Mathworks, Natick, MA).

### Genetic algorithm variable selection and partial least squares discriminant analysis

GA variable selection and PLSDA analysis were performed with the PLS Toolbox for Matlab (v4.1, Eigenvector Technologies, Wenatchee, WA). GA-PLSDA multivariate models using combined positive and negative ion mode data were created by appending the respective data matrices. This appended dataset is referred to as "multimode ionization data". Genetic algorithms were run using the "genalg" function with the following parameter settings: window width: 1, mutation rate 0.005, and PLS regression with a maximum number of 8 latent variables. Random-type cross-validation was used with 7 splits (10 samples in each split) and 4 iterations. PLSDA was performed using the "analysis" graphical user interface from the PLS Toolbox for Matlab, with autoscaled data, and venetian blinds cross-validation (8 splits, 9 samples per split).

### Metabolite identification

Due to the biological complexity of serum samples, adduct ion analysis was first performed to ensure the unambiguous assignment of the signal of interest in the mass spectrum. Adducts formed in positive ion mode ESI usually includes [M+H]⁺, [M+NH₄]⁺, [M+Na]⁺, [M+K]⁺, [M-H₂O+H]⁺ and [2M+H]⁺, while adduct and dimer formation in negative ion mode ESI includes [M-H]⁻, [M+CH₃COO]⁻, [M+Cl]⁻, [M+HCOO]⁻ and [2M-H]⁻. First, each centroided spectrum of interest was fully calibrated using the NaTFA standard run acquired immediately after the sample. Adducts in centroided mass spectra corresponding to GA-selected variables were identified by manually calculating the differences between the exact *m*/*z* values of peaks within the spectrum and comparing these differences to those between the common adduct species mentioned above. For spectra in which multiple adducts were not present, the accurate mass of the candidate neutral molecule was calculated based on the assumption that the peak of interest corresponded to either [M+H]⁺, [M+Na]⁺, or [M+NH₄]⁺ in positive ion mode and [M-H]⁻, [M+CH₃COO]⁻, [M+HCOO]⁻, or [M-CH₃]⁻ (for glycerophosphocholines) in negative ion mode yielding multiple possible neutral molecular masses for each *m*/*z* value.

Elemental formulae were estimated from the accurate mass spectra using a system of macros developed and freely distributed by Fiehn, et. al. (Kind and Fiehn, BMC Bioinformatics, 8:105-125 (2007)) which relies on a series of heuristic rules to identify possible formulae based on the mass accuracy of the peak of interest, as well as the corresponding isotopic ratios, while excluding unlikely formulae. The mass of the neutral molecule and relative isotopic abundances were imported directly into the "seven golden rules" Excel spreadsheet (http://fiehnlab.ucdavis.edu/projectslSeven_Golden_Rules/). The mass accuracy was set to 15 ppm, and the threshold for error in the relative isotopic abundances was set to 10%. The list of elements to include in the search was constrained to include C, H, N, O, P, S, Cl, and Br. The limits set for these elements were *m*/*z* dependent, and were automatically determined in a heuristic manner using formulas derived from examination of the Dictionary of Natural Products (DNP) and Wiley mass spectral databases (Kind and Fiehn, BMC Bioinformatics, 8:105-125 (2007)). The probability of a given formulae being the "correct" one is provided as a score calculated from the error rates in satisfying the aforementioned rules. In addition, each formula is automatically compared to the PubChem (http://pubchem.ncbi.nlm.nih.gov/), DNP (http://ccd.chemnetbase.com/) and Metabolome.jp databases (www.metabolome.jp/), and the top hits found in each of these databases is highlighted by the software. The top hits in the list of filtered elemental formulae and all accurate mass values obtained were searched in the following databases: METLIN (http://metlin.scripps.edu/), KEGG (www.genome.jp), HMDB (www.hmdb.ca/), MMCD (http://mmcd.nmrfam.wisc.edu/) and Lipid Maps (http://www.lipidmaps.org/) in order to determine the greatest possible number of candidate molecules. The criteria used for the assignment of a tentative chemical structure were: a mass difference with the simulated formula lower than 15 ppm, isotope abundance errors less than 10%, and that the candidate found in the database corresponded to an endogenous metabolite (i.e. a small molecule that participates in cellular metabolism as an intermediate or product).

### Results:

### LC/TOF MS-based metabolomic analysis of human serum samples

Metabolomic investigation of sera from patients with ovarian cancer and healthy women using LC/TOF MS revealed a total of 576 features extracted by mzMine in positive ion mode, and 280 in negative ion mode. The data was found to be highly complex, with numerous features across both analytical dimensions. Decreasing the absolute noise level and minimum peak height from 400 and 500 to 200 and 250 increased the number of detected features to 4439 and 329 for positive and negative ion modes respectively. While this allowed a "deeper dig" into the serum metabolome, the number of features consistently detected across samples decreased to 3.6% and 15%, respectively. A 3-D serum metabolic profile for a typical stage III ovarian cancer serum sample is displayed in Figure 2A demonstrating the capability of LC/TOF MS to resolve hundreds of compounds in a wide mass range within 180 minutes. Despite the shallow solvent gradient chosen for the LC run, there is still evidence of co-elution as evidenced by the projection of Figure 2A onto the chromatographic axis (Figure 2B). However, in most cases, the high resolving power of the TOF mass analyzer allowed the resolution of these signals by their selected monoisotopic ion chromatograms, as shown in Figure 2C for an ion with *m*/*z*=443.26 at a window width of 0.05 Da. The corresponding centroided negative ion mode spectrum obtained at 91 minutes is shown in Figure 2D. Due to the obvious complexity of these samples, the reproducibility of the LC/TOF MS approach was tested in early experiments to rule out column memory effects. Lipids, fatty acids and other hydrophobic components in sera that are easily adsorbed onto the reverse phase column can act as a new stationary phase, causing a change in selectivity, memory effects, and shifting retention times. Figures 3 and 4 show total ion chromatograms corresponding to 4 identical samples prepared in an identical fashion. The results demonstrate that good reproducibility was possible at the chosen flow rate of 300 µL min⁻¹.

In contrast to gas chromatography-mass spectrometry (GC-MS), where unsupervised compound identification is possible by direct comparison of each electron ionization spectrum with existing databases (e.g. the US National Institute of Standards and Technology database), compound identification in LC-MS experiments is more complex for two reasons: (a) the formation of various adducts and dimers with varying abundances (a function of the LC solvents and the desolvation conditions used), and (b) the extent to which different ESI sources impart varying degrees of internal energy to the observed ions, producing fragmentation of labile species, most commonly dehydration. For these reasons, compound identification was attempted *a posteriori*, only for spectral features observed to be significant in multivariate classification models.

### Exploratory PCA analysis and variable selection by genetic algorithms

Following LC/TOF MS analysis and data mining (Figure 1), PCA was used as an exploratory tool to investigate any noticeable differences in the ovarian cancer and control datasets in multivariate space. In PCA, the experimental variable space is reduced into the more easily visualized space of principal components (PCs), which are weighted sums of the original variables. Examination of the PCA score plots on the first three PCs for positive, negative and multimode ionization data showed no obvious separation between the objects. Development of PCA models with up to 20 PCs, still revealed no significant differences in the scores for the two object classes. This result was not surprising, given that PCA is known to be sensitive to noisy datasets, and is only able to detect large changes in the *X* block (Rousseau, et al., Chemom. Intell. Lab. Syst., 91:54-66 (2008)).

A GA-based evolutionary variable selection strategy was employed next to investigate if removal of uninformative spectral features from the *X* block followed by supervised clustering would lead to better discrimination between object classes. The biological complexity of ovarian cancer suggests that individual biomarkers may have limited diagnostic sensitivities and specificities. Instead, evolutionary selection of several biomarkers in the form of a panel could offer enhanced classification power. The GA was first applied to data obtained in each ionization mode separately and, in a second stage of analysis, to the dataset formed by appending the spectral features observed in both ionization modes. This was done under full crossvalidation conditions to prevent overfitting, and avoid local fitness maxima. The fitness criterion was the minimization of the root mean square error in crossvalidation (RMSECV) for PLSDA classification of samples in the "ovarian cancer" and "control" classes. Ten replicate runs of a recursive GA were conducted starting with an average of 15% initial terms for negative ion data and 10% for positive and multimode ionization data. In all cases, the GA was initialized with an initial population of 256 spectral features or "chromosomes" and run for a maximum of 150 generations, or until the percentage of identical variables in the population reached 90%. The crossvalidation conditions chosen resulted in a single chromosome being evaluated 28 times. For a typical GA run (Figure 5), it was observed that the fitness rapidly improved (RMSECV decreased) after 20 generations (Figure 5B), which was followed by a rapid decrease in the average number of variables used in each chromosome (Figure 5C). The initial average RMSECV was in all cases quite high, ranging between 0.7-0.8. This is in agreement with the PCA analysis for datasets including all variables which showed no clustering between classes. The final RMSECV value after GA variable selection was much lower than the initial one, reaching an average of 0.22 for the particular run shown in Figure 5, but lower for other runs, as described below. Interestingly, for the variable selection run presented in Figure 5C, the number of average variables remains approximately constant (~40) after 60 generations, but a decrease in RMSECV is still observed (Figure 5B), indicating that at that stage, crossover of the variables in the chromosome pool results in further improvement of the average fitness. The outcome of each GA run is a set of "chromosomes" with 90% similarity in the included variables, and with varying degrees of success in classifying ovarian cancer and control objects. Figure 5A shows the fitness observed for the final pool of "chromosomes" selected after 150 generations in this particular GA run on multimode ionization data. An analysis of the frequency of inclusion of distinct variables in these "chromosomes" showed that, as expected, a large number of variables are completely excluded in order to decrease classification error.

The resulting fitness of the chromosome pool after 10 GA iterations (150 generations each) on the multimode ionization data is shown in Figure 6. Most classification models using these "chromosomes" were based on 6-8 latent variables (LVs). The highlighted "chromosome" (red box) consisted of 37 selected variables with RMSECV=0.138, and was chosen for all subsequent clustering based on multimode ionization data. Inspection of the GA-selected variables showed very little redundant information, with only one metabolite present as a redundant adduct. Similar GA-selection and spectral inspection procedures were followed for datasets including only positive or negative ion mode mass spectral data, but the classification error was higher in these cases (0.245 for the best positive ion mode model and 0.163 for the best negative ion mode model).

### Examination of PLSDA classification models

PLSDA is a partial least squares regression aimed at predicting several binary responses *Y* from a set *X* of descriptors (Rousseau, et al., Chemom. Intell. Lab. Syst., 91:54-66 (2008)). Examples of *X* descriptors include bucketed ¹H-NMR spectral regions, and GC-MS or LC-MS spectral features identified by (retention time (RT), *m*/*z*) pairs. PLSDA lies midway between the traditional discriminant analysis on the original variables and a discriminant analysis on the significant principal components of the *X* descriptors. Compared with PCA, PLSDA attempts to capture "among-group" and "within-group" differences of the investigated data rather than seeking to capture the maximum variance in the *X* block independently of the *Y* block. Unlike PCA, which uses the total spectral variance to discriminate between groups, PLSDA relies on the use of classes, or *Y* binary responses, which maximizes the ability of the model to discriminate between disease and control objects (Massart, et al., Handbook of chemometrics and qualimetries, Elsevier: Amsterdam (1997)).

Supervised classification models were created using the best subset of GA-seleeted features for positive, negative and multimode electrospray datasets. Figure 7 describes the change in crossvalidation classification error as a function of the number of latent variables used in the construction of PLSDA models and the signal-to-noise ratio (SNR) of each LV. The smallest number of LVs that produced a minimum in the CV error in Figures 7A, 7B and 7C was 6 in all cases. The multimode ionization PLSDA model had the highest overall SNR for all LVs. PLSDA models using LVs with SNR lower than 2 were not tested, to avoid modeling noise. The multimode ion mode PLSDA model (Figure 7C) had the lowest crossvalidation classification error after 3 LVs were added, as it combines the largest amount of spectral information, and was therefore selected as the most promising approach for all further investigations.

During the PLSDA model building stage (training), the *Y* value of each object (i.e. serum sample) is assigned as either 0 (controls) or 1 (ovarian cancers), depending on its class membership. A plot of the PLSDA model predictions of class membership for serum samples of all cancer stages under calibration conditions using multimode ionization data is shown in Figure 8A. As it can be seen from this figure, no false positives or false negatives were detected in this dataset, which includes 4 stage I and 2 stage II ovarian cancer samples. Data dispersion in the *Y* axis reflects the goodness of fit of the PLSDA model. The discriminant *Y* value (i.e. decision threshold), was calculated by the PLS toolbox based on Bayesian statistics, and used to determine whether a future unknown belongs to a given class or not. Figure 9 displays the PLSDA score plot on the first three LVs for this model. As can be observed, the separation in multivariate space of the two object classes was complete within the first three LVs. Addition of the 4^{th}, 5^{th} and 6^{th} LVs further improved the overall classification under crossvalidation conditions (Figure 7C) and thus the 6-LV structure was preserved. Calibration was accompanied by Venetian-blinds crossvalidation. Figure 8B shows the predicted *Y* value for each object during crossvalidation. In this case, the dispersion in *Y* predicted values was larger than for the case shown in Figure 8A, as 8 consecutive subsets containing 12.5% of the samples (n=9 each) are sequentially removed from the model and predicted with a PLSDA structure created from the remaining objects. No misclassifications were observed during crossvalidation using multimode ionization data. Tables 3-5 detail the performance of PLSDA models using various ion mode datasets. For tables 3-5, crossvalidation: Venetian blinds w/ 8 splits. Preprocessing: autoscaling. Number of latent variables: 6.

**Table 3. PLS-DA results of all samples with different ESI modes by using selected features from GA: ESI positive data.**

| **Statistics for *Y*-Block** | | | **Percent Variance Captured by Regression Model** | | | | |
|---|---|---|---|---|---|---|---|
| *Modeled Class* | *OC* | *Control* | | *X-Block* | | *Y-Block* | |
| Sensitivity (Cal) | 0.972 | 1.000 | Comp | This | Total | This | Total |
| Specificity (Cal) | 1.000 | 0.972 | 1 | 9.01 | 9.01 | 46.52 | 46.52 |
| Sensitivity (CV) | 0.972 | 1 | 2 | 11.14 | 20.15 | 22.00 | 68.52 |
| Specificity (CV) | 1 | 0.972 | 3 | 9.66 | 29.81 | 13.66 | 82.18 |
| Class Err (Cal) | 0.014 | 0.014 | 4 | 6.12 | 35.93 | 4.42 | 86.60 |
| Class Err (CV) | 0.014 | 0.014 | 5 | 5.35 | 41.29 | 2.18 | 88.78 |
| RMSEC | 0.160 | 0.160 | 6 | 7.11 | 48.40 | 0.77 | 89.55 |
| Number of LVs | 6 | | | | | | |

**Table 4. PLS-DA results of all samples with different ESI modes by using selected features from GA: ESI negative data.**

| **Statistics for *Y*-Block** | | | **Percent Variance Captured by Regression Model** | | | | |
|---|---|---|---|---|---|---|---|
| *Modeled Class* | *OC* | *Control* | | *X-Block* | | *Y-Block* | |
| Sensitivity (Cal) | 1.000 | 1.000 | Comp | This | Total | This | Total |
| Specificity (Cal) | 1.000 | 1.000 | 1 | 9.18 | 9.18 | 50.83 | 50.83 |
| Sensitivity (CV) | 1.000 | 1.000 | 2 | 13.37 | 22.55 | 21.10 | 71.93 |
| Specificity (CV) | 1.000 | 1.000 | 3 | 5.23 | 27.78 | 12.76 | 84.69 |
| Class Err (Cal) | 0 | 0 | 4 | 5.04 | 32.82 | 6.74 | 91.43 |
| Class Err (CV) | 0 | 0 | 5 | 4.94 | 37.76 | 3.28 | 94.71 |
| RMSEC | 0.097 | 0.097 | 6 | 3.14 | 40.89 | 1.52 | 96.22 |
| Number of LVs | 6 | | | | | | |

**Table 5. PLS-DA results of all samples with different ESI modes by using selected features from GA: ESI multimode data.**

| **Statistics for *Y*-Block** | | | **Percent Variance Captured by Regression Model** | | | | |
|---|---|---|---|---|---|---|---|
| *Modeled Class* | *OC* | *Control* | | *X-Block* | | *Y-Block* | |
| Sensitivity (Cal) | 1.000 | 1.000 | Comp | This | Total | This | Total |
| Specificity (Cal) | 1.000 | 1.000 | 1 | 6.92 | 6.92 | 58.49 | 58.49 |
| Sensitivity (CV) | 1.000 | 1.000 | 2 | 9.82 | 16.75 | 20.75 | 79.23 |
| Specificity (CV) | 1.000 | 1.000 | 3 | 7.19 | 23.94 | 11.44 | 90.67 |
| Class Err (Cal) | 0 | 0 | 4 | 5.53 | 29.47 | 4.03 | 94.70 |
| Class Err (CV) | 0 | 0 | 5 | 6.35 | 35.82 | 1.76 | 96.46 |
| RMSEC | 0.082 | 0.082 | 6 | 5.22 | 41.05 | 0.87 | 97.33 |
| Number of LVs | 6 | | | | | | |

The multimode ionization PLSDA model with 6 LVs outperformed other models, with 100% sensitivity (probability that a subject with ovarian cancer will have a positive test result) and selectivity (probability that a subject without cancer will show a negative test result) under crossvalidation conditions, minimum root mean square error of calibration (RMSEC) and maximum *Y* block explained variance. The two single ionization mode PLSDA models performed quite differently (Tables 3 and 4). The positive ion mode model showed the lowest sensitivity of the two (97.2%). As a final test of the performance of the multimode ionization PLSDA model, 33% of the samples of each class (n=24) were randomly chosen regardless of cancer stage, and completely excluded from the model building process, thus effectively treated as unknowns. The prediction results of this external test set are shown in Figure 8C, showing the potential of the metabolomic GA-PLSDA LC/TOF MS approach applied to serum samples for ovarian cancer diagnostics.

Following PLSDA classification, the metabolite peak areas were individually tested to investigate if statistical differences between these species were detected. The robust non-parametric Wilcoxon rank sum test was applied to the metabolites selected by GA. Tables 6 and 7 show the p-values for each individual metabolite. A non-parametric test was chosen in order to avoid the assumption of normally-distributed data. Interestingly, only 27% of the multimode variables were statistically significant when considered in a univariate fashion. This suggests that the PLSDA model is capturing a pattern or "metabolic fingerprint" rather than the univariate change in a single metabolite.

### Metabolite identification

The calculated neutral masses, species investigated, and retention times of the positive and negative ion mode ESI variables used by the multimode PLSDA model, as well as their corresponding chemical formulae, mass differences (Δm), and matching scores, are reported in Tables 6 and 7, respectively.

**Table 6: GA-selected variables for multimode ionization dataset detected in positive ion ESI via accurate mass, isotope cluster matching and metabolite database searches (at most, the top-five matching formulae are listed).**

| Neutral Mass (Da) | Species Invest. | RT (min) | Wilcoxon (p=0.05) | Estimated Formulae (in order of decreasing score) | Mass Accur. (ppm) | Score (%) | Potential Metabolite(s) Identified | Source |
|---|---|---|---|---|---|---|---|---|
| 187.0614 | [M+H]⁺ | 6.4 | NS | C₉H₈F₃N, C₇H₅N₇, C₆H₉N₃O₄, C₄H₉N₇S, C₁₁H₉NO₂ | 3.1-11.6 | 96.8-95.2 | Not Identified | |
| 278.1434 | [M+Na]⁺ | 116.8 | 0.01 | C₁₆H₂₃O₂P, C₁₁H₂₃N₂O₄P, C₈H₁₄N₁₂, C₁₈H₁₈N₂O, C₁₃H₂₇O₂PS | 0.6-13.9 | 98.4-93.7 | Not Identified | |
| 278.1615 | [M+H]⁺ | 140.4 | 0.01 | C₁₅H₂₂N₂O₃ | 5.4 | 88.3 | Phe-Ile | MID 23716^{a} |
| 369.2999 | [M+H]⁺ | 50.4 | NS | C₂₀H₃₉N₃O₃, C₂₅H₃₉NO | 2.1-8.8 | 88.6-84.7 | Not Identified | |
| 453.2867 | [M+H]⁺ | 105.6 | NS | C₂₁H₄₄NO₇P | 2.6 | 93.0 | PE(16:0/0:0) | LMGP 02050002^{b} |
| 456.2856 | [M+H]⁺ | 119.4 | NS | C₂₃H₄₀N₂O₇, C₁₉H₃₆N₈O₅, C₂₈H₃₆N₆O₃, C₂₇H₄₁N₂O₂P, C₂₈H₄₀O₅ | 1.6-10.8 | 94.5-89.0 | Not Identified | |
| 467.295 | [M+H]⁺ | 82.3 | 0.01 | C₂₂H₄₆NO₇P | 12.2 | 93.6 | PC(14:0/0:0) | LMGP 01050012^{c} |
| 485.3773 ¹ | [M+Na]⁺ | 110.1 | 0.05 | C₂₇H₅₁NO₆, C₂₆H₅₁N₃O₅, C₂₈F₄₇N₅O₂, C₂₇R₄₇N₇O, C₃₃H₄₇N₃ | 0.7-11.7 | 74.7-68.8 | Not Identified | |
| 490.3327 ¹ | [M+NH₄]⁺ | 110.1 | 0.05 | C₂₇H₄₇N₄PS, C₂₄H₄₆N₂O₈, C₂₃H₄₇N₄O₅P, C₂₅H₄₂N₆O₄, C₂₄H₄₂N₈O₃ | 8.8-14.9 | 78.0-_{'} 74.5 | Not Identified | |
| 495.3380 | [M+H]⁺ | 106.8 | NS | C₂₄H₅₀NO₇P | 11.2 | 96.6 | PCA(16:0/0:0) | LMGP 01050018^{d} |
| 507.3592 | [M+H]⁺ | 110.1 | 0.05 | C₂₄H₄₅N₉O₃, C₂₉H₄₉NO₆, C₂₉H₅₀ClN₃O₂, C₃₀H₄₅N₅O₂, C₃₅H₄₅N₃ | 0.1-10.5 | 74.2-67.7 | Not Identified | |
| 517.3238 | [M+H]⁺ | 88.9 | NS | C₂₆H₄₈NO₇P | 13.4 | 91.6 | PC(18:3(9Z,12Z,15Z)/ 0:0[U]) | LMGP 01050012^{e} |
| 519.3070 | [M+Na]⁺ | 98.1 | NS | C₂₄H₄₆N₃O₇P, C₂₂H₃₇N₁₁O₄, C₂₆H₄₅NO₁₀, C₂₆H₄₁N₅O₆, C₂₇H₃₇N₉O₂ | 0.01-7.7 | 96.9-90.3 | Not Identified | |
| 521.3220 | [M+H]⁺ | 111.2 | NS | C₂₅H₄₇NO₁₀, C₂₆H₄₃N₅O₆, C₂₉H₄₈NO₅P, C₃₁H₄₃N₃O₄, C₃₂H₃₉N₇ | 1.5-9.4 | 93.5-83.3 | Not Identified | |
| 525.2924 | [M+H]⁺ | 103.2 | NS | C₂₁H₄₄N₅O₈P, C₂₇H₄₃NO₉, C₂₈H₃₈N₅O₅, C₃₀H₃₅N₇O₂, C₃₆H₄₃N₃O₆S | 0.6-13.7 | 92.6-80.1 | Not Identified | |
| 632.2342 | [M+H]⁺ | 53.6 | NS | C₂₃H₄₀N₂O₁₈ | 10.5 | 95.3 | 3-sialyllactosamine | HMDB 06607^{f} |
| 757.5572 | [M+Na]⁺ | 152.8 | NS | C₄₂H₈₀NO₈P | 6.6 | 82.8 | PE-NMe(18:1(9E)/18:1(9E )) | LMGP 02010331^{g} |
| 759.5895 | [M+H]⁺ | 134.8 | 0.03 | C₄₇H₈₃Cl₂N₃, C₄₈H₈₃Cl₂NO, C₄₅H₈₈Cl₂NOP, C₄₃H₈₃Cl₂N₃O₃, C₄₂H₄₈Cl₂N₅P | 5.7-11.2 | 90.2-85.9 | Not Identified | |

| | | | | | | | | |
|---|---|---|---|---|---|---|---|---|
| ¹ possible adduct species for ion with *mlz* 508.3362. ^{a} Three other isomers found for this candidate including: MID 23831, MID 24033, MID 24020. ^{b} Multiple isomers found for this candidate in Lipid Maps including LMGP 01050001, and 01050011. ^{c} Multiple isomers found for this candidate in Lipid Maps including LMGP 01020009, 01050013, 01050073, and 01020010. ^{d} Multiple isomers found for this candidate in Lipid Maps including LMGP 01020019, 01020020, 01050019, 01050020, 01050074, 01050075, 01050113,01050118, and 01050119. ^{e} Multiple isomers found for this candidate in Lipid Maps including LMGP 01050037, and 03050035, ^{f} An additional isomer (MMCD cq_12636) was found for this candidate. ^{g} Thirty one additional records for isomeric structures found in Lipid Maps. | | | | | | | | |

**Table 7: GA-selected variables for multimode ionization dataset detected in negative ion ESI via accurate mass, isotope cluster matching and metabolite database searches (at most, the top-five matching formulae are listed).**

| Neutral Mass (Da) | Species Investigated | RT (min) | Wilcoxon (p=0.05) | Estimated Formulae (in order of decreasing score) | Mass Accur. (ppm) | Score (%) | Name | Source |
|---|---|---|---|---|---|---|---|---|
| 256.2398 | [M-H]⁻ | 104.7 | NS | C₁₆H₃₂O₂ | 1.7 | 96.3 | Palmitic acid | HMDB 00220 |
| 304.2407 | [M-H]⁻ | 100.0 | NS | C₂₀H₃₂O₂ | 1.5 | 74.8 | Arachidonic acid | HMDB 01043^{a} |
| 304.2512 | [M-H]⁻ | 132.7 | NS | C₁₉H₃₂N₂O, C₁₇H₃₇O₂P, C₁₆H₃₆N₂OS | 0.8-11.9 | 96.1-88.9 | Not Identified | |
| 306.3145 | [M-H]⁻ | 135.8 | NS | C₂₁H₃₉N, C₂₂H₄₁, C₂₀H₃₇N₂, C₂₁H₃₇O, C₁₉H₃₅N₃ | 19.8-51.8 | 98.9 | Not Identified | |
| 308.2881 | [M-H]⁻ | 141.3 | NS | Not Found | | | | |
| 308.1377 | [M+CH₃CO O]⁻ | 85.5 | 0.05 | C₁₉H₂₀N₂S, C_{2O}H₂₀O₃, C₁₃H₂₀N₆OS, C₁₆H₁₆N₆O, C₁₅H₂₀N₂O₅ | 1.6-11.5 | 97.1-92.1 | Not Identified | |
| 322.1534¹ | [M+HCOO]⁻ | 85.5 | 0.05 | C₁₄H₂₂N₆OS, C₂₀H₂₂N₂S, C₁₇H₁₈N₆O, C₂₁H₂₂O₃, C₂₁H₂₃OP | 2.5-14.8 | 95.8-94.9 | Not Identified | |
| 354.1682 | [M-H]⁻ | 36.9 | 0.04 | C₁₄H₂₂N₆O₅ | 8.6 | 95.4 | Gin His Ala | MID 23091 |
| 368.1588¹ | [M-H]⁻ | 85.5 | 0.05 | C₁₅H₂₄N₆O₃S, C₁₈H₂₀N₆O₃, C₁₇H₂₄N₂O₇, C₂₂H₂₄O₅, C₂₂H₂₅O₃P | 1.2-12.7 | 96.2-94.3 | Not Identified | |
| 428.3340 | [M+HCOO]⁻ | 143.1 | NS | C₂₈H₄₄O₃ | 11.5 | 90.6 | 4a-Carboxy-4b-methyl-5a-cholesta-8,24-dien-3b-oil ercalcitriol | HMDB 01181 HMDB 06225 |
| 453.2861 | [M-H]⁻ | 82.3 | 0.05 | C₂₁H₄₄NO₇P | 1.2 | 80.9 | PE(16:0/0:0) | LMGP 02050002 |
| 470.2904² | [M+CH₃CO O]⁻ | 110.9 | NS | C₁₉H₂₄N₄O₈, C₂₂H₄₈O₆P₂, C₂₁H₃₈N₆O₆, C₂₄H₄₃N₂O₅P, C₂₅H₄₂O₈ | 1.2-10.9 | 98.8-93.1 | Not Identified | |
| 481,2914 | [M-H]⁻ | 108.0 | NS | C₂₃H₃₉N₅O₆, C₂₄H₃₅N₉O₂, C₂₆H₄₄NO₅P, C₂₇H₄₀N₅OP, C₂₈H₃₉N₃O₄ | 0.1-11.7 | 88.8-83.2 | Not Identified | |
| 484.3061² | [M+HCOO]⁻ | 110.9 | NS | C₂₁H₄₀N₈O₅, C₂₂H₄₀N₆O₆, C₂₆H₄₄O₈, C₂₇H₄₀N₄O₄, C₂₈H₃₆N₈ | 0.4-12.5 | 95.9-87.4 | Not Identified | |
| 495.3206 | [M-H]⁻ | 115.8 | NS | C₂₇H₄₅NO₇, C₂₄H₅₀NO₅PS, C₂₈U₄₁N₅O₃, C₂₄H₄₉NO₇S, C₂₅H₄₅N₅O₃S | 0.6-11.9 | 78.4-73.7 | Not Identified | |
| 495.3394 | [M-CH₃]⁻ | 108.1 | NS | C₂₄H₅₀NO₇P | 13.9 | 87.8 | PC(16:0/0:0) | LMGP 01050018 |
| 499.9355 | [M-H]⁻ | 166.3 | 0.05 | C₁₀H₃N₁₀O₉P₃; C₁₃H₈N₆O₃P₄, C₁₀H₂N₁₀O₁₁P₂, C₁₄H₇N₄O₁₁P₃, C₁₃H₁₁O₁₅P₃ | 0.2-11.5 | 95.9-94.4 | Not Identified | |
| 505.2842 | [M-H]⁻ | 100.1 | NS | C₂₃H₄₄N₃O₇P, C₂₄H₄₃NO₁₀, C₂₅H₃₉N₃O₆, C₂₆H₃₉N₃O₇, C₂₇H₃₅N₇O₃ | 8.1-14.8 | 97.1-90.7 | Not Identified | |
| 523.3690 | [M-H]⁻ | 121.2 | NS | C₂₆H₅₄NO₇P | 10.0 | 88.3 | PC(O-16:0/2:0) Platelet activating factor | LMGP 01050046^{d} MMCD cq_14947 |
| 530.3115² | [M-H]⁻ | 110.9 | NS | C₂₄H₅₂O₈P₂, c₂₃H₄₂N₆O₈, C₂₂H₄₂N₈O₇, C₂₈H₅₂O₃P₂S, C₂₇H₄₆O₁₀ | | 92.3-90.7 | Not Identified | |
| 553.3424 | [M-H]⁻ | 101.2 | NS | C₃₄H₄₃N5O2, C₃₃H₄₇NO₆, C₂₉H₄₃N₇O₄, C₃₉H₄₃N₃, C₂₇H₄₇N₅O₇ | 1.3-9.3 | 90.5-84.9 | Not Identified | |
| 635.4104 | [M-H]⁻ | 131.3 | NS | C₃₅H₅₇NO₉, C₃₀H₅₃N₉O₆, C₃₆H₅₃N₅O₅, C₃₂H₃₂NO₉P, C₄₁H₅₃N₃O₃ | 2.3-11.2 | 88.3-80.7 | Not Identified | |
| 640.4429³ | [M+CH₃CO O]⁻ | 123.0 | NS | C₄₄H₅₆N₄, C₄₅H₅₆N₂O, C₄₃H₆₀O₄, C₃₉H₅₆N₆O₂, C₅₀H₅₆N₄O₃ | 5.7-12.9 | 82.8-80.2 | Not Identified | |
| 654.4586³ | [M+HCOO]⁻ | 123.0 | NS | C₄₆H₅₈N₂O, C₄₄H₆₃O₂P, C₄₄H₆₂O₄, C₄₀H₅₈N₆O₂, C₄₁H₅₈N₄O₃ | 3.1-11.8 | 83.1-80.7 | Not Identified | |
| 700.4640³ | [M-H]⁻ | 123.0 | NS | C₄₆H₆₀N₄O₂, C₄₅H₆₄O₆, C₄₁H₆₀N₆O₄, C₄₀H₆₄N₂O₈, C₄₁H₆₄O₉ | 3.2-12.8 | 93.1-78.4 | Not Identified | |
| 743.5473 | [M-H]⁻ | 145.5 | NS | C₄₁H₇₈NO₈P | 1.1 | 39.4^{*} | PE(18:1(9E)/18:1(9E)) | LMGP 02010039^{e} |

| | | | | | | | | |
|---|---|---|---|---|---|---|---|---|
| ¹ Possible adduct species of ion with *m*/*z* 367.1934. ² Possible adduct species of ion with *m*/*z* 429.3038. ³ Possible adduct species of ion with *m*/*z* 699.5266. Low matching score due to lack of isotopic peaks for low SNR signal. ^{a} Multiple isomers found for this candidate including HMDB 06036 and HMDB 02177. ^{b} Multiple isomers found for this candidate in Lipid Maps including LMGP 01050001, and 01050011. ^{c} Multiple isomers found for this candidate in Lipid Maps including LMGP 01020019, 01020020, 01050019, 01050020, 01050074, 01050075, 01050113, 01050118, and 01050119. ^{d} Multiple isomers found for this candidate in Lipid Maps including LMGP 01020026, 01020047, 01020048, 01020049, 01020050, 01020135, 01050027, 01050028, 01050076, 1050077, 1050078, and 01050120. ^{e} Multiple isomers found for this candidate in Lipid Maps including LMGP 01010543, 01010544, 02010011, 02010028, 02010034, 02010443, 02010044, 020I0052, D2010109, 02D 10110. | | | | | | | | |

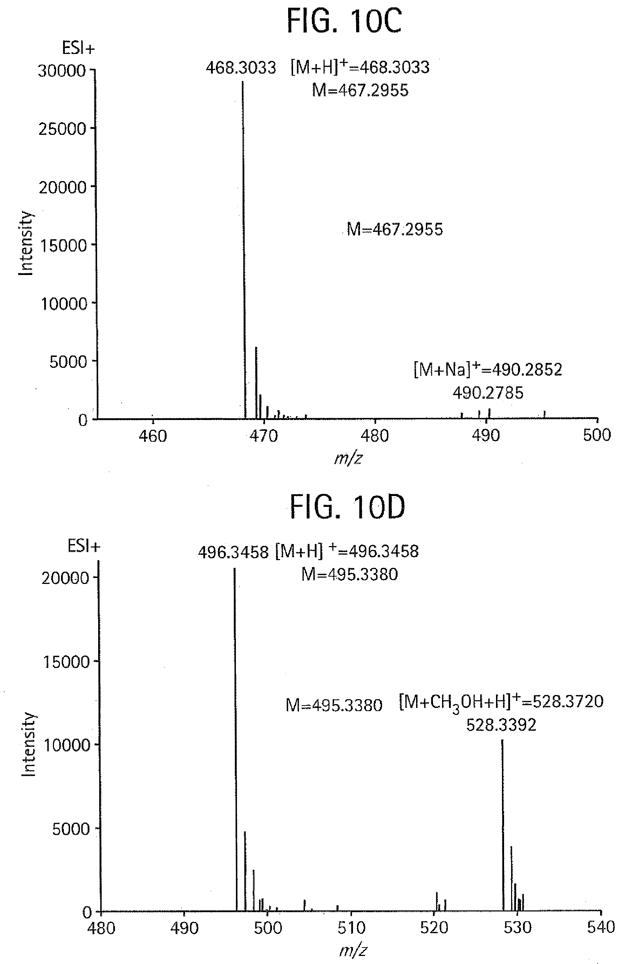

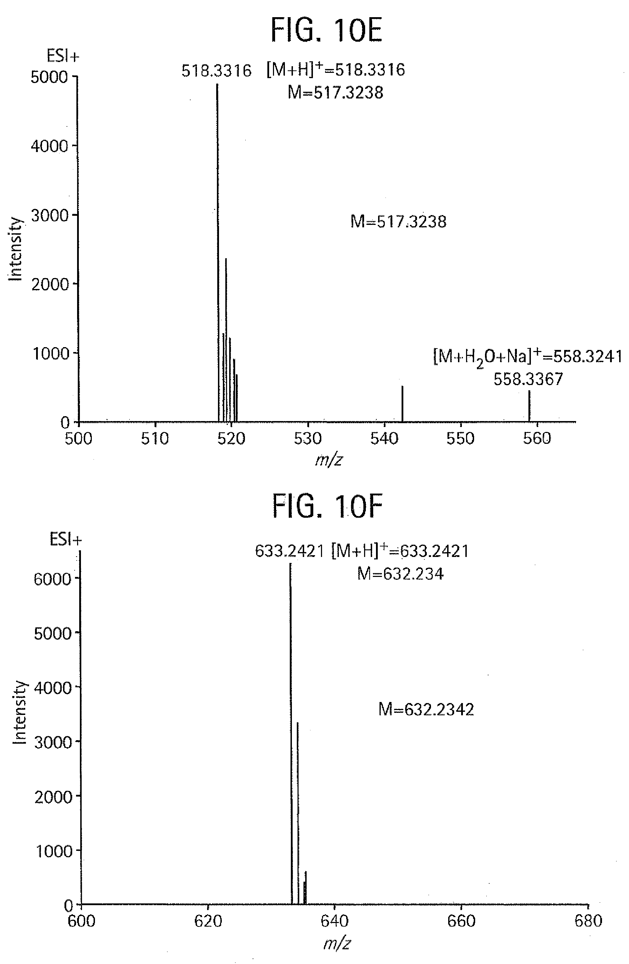

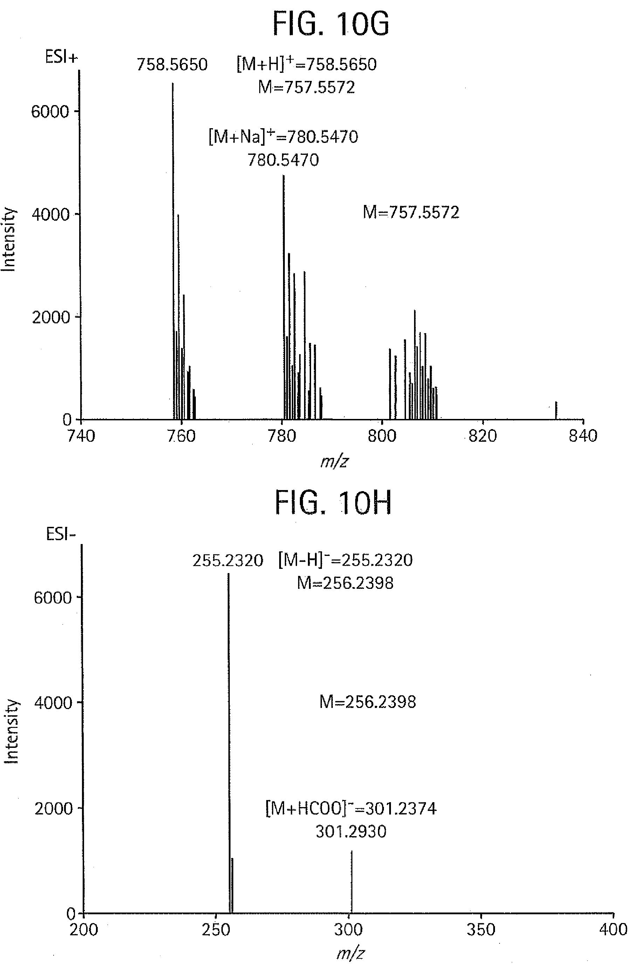

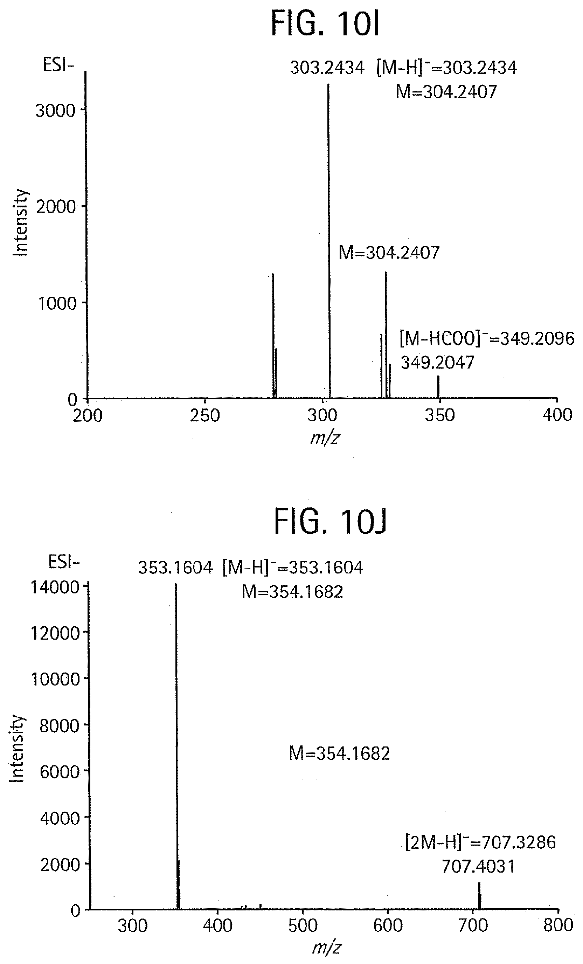

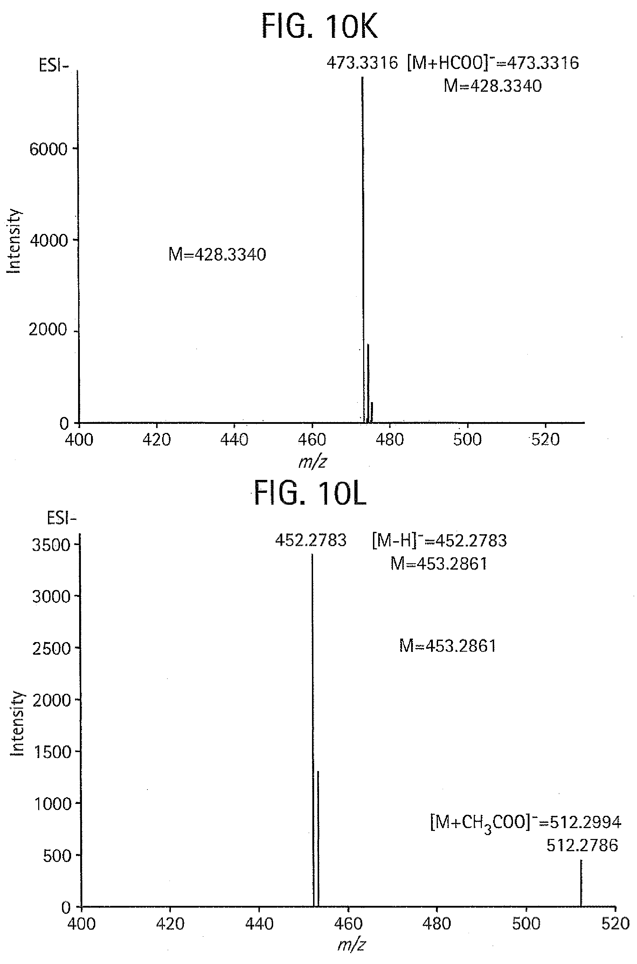

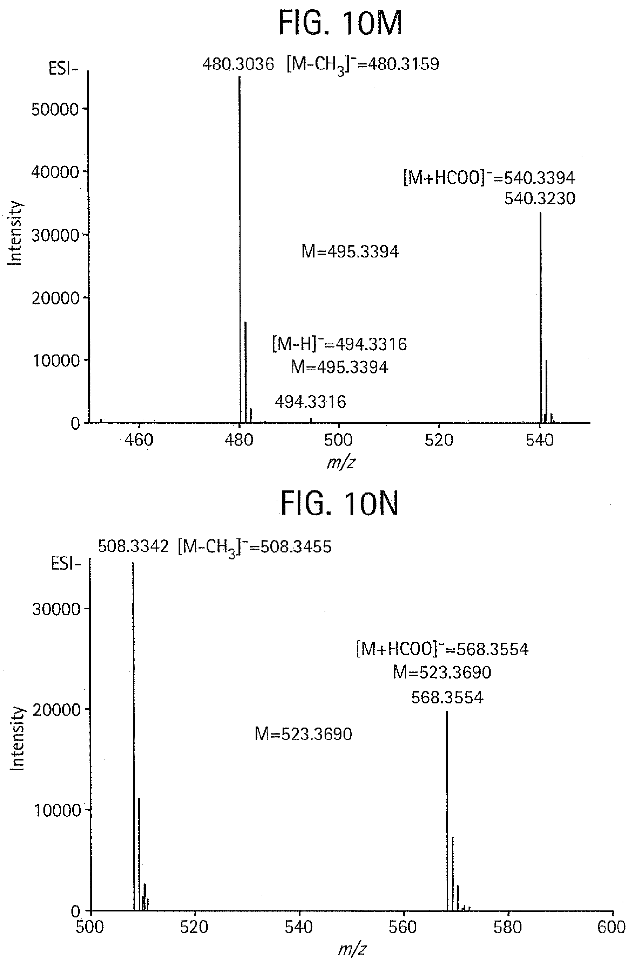

Adduct analysis of the 17 and 20 variables selected from positive and negative ESI mode, respectively, provided a total of 44 neutral masses to search against the databases as 1 variable was found to be redundant while 4 variables had multiple possible neutral masses due to ambiguity in the adduct assignment of the signal of interest. Seven of the positive ion mode ESI variables were preliminarily identified as the following metabolites: Phe-Ile, phosphatidylethanolamine PE(16:0/0:0), phosphatidylcholine PC(14:0/0:0), PC(16:0/0:0), PC(18:3/0:0), 2-sialyllactosamine, and PE-NMe(18:l/18:l) with mass accuracies ranging from 2.6-13.4 ppm and "seven-golden-rules" scores from 82.8-96.6. Eight metabolites were preliminarily identified from the negative ion mode subset of variables: palmitic acid, arachidonic acid, Gln-His-Ala, 4a-carboxy-4b-methyl-5a-cholesta-8,24-dien-3b-ol (also possibly identified as ercalcitriol), Pie(16:0/0:0), PCA(16:010:0), PC(O-16:0/2:0) (also referred to as platelet activating factor), and PE(18:1(9E)/18:1(9E)) with mass accuracies ranging from 1.1-13.9 and scores between 74.8 and 96.3. It must be noted that, in the case of phospholipids, assignment of the GA-selected variables to a given isomer is arbitrary, as single-stage MS cannot differentiate among these species. In this case, all possible *m*/*z* matches are noted. Figure 10 shows the centroided mass spectra corresponding to all annotated variables.

The variation in mass accuracies and identification scores observed in Tables 6 and 7 can be attributed to two major factors: 1) ambient temperature variations during the lengthy LC analysis time affecting both the output of the TOF mass spectrometer power supplies and the length of the flight tube, and 2) low signal intensity of some of the variables selected by GA. The software provided by the mass spectrometer manufacturer provides two methods to perform post-analysis correction of the *m*/*z* values obtained-mass drift compensation and mass calibration. Mass drift compensation, which is typically used to correct for temporal drift during long analysis times, was found to be insufficient to accurately calibrate the entire run. Instead, a full recalibration of the sample run using a calibration curve generated from the NaTFA standard run immediately after the sample was performed and provided a marked improvement in mass accuracy. It was further observed that inclusion of the isotope matching rule had a positive impact on decreasing the number of false-positive or negative entries on the hit lists.

### Example 2. Ovarian cancer detection from metabolomic liquid chromatography/mass spectrometry data by support vector machines

### Materials and Methods:

### Cohort description

Serum samples were obtained from 37 patients with papillary serous ovarian cancer (mean age 60 years, range 43-79, stages I-IV) and 35 controls (mean age 54 years, range 32-84). The control population consisted of patients with histology considered within normal limits (WNL) and women with non-cancerous ovarian conditions. The patients' information is detailed in Table 8.

**Table 8. Characteristics or ovarian cancer patients and controls**

| Characteristics | Stages I/II | Stages III/IV | Controls | Total |
|---|---|---|---|---|
| Age (y), mean (range) | 60 (43-74) | 61 (46-79) | 54 (32-84) | 58 (32-84) |
| Papillary serous carcinoma | 9 | 28 | 0 | 37 |
| Control | 0 | 0 | 35 | 35 |

All serum samples were obtained from the Ovarian Cancer Institute (OCI, Atlanta, GA) after approval by the Institutional Review Board (IRB). All donors were required to fast and to avoid medicine and alcohol for 12 hours prior to sampling, except for certain allowable medications, for instance, diabetics were allowed insulin. Following informed consent by donors, 5 mL of whole blood were collected at Northside Hospital (Atlanta, GA) by venipuncture from each donor into evacuated blood collection tubes that contained no anticoagulant. Serum was obtained by centrifugation at 5000 rpm for 5 minutes at 4° C. Immediately after centrifugation, two hundred and fifty µL aliquots of serum were frozen and stored at -80° C for further use. The sample collection and storage procedures for both ovarian cancer patients and control individuals were identical.

### Serum sample pretreatment and LClTOF MS analysis

A stock sample of human serum purchased from Sigma (S7023, St. Louis, MO) was used during the development of the serum sample pretreatment and LC/TOF MS analysis protocols. Upon arrival, the frozen stock sample was thawed and separated into 250 µ*L* aliquots which were stored at -80° C for further use.

Serum samples were thawed, and proteins precipitated by addition of acetonitrile to the serum sample in a 5:1 ratio (1000 µ*L* acetonitrile + 200 µ*L* serum). The mixture was vortexed for 1 minute and incubated at room temperature for 40 minutes, then the sample was centrifuged at 13,000g for 15 minutes and the supernatant retained. The supernatant was vacuum evaporated and the residue reconstituted in 80% acetonitrile/0.1% TFA.

LC/TOF MS analyses were performed on a JEOL AccuTOF (Tokyo, Japan) mass spectrometer coupled to an Agilent 1100 Series LC system (Santa Clara, CA) via an ESI source. The TOF resolving power measured at full width half maximum (FWHM) was 6000 and the observed mass accuracies ranged from 5-15*ppm,* depending on the signal-to-noise ratio (S/N) of the particular ion investigated. The LC system was equipped with a solvent degasser, a binary pump, an autosampler, and a thermostatic column compartment (held at 25°C). The injection volume was 15 µ*L* in all cases. Reverse phase separation of serum samples was performed using a Symmetry® C18 column (3.5µ*m*, 2.1*mm* x 150*mm,* pore size 100*Å*; Waters, Milford, MA) at a flow rate of 150µ*Lmin⁻¹.* The analytical column was preceded by a Zorbax® RX-C18 guard column (5.0µ*m*, 4.6*mm* x *12.5mm,* pore size 2□*m*; Agilent). The LC solvent mixtures used were: A = 0.1% formic acid in water and B = 4.1% formic acid in acetonitrile. After a pre-run equilibration with 5% B for 5 minutes, data acquisition was started and the solvent composition was varied according to the solvent program described in Table 9.

**Table 9: LC solvent gradient used in metabolomic experiments.**

| Time (min) | %B (acetonitrile/0.1% formic acid) | Flow Rate (µ*Lmin⁻¹*) |
|---|---|---|
| Pre-Run | | |
| 0.0 | 100 | 300 |
| 10.0 | 5 | 150 |
| 15.0 | 5 | 150 |
| Run | | |
| 0.0 | 5 | 150 |
| 5.0 | 5 | 150 |
| 10.0 | 20 | 150 |
| 20.0 | 25 | 150 |
| 28.0 | 30 | 150 |
| 38.0 | 35 | 150 |
| 50.0 | 40 | 150 |
| 90.0 | 45 | 150 |
| 100.0 | 50 | 150 |
| 110.0 | 60 | 150 |
| 120.0 | 75 | 150 |
| 130.0 | 85 | 150 |
| 160.0 | 95 | 150 |
| 180.0 | 100 | 150 |
| Post-Run | | |
| 0.0 | 100 | 300 |
| 30.0 | 100 | 300 |

After analysis of a given serum specimen, a 0.20*mM* sodium trifluoroacetate standard (NaTFA) was run for mass drift compensation purposes. For NaTFA analysis, 100% B at a flow rate of 300 *µ*L min⁻¹ was used and data was acquired for 10 minutes. After injection of the drift correction standard, the column was washed with 100% B for 30 minutes.

Spectral data was collected in the 100-1750 m/z range with a spectral recording interval of 1.5s and a data sampling interval of 0.5*ns* for both positive and negative ion ESI modes. The settings for the TOF mass spectrometer for positive or negative ion mode were as follows: needle voltage: +/- 2000*V*, ring lens: +8*V* or -9*V*, orifice 1: +30*V* or -69*V*, orifice 2: +6*V* or 8*V*, desolvation chamber temperature: 2S0°C, orifice 1 temperature: 80°C, nebulizing gas flow rate: 1.0*Lmin⁻¹,* desolvation gas flow rate 2.5*Lmin⁻¹*, and detector voltage +/- 2800*V*. The TOF analyzer pressure was 4.8E*-6Pa* during analysis. The RF ion guide voltage amplitude was swept to ensure adequate transmission of analytes in a wide range of *m*/*z* values. The sweep parameters were as follows: initial peaks voltage: 700*V*, initial time: 20%, sweep time: 50%, final peaks voltage: 2500*V.* After LC/TOF MS data was collected, it was centroided, mass drift corrected using the NaTFA reference spectrum, and exported in NetCDF format for further mining.

To ensure maximum reproducibility in metabolomic experiments, all serum specimens were run consecutively within a 2.5 month period. Every cancer sample was randomly paired with a normal sample and run on the same day to ensure that no temporal bias was introduced in the way samples were analyzed. Sample pairs were run in random order and in duplicate.

### LC/TOF MS data preprocessing

All data were preprocessed identically and simultaneously. Preprocessing was performed by loading NetCDF files into mzMine (v0.60) (Katajamaa, et al., Bioinformatics, 22(5):634-6 (2006)). Data were smoothed by chromatographic median filtering with a tolerance in *mlz* of 0.1, and one-sided scan window length of 3s. Peaks were picked with a *mlz* bin size of 0.15, chromatographic threshold level of 0%, absolute noise level of 200, absolute minimum peak height of 250, minimum peak duration of 5s, tolerance for *mlz* variation of 0.06, and tolerance for intensity variation of 50%. The method for de-isotoping was to assume +1 charge states, and monotonic isotopic patterns. The retention time tolerance (RT) for de-isotoping was 65s and the m=z tolerance 0.07. The chromatographic peak alignment *m*/*z* tolerance was 0.2, and the RT tolerance was 12%, with a balance coefficient between *m*/*z* and RT of 30. The minimum number of detections for rare peak filtering in the alignment results was set to 41. Spectral features not initially detected by the peak detection algorithm were subsequently added by a gap filling method using an intensity tolerance of 30%, *m*/*z* tolerance size of 0.2, and RT tolerance size of 12%. Correction for systematic drift in intensity levels between different data files was performed by using linear intensity normalization of the total raw signal. After the normalized alignment file containing all peak intensities was created, peak areas were exported to Excel and peaks of contaminants, dimers, redundant adducts, and isotopes not adequately detected were removed. Approximately 37% of the peaks from positive mode and 18% of the peaks from negative mode were eliminated after this filtering step. Peak areas from duplicate runs were then averaged, and positive and negative mode ESI data were exported as ASCII files into Matlab for subsequent machine learning analysis.

### SVMs and related feature selection methods

SVMs (Vapnik, The Nature of Statistical Learning_Theory, Springer (2000)) have been successfully applied to various scientific problems as they generally achieve classification performance superior to that of many older methods, particularly in high-dimensional settings (Li, et al., Artificial Intelligence Med., 32(2):71-83 (2004); Rajapakse, et al., Am. J, Pharmacogenomics, 5(5):281 (2005); Yu, et al., Bioinformatics, 21(10):2200-2209 (2005); Shen, et al., Cancer Informatics, 3:339-349 (2007); Wu, et al., Bioinformatics, 19(13):1636-43 (2003); Pham, et al., Stat. Appl. Genetics. Mol. Biol., 7(2):11 (2008)). Though computationally intensive, SVMs are efficient enough to handle problems of the size we consider here. Given a dataset $S = {\left\{{x}_{j}, {y}_{j}\right\}}_{j = 1}^{M}$ (*xⱼ* is the feature vector of *j*th instance and *yⱼ* is the corresponding label), for a two-class classification problem, the standard linear SVM solves the following convex optimization: ${min}_{ω , b , ξ} \frac{1}{2} {‖ ω ‖}^{2} + C {\sum }_{i = 1}^{M} {ξ}_{i} s . t . { y}_{i} \left(ω⋅{x}_{i}+b\right) + {ξ}_{i} \geq 1 , {ξ}_{i} \geq 0 i = 1 , … , M$ In the case of nonlinear SVMs, the feature vectors x ∈ *R^{d}* are mapped into high dimensional Euclidean space, *H*, through a mapping function Φ(·) : *R^{d}* → *H.* The optimization problem becomes: ${min}_{ω , b , ξ} \frac{1}{2} {‖ ω ‖}^{2} + C {\sum }_{i = 1}^{M} {ξ}_{i} s . t . { y}_{i} \left(ω⋅Φ\left({x}_{i}\right) + b\right) + {ξ}_{i} \geq 1 , {ξ}_{i} \geq 0 i = 1 , … , M$ The kernel function is defined as *K*(*xᵢ, xⱼ*) = Φ(*xᵢ*)Φ(*xⱼ*), for example, a polynomial kernel of degree 2 is defined as *K*(*xᵢ, xⱼ*) = *(gxᵢ* · *xⱼ* + *r*)², where *g*, *r* are kernel parameters. The linear kernel function is defined as *K*(*xᵢ, xⱼ*) = *xᵢ · xⱼ*. Tools such as libSVM (http://www.csie.ntu.edu.tw/~cjlin/libsvm) can efficiently solve the dual formation of the above problem: ${min}_{α} \frac{1}{2} {\sum }_{i , j = 1}^{M} {y}_{i} {y}_{j} {α}_{i} {α}_{j} K \left({x}_{i}, {x}_{j}\right) - {\sum }_{i = 1}^{M} {α}_{i} s . t . {\sum }_{i = 1}^{M} {y}_{i} {α}_{i} = 0 , 0 \leq {α}_{i} \leq C i = 1 , … , M$ where *αᵢ* is the Lagrange multiplier corresponding to the *i*th inequality in the primal form. The solution is $ω = {\sum }_{i = 1}^{M} {α}_{i} {y}_{i} Φ \left({x}_{i}\right)$ for linear SVM, $ω = {\sum }_{i = 1}^{M} {α}_{i} {y}_{i} {x}_{i}$ The optimal decision function for an input vector x is $f \left(x\right) = ω ⋅ x + b = {\sum }_{i = 1}^{M} {y}_{i} {α}_{i} K \left({x}_{i}, x\right)$ where the predicted class is +1 if *ƒ*(x) > 0 and -1 otherwise.

Bagging strategies (Breiman, Machine Learning, 24(2):113-140 (1996))] are often used to boost the prediction performance of a classifier (Zhang, et al., Lecture Notes in Computer Science, 4830:820 (2007)). This approach involves generating multiple versions of a classifier and using these to obtain an aggregated predictor. A bagging process repeats the following procedure T times: i) bootstrap (sample from the dataset with replacement) from the training data to build a classifier and ii) obtain the prediction results on the test data. The process then uses the majority voting results as the final prediction results and their accuracy as the final test accuracy.

t2-statistics (Baldi and Long, Bioinformatics, 17(6):509-19 (2001)) is a widely used filter-based feature selection method in bioinformatics, $\frac{{µ}_{+} - {µ}_{-}}{\sqrt{\frac{{δ}_{+}}{{n}_{+}} + \frac{{δ}_{-}}{{n}_{-}}}}$ with degree of freedom $df = \frac{{\left[\left({δ}_{-}^{2}/{n}_{-}\right)+\left({δ}_{+}^{2}/{n}_{+}\right)\right]}^{2}}{\frac{{δ}_{-}^{2} / {n}_{-}}{{n}_{-} - 1} + \frac{{δ}_{-}^{2} / {n}_{-}}{{n}_{-} - 1}}$ Where *µ*+, *µ*- are the mean of the feature values of cancer patients and controls, respectively. *δ*+, *δ*- are the corresponding standard deviations and *n*₊, *n₋* are the corresponding patient numbers. Though computationally efficient, filter-based feature selection methods generally achieve inferior prediction performance compared to the wrapper based feature selection methods. Therefore, several feature selection methods based on SVMs, such as the commonly used recursive feature elimination (RFE) method (Guyon, et al.., Machine Learning, 46:389-422 (2002)), were applied.

At each RFE iteration, first, an SVM is trained with the currently selected feature set; next, the importance of a feature is measured according to the sensitivity of the cost function $J = \frac{1}{2} {\sum }_{i , j = 1}^{M} {y}_{i} {y}_{j} {α}_{i} {α}_{j} K \left({x}_{i}, {x}_{j}\right) - {{\sum }_{i = 1}^{M} α}_{i}$ with respect to the feature; then, less important features are dropped successively from the remaining feature set. Typically the bottom 10% features are removed at each iteration for efficiency, but empirical experiments suggest removing the bottom feature one at a time for highest accuracy. This procedure is repeated iteratively to study the prediction accuracy as a function of the number of remaining features and the smallest feature set that achieved the highest training accuracy is selected as the final output.
The cost function can be rewritten as $J = \frac{1}{2} {α}^{T} Hα - {α}^{T} {1}_{n}$ and the sensitivity of the cost function to a feature is $dJ \left(k\right) = \frac{1}{2} {α}^{T} Hα - \frac{1}{2} {α}^{T} H \left(-k\right) α$ where *H* and *H*(-*k*) are *M* x M matrices with ${H}_{ij} = {y}_{i} {y}_{j} K \left({x}_{i}, {x}_{j}\right) and H {\left(-k\right)}_{ij} = {y}_{i} {y}_{j} K \left({x}_{i}\left(-k\right),{x}_{j}\left(-k\right)\right)$ where *x*(-*k*) means the *k*th feature has been removed from the input vectors.
In the case of linear SVM $dJ \left(k\right) = \frac{1}{2} {\sum }_{i , j = 1}^{M} {α}_{i} {α}_{j} {x}_{ik} {x}_{jk} = \frac{1}{2} {ω}_{k}^{2} .$ The feature whose removal leads to a smaller increase to the cost function, *dJ*(*i*), is marked as less important.

Bradley et al. (Bradley, et al.., Machine Learning Proc. Of the 15th International Conference (ICML98), 82-90 (1998)) proposed L1SVM, which minimizes the L1-norm: ${‖ ω ‖}_{L 1} = {\sum }_{k = 1}^{N} \left|{ω}_{k}\right|$ rather than minimizing the L2-norm of the weight vector (or normal of the separating hyperplane) ${‖ ω ‖}_{L 2} = {\sum }_{k = 1}^{N} {ω}_{k}^{2} .$ Thus, the optimization problem becomes: ${min}_{ω , b , ξ} \frac{1}{2} {\sum }_{k = 1}^{N} \left|{ω}_{k}\right| + C {\sum }_{i = 1}^{M} {ξ}_{i} s . t . { y}_{i} \left(ω⋅{x}_{i}+b\right) + {ξ}_{i} \geq 1 , {ξ}_{i} \geq 0 i = 1 , ⋯ , M .$ Since the L1-norm is used, the optimal weight vector w is often very sparse, thus L1SVM can simultaneously perform classification as well as feature selection. However, this is only applicable in the case of the linear kernel. Although L1SVM performs well in feature selection, its classification results can be improved by applying the standard L2-norm SVM classifier on the selected feature subset (Weston, et al., J. Machine Learning Res., 3:1439-61 (2003)). Fast algorithms for solving the L1SVM optimization problem were proposed by Fung & Mangasarian in 2004 (Fung and Mangasarian, Comp. Opt. Appl., 28(2):185-202 (2004)) and Mangasarian in 2007 (Mangasarian, et al., J. Machine Learning Res., 7(2):1517-30 (2007)).

Weston et al. (Weston, et al., Adv. Neural Info. Proc. Sys., (NIPS01), 668-74 (2001)) proposed another SVM related feature selection method that minimizes a generalization error bound, namely the radius to margin distance ratio *R²W²*. *R²* is the radius of the smallest sphere, centered at the origin that contains all $Φ \left({x}_{i}\right) , i = 1 , ⋯ , M ;$ *W²* is the L2 norm of the normal vector to the optimal separating hyperplane.
*R²* and *W²* can be formulated as follows with the introduction of kernel ${K}_{δ} \left({x}_{i}, {x}_{j}\right) = K \left({δx}_{i}, {δx}_{j}\right)$ where matrix $δ = diag \left({δ}_{1},⋯,{δ}_{n}\right) , {δ}_{k} ∈ \left\{0, 1\right\} , k = 1 , ⋯ , n :$ ${R}^{2} \left(β, δ\right) = {max}_{β} {\sum }_{i} {β}_{i} {K}_{δ} \left({x}_{i}, {x}_{i}\right) - {\sum }_{i , j} {β}_{i} {β}_{j} {K}_{δ} \left({x}_{i}, {x}_{j}\right) s . t . {\sum }_{i} {β}_{i} = 1 , {β}_{i} \geq 0 , i = 1 , ⋯ , M$ ${W}^{2} \left(α, δ\right) = {max}_{α} {\sum }_{i} {α}_{i} - \frac{1}{2} {\sum }_{i , j = 1}^{M} {α}_{i} {α}_{j} {y}_{i} {y}_{j} {K}_{δ} \left({x}_{i}, {x}_{j}\right) s . t . {\sum }_{i} {α}_{i} {y}_{i} = 0 , {α}_{i} \geq 0 , i = 1 , ⋯ , M$ The above optimization problem is approximated using gradient descent. At each iteration, the algorithm firstly optimizes *R²*(*β,δ*) with respect to *β*, *W²*(*α,δ*) with respect to α (denoting the optimal solution as α° and β°, respectively); next, it minimizes R*²*(*α,δ*)*W²*(*β,δ*) with *α* fixed to *α*⁰ and *β* fixed to β⁰ using steepest descent; then, it sets the smallest *δₖ* to zero, i.e. removes the corresponding *k*th feature from the feature set. The algorithm repeats the above procedure until only *d* nonzero elements, *δ*₁,..., *δ*_{d} are left.

### Statistical significance estimation

In addition to estimating the classification/feature selection performance using various cross-validation approaches, the statistical significance of these observations was further assessed through hypothesis testing. One possible non-parametric approach to hypothesis testing is permutation test, where no assumptions are made regarding the data distribution and the p-value is computed as the cumulative sum using the empirical distribution. The permutation test works by comparing the statistic of interest with the distribution of the statistic obtained under the null (random) condition, and can be defined as follows (Mukherjee, et al., J. Comp. Biol., 10(2):119-42 (2003)):
1. Repeat *T* times (where *t* is an index from 1,...,*T*):
   - Randomly permute the labels of the input data vectors.
   - Compute the statistic of *interest Sₜ* = *TS*(*x*₁, y_{*t*i},..., _{xM}; *y_{tM}*) for this permutation of labels, where *yₜᵢ* is the assigned label to *xᵢ* at *t^{th}* label randomization.
2. Compute the statistic of interest for the actual labels, *s₀.*
3. Obtain the p-value ${\sum }_{t = 1}^{T} I \left({s}_{t}\geq {s}_{0}\right) :$ the cumulative probability *of sₜ* being greater than or equal to the observed statistics *s₀*.
4. If the p-value < a (usually *a =* 0.05 or 0.1), reject the null hypothesis *H0*; otherwise, the observed result is not statistically significant.

### Metabolite Identification procedure

Compound identification was attempted only for those spectral features remaining after the feature selection processes. Due to the biological complexity of serum samples, adduct ion analysis was first performed to ensure the unambiguous assignment of the signal of interest in each mass spectrum. Adducts formed in positive ion mode ESI usually include [M + H]⁺, [M + NH₄]⁺, [M + Na]⁺, [M + K]⁺, [M - H₂O + H]⁺ and [2M + H]⁺ species; in negative ion mode ESI [M - H]⁻, [M + CH₃COO]⁻, [M + Cl]⁻, [M + HCOO]⁻ and [2M - H]⁻ are generally observed. Adducts in centroided mass spectra corresponding to SVM-selected variables were identified by manually calculating the differences between the exact *m*/*z* values of peaks within the spectrum and comparing these differences to those between the common adduct species mentioned above. For spectra in which multiple adducts were not present, the accurate mass of the candidate neutral molecule was calculated based on the assumption that the peak of interest corresponded to either [M + H]⁺, [M + Na]⁺, or [M + NH₄]⁺ in positive ion mode and [M - H]⁻, [M + CH₃COO]⁻, [M + HCOO]⁻, or [M - CH₃]⁻ (for glycerophosphocholines) in negative ion mode, yielding multiple candidate masses for each *m*/*z* value.

Elemental formulae were estimated from the accurate mass spectra using a freely distributed system of macros (Kind and Fiehn, BMC Informatics, 8:105 (2007)) that relies on a series of heuristic rules to identify possible formulae based on the mass accuracy of the peak of interest and the corresponding isotopic ratios. The mass of the neutral molecule and relative isotopic abundances were imported directly into the \seven golden rules" Excel spreadsheet (http://fiehnlab.ucdavis.edu/projects/Seven_Golden_Rules). The mass accuracy was set to 15 ppm, and the threshold for error in the relative isotopic abundances was set to 10%.The list of elements to include in the search was constrained to include C, H, N, O, P, S, Cl, and Br. The probability of a given formulae being the "correct" one is provided as a score calculated from the error rates in satisfying the aforementioned rules. The top hits in the list of filtered elemental formulae and all accurate mass values obtained were searched against the following databases: Metlin (http://metlin.scripps.edu), KEGG (http://www.genome.jp), HMDB (http://www.hmdb.ca), MMCD (http://mmcd.nmrfam.wisc.edu) and Lipid Maps (LM) (http://www.lipidmaps.org) in order to determine the greatest possible number of candidate molecules. The criteria used for the assignment of a tentative chemical structure were: a mass difference with the simulated formula lower than 15 ppm, isotope abundance errors less than 10%, and that the candidate found in the database corresponds to an endogenous metabolite.

### Results:

### LC/TOF MS-based metabolomic analysis of human serum samples

Metabolomic investigation of sera from patients with ovarian cancer and controls using LC/TOF MS revealed a total of 576 features extracted by mzMine in positive ion mode, and 280 in negative ion mode. The data were found to be highly complex, with numerous features across both analytical dimensions. Decreasing the absolute noise level and minimum peak height from 400 and 500 to 200 and 250 increased the number of detected features to 4439 and 329 for positive and negative ion modes, respectively. While this allowed a "deeper dig" into the serum metabolome, the number of features consistently detected across samples decreased by 3.6% and 15%, respectively, suggesting that use of the previous settings provided a broad range of more stable features on which to base our feature selection methods. Detailed manual analysis of the entire dataset revealed the presence of additional redundant species (dimers, adducts, isotopes) that were removed, thus reducing the final number of features used to 360 positive ion mode and 232 negative ion mode features. The dataset with only positive ion mode features is referred to as "pos-ion-mode", the dataset with only negative ion mode features is referred to as "neg-ion-mode", and the dataset combining positive and negative ion mode features is referred to as "multimode", respectively.

A 3D serum metabolic profile for a typical stage III ovarian cancer serum sample is shown in Figure 2A demonstrating the capability of LC/TOF MS to resolve hundreds of compounds in a wide mass range within 180 minutes. Despite the shallow solvent gradient chosen for the LC run, there is still evidence of co-elution as observed in the projection of Figure 2A onto the chromatographic axis (Figure 2B). However, in most cases, the high resolving power of the TOF mass analyzer allowed the resolution of these signals by their selected ion chromatograms, as shown in Figure 2C for an ion with *m*/*z* = 443.26 at a window width of 0.05Da. The corresponding centroided negative ion mode spectrum obtained at 91 minutes is shown in Figure 2D. Due to the obvious complexity of these samples, the reproducibility of the LC/TOF MS approach was tested in early experiments to rule out column memory effects. Lipids, fatty acids and other hydrophobic components in sera that are easily adsorbed onto the reverse phase column can act as a new stationary phase, causing a change in selectivity, memory effects, and shifting retention times.

### Prediction performance and statistical significance analysis

SVMs and state-of-the-art feature selection methods were used to analyze the data. In the following sections, the linear SVM classifier is denoted as SVM, nonlinear SVM classifier with degree 2 polynomial kernel as SVM_NL; RFE feature selection with linear SVM as SVMRFE, RFE with nonlinear SVM as SVMRFE_NL, and Weston's feature selection method with nonlinear SVM as SVMRW. Three evaluation procedures were considered: i) leave-one-out-cross-validation (LOOCV); ii) 12-fold cross validation (12-fold
CV) averaged over 10 trials (for each trial, the data were randomly ordered and split into 12 different folds and a 12-fold CV was performed); and iii) 52-20-split-validation averaged over 50 trials (for each trial, the data were randomly ordered and split into a training set of size 52 and a test set of size 20). Of these,
LOOCV is expected to be the most reliable given the small sample size, but all three were investigated for thoroughness.

### Prediction and feature selection performance

The prediction performance for each dataset was first evaluated without feature selection (Figure 11 A). The results are summarized in Table 10. As apparent in the table, the multimode dataset had the best prediction performance (83.3%) using a nonlinear SVM classifier, while the neg-ion-mode dataset had a better prediction performance than the pos-ion-mode dataset. The nonlinear SVM classifier generally outperformed the linear SVM classifier except on the neg-ion-mode dataset.

**Table 10: Prediction performance (%) without feature selection**

| **Classifier** | **LOOCV** | **12-fold CV (10 trials)** | **52-20-split validation (50 trials)** |
|---|---|---|---|
| ***Multimode (n=592)*** | | | |
| SVM | 81.9 | 80.3 | 75.8 |
| SVM_NL | 83.3 | 81.7 | 76.3 |
| ***Pos-ion-mode (n=360)*** | | | |
| SVM | 72.2 | 71.3 | 70.0 |
| SVM_NL | 73.6 | 75.6 | 71.8 |
| ***Neg-ion mode (n=232)*** | | | |
| SVM | 81.9 | 80.4 | 73.2 |
| SVM_NL | 80.6 | 79.9 | 72.4 |

Next, the prediction performance was evaluated following feature selection. As discussed in the previous section, except for L1SVM, the other three feature selection methods tested are iterative methods with optimal feature sets determined according to criteria such as training accuracy (for SVMRFE,
SVMRFE_NL), or generalization error bound (for SVMRW). In the experiments, a LOOCV average classification accuracy over the input dataset (for feature selection) containing only the selected feature subset was used as the criterion for determining the optimal feature subset for the following reasons: i) the SVM training accuracy (using the same dataset to train and test the classifier) was almost always 100% until the feature set became unreasonably small and ii) the minimal generalization error was usually achieved when the feature set was quite large. The size of the feature set was further restricted to be less than 50 to allow for fair comparison of the performance with the L1SVM feature selection results.

In the second set of experiments (Figure 11B), each feature selection method was applied to the whole dataset, then the prediction performance of the dataset containing only the selected feature subset (panel) was measured using the three evaluation processes described above. The estimated predictive performance was surprisingly high (greater than 90%) under LOOCV (Tables 11 and 12), which is perhaps the most accurate evaluation technique in this low-sample setting. For the multimode dataset, the feature selection results of SVMRFE_NL had the best discriminative power according to both LOOCV and 12-fold CV evaluation, while the feature subset selected by SVMRFE archived the best test accuracy in 52-20 split validation evaluation and the second best test accuracy in LOOCV and 12-fold CV evaluation. For the pos-ion-mode and neg-ion-mode datasets, the feature selection results of SVMRFE achieved the best test accuracy.

**Table 11. Prediction performance (%): feature selection methods applied to the whole dataset.**

| **Classifier** | **Feature Selection** | **LOOCV** | **12-fold CV (10 times)** | **52-20-split Validation (50 times)** |
|---|---|---|---|---|
| ***Multimode (n=592)*** | | | | |
| SVM | SVMRFE | 95.8 | 94.2 | 91.1 |
| SVM | L1SVM | 93.1 | 92.1 | 84.8 |
| SVM_NL | SVMRFE NL | 97.2 | 94.3 | 88.7 |
| SVM_NL | SVMRW | 91.7 | 86.8 | 79.4 |
| ***Pos-ion-mode*** | ***(n=380)*** | | | |
| SVM | SVMRFE | 91.7 | 87.6 | 81.6 |
| SVM | L1SVM | 76.4 | 75.1 | 72.9 |
| SVM_NL | SVMRFE_NL | 83.3 | 81.1 | 76.2 |
| SVM_NL | SVMRW | 65.3 | 61.3 | 60.5 |
| ***Neg-ion mode (n=232)*** | | | | |
| SVM | SVMRFE | 100.00 | 98.5 | 94.0 |
| SVM | L1SVM | 95.8 | 91.8 | 82.5 |
| SVM_NL | SVMRFE_NL | 97.2 | 95.7 | 88.5 |
| SVM_NL | SVMRW | 88.9 | 83.3 | 77.4 |

**Table 12. Statistics on the number of important features from models described in Table 11.**

| **Feature #** | **SVMRFE** | **L1SVM** | **SVMRFE_NL** | **SVMRW** |
|---|---|---|---|---|
| ***Multimode (n=592)*** | | | | |
| | 33 | 43 | 45 | 41 |
| ***Pos-ion-mode (n=360)*** | | | | |
| | 36 | 37 | 22 | 32 |
| ***Neg-ion mode (n=232)*** | | | | |
| | 47 | 47 | 23 | 32 |

The aforementioned experiments can be regarded as measuring the SVM predictive performance of certain feature subsets, regardless of how the subsets were obtained. Note that a production classifier for ovarian cancer diagnosis would use an a priori-fixed feature set. However, Furlanello et al, 2003 (Furlanello, et al., BMC Bioinformatics, 4:54 (2003)) indicated that applying feature selection over the whole dataset might introduce selection bias into the evaluation of the feature selection results even if the prediction performance is obtained through cross-validation. Therefore, a third set of experiments to compare the generalization performance of the feature selection methods themselves in combination with SVM was performed under more conservative settings as illustrated in Figure 11C. For each feature selection method, at each evaluation, the method was first applied only to the training dataset and then the prediction performance of the selected feature subset on the validation (test) dataset was measured. As shown in Table 13, the best prediction performance in this setting is 80.6%, which is comparable to the prediction performance without feature selection, while the feature size is reduced, on average, from 592 to 38 (with SVMRFE_NL) and from 232 to 41 (with SVMRFE), respectively (Table 14).

**Table 13. Prediction performance (%): Feature selection methods applied to training subsampling of dataset during each validation.**

| **Classifier** | **Feature Selection** | **LOOCV** | **12-fold CV (10 times)** | **52-20-split Validation (50 times)** |
|---|---|---|---|---|
| *Multimode (n=592)* | | | | |
| SVM | SVMRFE | 69.4 | 71.4 | 67.7 |
| SVM | L1SVM | 76.4 | 76.8 | 72.9 |
| SVM_NL | SVMRFE_NL | 80.6 | 74.0 | 71.6 |
| SVM_NL | SVMRW | 70.8 | 68.2 | 61.9 |
| ***Pos-ion-mode (n 360)*** | | | | |
| SVM | SVMRFE | 72.2 | 67.5 | 64.0 |
| SVM | L1SVM | 70.8 | 70.6 | 65.5 |
| SVM__NL | SVMRFE NL | 66.7 | 71.4 | 66.5 |
| SVM_NL | SVMRW, | 59.7 | 59.7 | 60.2 |
| ***Neg-ion mode (r=232)*** | | | | |
| SVM | SVMRFE | 80.6 | 74.7 | 68.4 |
| SVM | L1SVM | 75.0 | 76.2 | 71.5 |
| SVM_NL | SVMRFE_NL | 73.6 | 74.3 | 69.1 |
| SVM_NL | SVMRW | 69.4 | 63.6 | 59.6 |

**Table 14. Statistic on the average number of important features of the models described in Table 13.**

| **Classifier** | **Feature Selection** | **LOOCV** | **12-fold CV (10 times)** | **52-20-split Validation (50 times)** |
|---|---|---|---|---|
| *Multimode (n=592)* | | | | |
| SVM | SVMRFE | 28 ± 7 | 27 ± 9 | 22 ± 9 |
| SVM | L1SVM | 43 ± 1 | 41 ± 2 | 34 ± 2 |
| SVM_NL | SVMRFE_NL | 38 ± 9 | 31 ± 8 | 26 ± 8 |
| SVM_NL | SVMRW | 40 ± 5 | 36 ± 8 | 29 ± 9 |
| ***Pos-ion-mode (n=360)*** | | | | |
| SVM | SVMRFE | 35 ± 5 | 31 ± 8 | 25 ± 7 |
| SVM | L1SVM | 36 ± 1 | 35 ± 2 | 30 ± 2 |
| SVM_NL | SVMRFE_NL | 26 ± 7 | 30 ± 10 | 21 ± 7 |
| SVM_NL | SVMRW | 31 ± 9 | 27 ± 11 | 20 ± 9 |
| ***Neg-ion mode (n*=*232)*** | | | | |
| SVM | SVMRFE | 41 ± 9 | 33 ± 8 | 27 ± 9 |
| SVM | L1SVM | 44 ± 2 | 41 ± 2 | 34 ± 2 |
| SVM_NL | SVMRFE_NL | 36 ± 9 | 37 ± 7 | 33 ± 8 |
| SVM_NL | SVMRW | 34 ± 7 | 34 ± 7 | 32 ± 10 |

LOOCV evaluation leads to a higher test accuracy than the other two evaluation procedures demonstrating the effect of the training set size on the test accuracy. LOOCV evaluation results indicate that i) feature selection using SVMRFE_NL achieved the best prediction performance on the multimode dataset, ii) feature selection using SVMRFE achieved the best prediction performance on the pos-ion-mode and neg-ion-mode datasets, and iii) the L1SVM method was the second best feature selection method while SVMRW was the worst. Both 52-20-split validation and 12-fold CV evaluation results indicate that i) L1SVM performed the best on the multimode and neg-ion-mode datasets, ii) SVMRFE NL method performed the best on the pos-ion-mode dataset, and iii) SVMRW method resulted in the worst prediction accuracy. Overall, a clear winner was not easily identifiable among the tested methods.

As shown in Table 13, the neg-ion-mode dataset had a similar prediction performance as the multimode dataset. The analysis of sensitivity (how well cancer patients can be detected) and specificity (how well controls can be detected) (Tables 15 and 16), somewhat favors usage of the multimode dataset, in that, the results show that this dataset achieved a better balance between sensitivity and specificity.

**Table 15. Averaged LOOCV specificity and sensitivity (%) without feature selection.**

| **Classifier** | **Test Accuracy** | **Sensitivity** | **Specificity** |
|---|---|---|---|
| ***Multimode (n=592)*** | | | |
| SVM | 81.9 | 81.8 | 81.6 |
| SVM_NL | 83.3 | 86.5 | 80.0 |
| ***Pos-ion-mode (n=360)*** | | | |
| SVM | 72.2 | 64.9 | 80.0 |
| SVM_NL | 73.6 | 78.4 | 68.6 |
| ***Neg-ion mode (n=232)*** | | | |
| SVM | 81.9 | 81.1 | 82.9 |
| SVM NL | 80.6 | 81.1 | 80.0 |

**Table 16. Averaged LOOCV specificity and sensitivity (%): Feature selection methods applied to training subsampling of dataset.**

| **Classifier** | **Feature Selection** | **Test Accuracy** | **Sensitivity** | **Specificity** |
|---|---|---|---|---|
| ***Multimode (n=592)*** | | | | |
| SVM | SVMRFE | 69.4 | 70.3 | 68.6 |
| SVM | L1SVM | 76.4 | 78.4 | 74.3 |
| SVM_NL | SVMRFE_NL | 80.6 | 83.8 | 77.1 |
| SVM_NL | SVMRW | 70.8 | 67.6 | 74.3 |
| ***Pos-ion-mode (n=360)*** | | | | |
| SVM | SVMRFE | 72.2 | 64.9 | 80.0 |
| SVM | L1SVM | 70.8 | 70.3 | 71.4 |
| SVM NL | SVMRFE_NL | 66.7 | 73.0 | 60.0 |
| SVM_NL | SVMRW | 59.7 | 62.2 | 57.1 |
| ***Neg-ion mode (n=232)*** | | | | |
| SVM | SVMRFE | 80.6 | 86.5 | 74.3 |
| SVM | L1SVM | 75.0 | 83.8 | 65.7 |
| SVM_NL | SVMRFE_NL | 73.6 | 78.4 | 68.6 |
| SVM_NL | SVMRW | 69.4 | 70.3 | 68.6 |

Experiments designed to test the effect of the bagging strategy on the prediction performance were also performed (bootstrap sampling was repeated 101 times, i.e. T = 101). The LOOCV evaluation results (Table 17) indicate that bagging does not boost the best prediction performance (80.6%). Although it did improve the classification accuracy for the data with certain feature selection methods (highlighted in bold), it also reduced the classification accuracy for other cases (highlighted in italics). Due to these observations and its high computational cost, the bagging process was not evaluated in further tests.

**Table 17. Averaged LOOCV prediction performance with bagging (%): Feature selection methods applied to training subsampling of dataset.**

| **Performance** | **SVMRFE** | **L1SVM** | **SVMRFE_NL** | **SVMRW** |
|---|---|---|---|---|
| ***Multimode (n=592)*** | | | | |
| | **72.2** | **79.2** | 80.6 | 70.8 |
| ***Pos-ion-mode (n=360)*** | | | | |
| | *70.8* | **73.6** | *65*.*3* | **61.1** |
| ***Neg-ion mode (n=232)*** | | | | |
| | 80.6 | *70.8* | **76.4** | *66*.*7* |

### Statistical significance of prediction and feature selection

The statistical confidence of the prediction performance of SVM classifers for the multimode dataset with LOOCV evaluation as compared to a random classifier was investigated using a permutation test. The statistic of interest was the observed difference in classification accuracy. Permutation test (T= 1000) showed that the classification accuracy differences between linear SVM and a random classifer, as well as that between a polynomial kernel SVM (degree 2) and a random classifier, were statistically significant (p-value = 0), while the difference between linear SVM and polynomial kernel SVM was not (p-value - 0.32). Details are summarized in Figure 12 where the red dotted line indicates the observed statistic of interest (such as classification accuracy difference) and a blue bar describes the frequency at a given value of the statistic of interest from the permutation test.

The statistical significance of the observed classification accuracy (Table 10) was also evaluated. This is captured by the null hypothesis *(Hₒ)* where the performance statistics of a classifier on the true data are consistent with the performance statistics of the classifier on the data with randomly assigned classes. The statistic of interest is the classification performance. The permutation test (T=1000) showed that the results with SVM classifiers are statistically significant (p-value = 0).

Further assessment of the statistical significance of prediction performance (Table 11) subsequent to feature selection (with feature selection applied on the whole dataset) was performed. The permutation test in this case was designed as follows: at the t^{th} test, i) a dataset *Dₜ* was generated by random label permutation on the original dataset Do, ii) each feature selection method A was applied to the dataset *Dₜ* to select an optimal feature subset *F_{A,t},* and iii) the prediction performance *P_{F,A,t}* on the dataset *Dₜ* with features in *F_{A,t}* was measured using LOOCV evaluation. The permutation test (T = 100) results indicate a p-value of 0.94 for SVMRFE (i.e. for 94% of the dataset with random label permutation, the method was able to find a feature subset that achieves at least as good a classification accuracy as it did on the original dataset); while SVMRFE_NL had a p-value of 0.11. These results again demonstrated the effect of selection bias in feature selection as indicated by Furlanello et al, 2003 (Furlanello, et al., BMC Bioinformatics, 4:54 (2003)). Therefore, these feature selection methods were further evaluated through validation. L1SVM (p-value = 0.04) and SVMRW (p-value = 0.02) appeared to be less affected by selection bias.

A statistical comparison between the tested feature selection methods was performed to determine if SVMRFE_NL > SVMRFE > L1SVM > SVMRW, as observed in previous experiments. A > B denotes that the feature selection results of method A generally outperform that of method B in prediction accuracy. The descriptor used in this permutation test was *P_{Fa}* - *P_{FB},* the difference between the prediction performance on the dataset with the feature subset output by methods A and B, respectively. The prediction performance difference between the SVMRFE NL and SVMRFE methods was statistically significant (p-value = 0.01, Figure 13) while the other observed prediction performance differences were not (Figure 14). These results were probably affected by the selection bias of applying feature selection to the whole dataset, therefore, statistical comparison between feature selection methods were also conducted in a more conservative way, i.e. through validation, as described below.

The statistical significance of prediction performance (Table 13) subsequent to feature selection in the more conservative setting (with feature selection applied only to the training subsampling of each cross-validation) was also assessed. First, the feature selection methods were applied to the training subsampling of the dataset to determine the optimal feature subset. Next, the prediction accuracy on the test subsampling of the dataset (nonoverlapping with the training subsampling) was obtained using the SVM model built on the training subsampling with only the selected features. The statistic of interest is the average prediction accuracy over the LOOCV procedure. The permutation test (T = 100) showed that the feature selection results of L1SVM were statistically significant (p-value = 0, see Figure 15A). Due to the heavy workload of the involved computations for the iterative methods SVMRFE, SVMRFE_NL and SVMRW over LOOCV evaluation, permutation tests to analyze the statistical significance of these methods were not conducted. Instead, L1SVM was compared with t2-statistics. In this statistical comparison, for each validation of LOOCV evaluation process, L1SVM was applied to the training set to select out *k* features and the prediction accuracy on the test set with these k features was obtained. Next, another set of *k* features using t2-statistics computed on the training set was selected and the prediction accuracy of the test set with the selected features was measured. The results (T = 100) showed that the classification accuracy differences between the feature selection results of L1SVM (76.4%) and t2 statistics (59.7%) could be considered statistically significant (p-value = 0.08, Figure 15B).

For completeness, the stability of the feature selection results over the LOOCV folds was evaluated. At each cross-validation, a feature subset was obtained; hence the frequency of occurrence of features in these feature subsets was collected. Utilizing this frequency required the concepts of stable features, features with an occurrence frequency over a certain threshold (80% was used here), and stability, the ratio of stable features in the union of the selected feature subsets during cross-validations. Out of the 73 features selected by L1SVM during LOOCV evaluation, 39 were found to be stable (53.4% stability), SVMRFE had 16 stable features out of 90 (stability of 17.8%), SVMRFE NL had 26 stable features out of 82 (stability of 31.7%) and SVMRW had 33 stable features out of 77 (stability 42.9%). The statistical significance of the features' stability (Ancona, et al., BMC Bioinformatics, 7:387 (2006))) was further evaluated using the stability statistics of feature selection results on the data with random label permutation over the LOOCV evaluation process as the statistic of interest. The results of the permutation tests (T=100) show that the stability of the L1SVM method was statistically significant with a p-value of 0.01 (see Figure 15C). Because of the intensive computations involved, statistical analyses of stability for the SVMRFE, SVMRFE_NL and SVMRW methods were not performed.

### Metabolite identification on selected features

The calculated neutral masses, species investigated, and retention times of the positive and negative ion mode ESI variables used by the multimode SVMRFE NL model are reported in Tables 18 and 19. This model consists of the relatively stable features (threshold 54%) obtained over the LOOCV folds as described above, here threshold 54% was used because there is a significant drop of feature occurrence frequency from 39 to 22. Tables 18 and 19 also list the corresponding chemical formulae, mass differences (Δm), and matching scores for these features.

**Table 18: Tentative identifications for SVMRFE_NL-selected features from multimode dataset detected in positive ion mode ESI. Matches to identified compounds were made using accurate mass measurements and isotope cluster matching. For species which could not be matched against metabolite databases, the top-five matching formulae (according to score) are listed (for features matching fewer than five formulae, all formulae are shown). ^{A}**

| Neutral Mass (Da) | Species Invest. | RT (min) | Estimated Formulae (in order of decreasing score) | Mass Accur. (ppm) | Score (%) | Potential Metabolite(s) Identified | Source | Spectra |
|---|---|---|---|---|---|---|---|---|
| 148.0129 | [M+H]⁺ | 116.8812 | C₄N₆O, C₅H₉OPS, C₄H₈N₂S₂ | 0.1-11.7 | 99.5-90.1 | | | 16A |
| 204.0695 | [M+H]⁺ | 116.8743 | C₁₂H₁₃OP, C₆H₁₂N₄O₂S, C₁₄H₈N₂, C₁₁H₁₂N₂S | 3.7-12.8 | 96.5-91.5 | | | 16B |
| 278.1434 | [M+CH₃CN +Na]⁺ | 144.2175 | C₈H₁₄N₁₂, C₁₀H₂₄N₄OP₂, C₁₁H₂₃N₂O₄P,C₁₂H₁₉N₆P, C₇H₁₈N₈O₄ | 3.1-13.9 | 96.3-99.0 | | | 16C |
| 495.3210 | [M+H]⁺ | 109.6750 | C₂₁H₄₆N₅O₆P, C₂₁H₄₅N₅O₈, C₁₈H₃₇N₁₅O_{2,} C₁₉H₃₇N₁₃O₃, C₂₀H₄₇N₇O₃P₂ | 1.1-13.7 | 99.7-98.8 | | | 16D |
| 519.3330 | [M+H]⁺ | 100.1739 | C₂₆H₅₀NO₇P | 1.0 | 99.0 | 3 PCA(18:2/0:0) isomers (e.g. LysoPC(18:2(9Z,12Z)) | See footnote (B) | 16E |
| 757.5678 | [M+H]⁺ | 127.8454 | C₄₂H₈₀NO₈P | 7.5 | 83.3 | 31 glycerophospholipid isomers (e.g. PE-NMe(18:1(19E)/18:1(9E))) | See *footnote* (C) | 16F |
| 759.5775^{D} | [M+Na]⁺ | 138.3808 | C₄₂H₈₂NO₈P | 0.4 | 42.6 | 18 glycerophosphocholine isomers (e.g. PC(14:0/20:1(11Z))) | See footnote (E) | 16G |
| 781.5595^{D} | [M+H]⁺ | 138.3808 | C₄₄H₈₀NO₈P | 3.4 | 46 | 32 glycerophosphocholine isomers (e.g. PC(14:0/22:4(7Z,10Z,13Z,1 6Z))) | See footnote (F) | 16G |
| 787.6000^{G} | [M+Na]⁺ | 136.6754 | C₄₄H₈₆NO₈P | 11.6 | 74.6 | 22 glycerophosphocholine isomers (e.g. PC(14:0/22:1(13Z))) | See footnote (H) | 16H |
| 932.6173 | [M+NH₄]⁺ | 143.6995 | C₅₄H₉₄O₅P₂S, C₅₃H₈₈O₁₁S, C₅₂H₈₈N₂O₁₀S, C₅₄H₉₅O₄P₃S, C₅₂H₈₄N₁₁O₅S | 1.0-13.5 | 97.3-96.6 | | | 161 |

| | | | | | | | | |
|---|---|---|---|---|---|---|---|---|
| **(A)** For species having multiple isomers the following nomenclature is given: # isomers found including name of isomer *[sources (cross-listed source, if any)].* **(B)** 3 isomers found including **PC(18:2/0:0)** *[LMGP 01050036 (HMDB 10386), 01050034, and 01050035].* **(C)** 31 isomers found including **PE-NMe (18:1/18:1)** *[LMGP 02010331 (MMCD cq_7959), 02010333, 02010338, 02010350],* **PC(16:0/18:2)** *[LMGP 01010585, 01010586, 01010587, 01010588, 01010589, 01010590, 01010591, 01010592, 01010593, 01010594, 01010595, 01010596],* **PC(16:1118:1)** *[LMGP 01010678, 01010680, 01010687, 01010688, 01010689],* **PC(17:1/17:1)** *[LMGP 01010726, 01010727, 01010728],* **PC(18:0/16:2(2E,4E))** *[LMGP 01010 745],* **PC(18:1/16:1)** *[LMGP 01010886, 01010887],* **PC(18:2/16:0)** *[LMGP 01010920, 01010926, 01010932, 01010933].* (D) Adduct analysis yielded several possible ion species for the selected feature. Only species having tentative matches are listed. (E) 18 isomers found including **PC(14:0/20:1(11Z))** *[HMDB 07879],* **PC(16:0/18:1)** *[LMGP 01010005, 01010575, 01010576, 01010577, 01010578, 01010579, 01010580, 01010581, D1010582, 01010583, 01010584],* PC(16:1/18:0) *[LMGP 01010679, 01010686],* **PC(18:0/16:1(9Z))** *[LMGP 01010744],* **PC(18:1/16:0)** *[LMGP 01010874, 01010884, 01010885].* (F) 32 isomers found including **PC(14:0/22:4(7Z,10Z,13Z,16Z))** *[HMDB 07889],* PC(16:0/20:4) *[LMGP 01010007, 01010629, 01010630, 01010631],* **PC(18:0/18:4)** *[LMGP 01010772, 01010773, 01010774, 01010775, 01010776],* PC(18:1/18:3) *[LMGP 01010897, 01010898, 01010899]*, **PC(018:2118:2)** *[LMGP 01010918, 01010919, 01010921, 01010922, 01010923, 01010924, 01010925, 01010927, 01010928, 01010929, 01010930, 01010937, 01010938, 01010939],* **PC(18:3/18:1)** *[LMGP 01010949, 01010955],* **PC(20:4/16:0)** *[LMGP 01011049, 01011050, 0.10110506].* (G) Adduct analysis yielded several possible ion species for the selected feature. Only 1 species could be tentatively identified. (H) 22 isomers found including **PC(14:0/22:1(13Z))** *[HMDB 07887]*, **PC(16:0/20:1(11Z))** *[LMGP 01010618]*, **PC(18:0/18:1)** *[LMGP 01010749, 01010750, 01010751, 01010752, 01010753, 01010754, 01010755, 01010756, 01010757, 01010758, 01010759, 01010760, 01010761, 01010762, 01010763],* **PC(18:1/18:0)** *[LMGP 01010840, 01010875, 01010888, 01010889],* **PC(20:1(11Z)116:0)[U]** *[LMGP 01011037].* | | | | | | | | |

**Table 19. Tentative identifications for SVMRFE_NL-selected features from multimode dataset detected in negative ion mode ESI. Matches to identified compounds were made using accurate mass measurements and isotope cluster matching. For species which could not be matched against metabolite databases, the top matching formulae (according to score) are listed (for features matching fewer than five formulae, all formulae are shown).^{A}**

| Neutral Mass (Da) | Species Invest. | RT (min) | Estimated Formulae (in order of decreasing score) | Mass Accur. (ppm) | Score (%) | Potential Metabolite(s) Identified | Source | Spectra |
|---|---|---|---|---|---|---|---|---|
| 256.2398 | [M-H]⁻ | 104.6898 | C₁₆H₃₂O₂ | 1.7 | 96.3 | 16 carboxylic acid isomers (e.g. palmitic acid) | See footnote (B) | 17A |
| 274.1710 | [M-H]⁻ | 39.2953 | C₁₄H₂₇O₃P, C₁₃H₂₈N₂P₂, C₁₆H₂₂N₂O₂, C₁₀H₂₃N₆OP, C₁₃H₂₆N₂O₂S | 1.8-14.3 | 99.0-95.2 | | | 17B |
| 280.2446 | [M-H]⁻ | 133.2433 | C₁₅H₃₈P_{2,} c₁₅h₃₆o₂s, C₁₁H₃₂N₆S | 0.9-13.2 | 93.7-91.4 | | | 17C |
| 280.2460 | [M-H]⁻ | 98.8490 | C₁₅H₃₈P₂, C₁₅H₃₆O₂S | 4.1-8.6 | 95.0-94.4 | | | 17D |
| 282.2154*^{C}* | [M-H]⁻ | 139.6963 | C₁₇H₃₀O₃ | 14.5 | 99.3 | 12-hydroxy-8E, 10Eheptadecadienoic acid | MID 35560 | 17E |
| 284.2701*^{D}* | [M-H]⁻ | 123.8672 | C₁₈H₃₆O₂ | 5.0 | 96.1 | 12 carboxylic acid isomers (e.g. stearic acid) | See footnote (E) | 17F |
| 340.2489 | [M-H]⁻ | 130.1342 | C₂₀H₃₈P₂, C₂₀H₃₇O₂P, C₂₂H₃₂N₂O, C₁₇H₃₂N₄O₃, C₁₆H₃₃N₆P | 4.3-12.4 | 98.1-95.4 | | | 17G |
| 354.1676 | [M-H]⁻ | 42.4019 | C₁₄H₂₂N₆O₅ | 6.9 | 95.4 | 6 peptide isomers (e.g. GlnHisAla) | See footnote (F) | 17H |
| 368.1652^{G} | [M-H]⁻ | 85.4803 | C₁₉H₂₈O₅S | 1.4 | 93.1 | 2 isomers (e.g. DHEA Sulfate) | See footnote (H) | 17I |
| 384.2831 ^{I} | [M+CH₃COO ]⁻ | 90.7391 | C₂₆H₄₀S, C₂₃H₄₄S₂, C₂₁H₄₀N₂O₂S, C₂₉H₃₆, C₁₈H₄₄N₂O₂S₂ | 3.4-13.9 | 94.0-85.9 | | | 17J |
| 398.2982^{I} | [M+HCOO]⁻ | 90.7391 | C₂₇H₄₂S, C₂₄H₄₆S₂, C₂₂H₄₂N₂O₂S, C₂₅H₃₈N₂O₂, C₁₉H₄₆N₂O₂S₂ | 3.8-14 | 94.0-86.9 | | | 17J |
| 433,3256^{J} | [M+HCOOr | 91.9683 | C₂₆H₄₃NO₄ | 14.8 | 98.8 | Lithocholic acid glycine conjugate | HMDB 00698^{K} | 17K |
| 444.3037^{I} | [M-H]⁻ | 90.7391 | C₂₄H₄₀N₆S, C₂₈H₄₅PS, C₂₈H₄₄O₂S, C₂₅H₄₉PS₂, C₂₅H₄₈O₂S₂ | 0,45-13.0 | 94.1-91.9 | | | 17J |
| 479.3310^{J} | [M-H]⁻ | 91.9683 | C₂₄H₅₀NO₆P | 13.7 | 96.6 | 8 glycerophosphocholine isomers (e.g. PC(P-16:0/0:0)) | See footnote (L) | 17K |
| 481.2835 | [M-H]⁻ | 106.0719 | C₂₂H₄₄NO₈P | 6.3 | 90.4 | 10 glycerophosphocholine isomers (e.g. PC(10:0/4:0)) | See footnote (M) | 17L |
| 481.3047 | [M-H]⁻ | 116.2758 | C₁₂H₃₅N₁₇O4, C₁₂H₃₆N₁₇O₂P, C₁₇H₅₀N₅O₄P₃, C₁₆H₃₉N₁₁O₆, C₁₇H₅₁N₅O₂P₄ | 2.2-14.9 | 95.1-93.4 | | | 17M |
| 499.9613 | [M-H]⁻ | 166.3375 | C₂₁H₈O₁₃S, C₂₁H₉O₁₁PS, C₂₀H₁₀N₂O₈P₂S, C₁₉H₂₂P₆S₂, C₁₈H₄N₄O₁₂S | 22.0-14.5 | 96.6-96.1 | | | 17N |
| 505.2842 | [M-H]⁻ | 100.0856 | C₂₂H₄₇N₅P₄, C₂₂H₄₆N₅O₂P₃, C₁₇H₃₁N₁₇O₂, C₂₀H₃₆N₁₃OP, C₁₉H₄₁N₉O₃P₂ | 0.9-12.1 | 99.5-97.9 | | | 170 |
| 505.330S^{N} | [M+CH₃COO ]⁻ | 147.7737 | C₂₈H₄₉N₃OP₂, C₂₉H₄₉NO₂P₂, C₂₇H₃₉N₉O, C₂₉H₄₈NO₄P, C₂₅H₄₄N₇O₂P | 2.5-13.8 | 94.0-92.6 | | | 17S |
| 507.3131 | [M-H]⁻ | 112.7721 | C₂₈H₄₅NO₇, C₂₈H₄₆NO₅P, C₂₆H₄₀N₅OPS, C₂₇H₄₅N₃O₄S, C₂₆H₃₇N₉O₂ | 0.1-12.8 | 97.3-96.2 | | | 17P |
| 509.3156 | [M-H]⁻ | 121.2736 | C₂₄H₄₈NO₈P | 7.6 | 91.8 | 6 glycerophospholipid isomers (e.g.PE(9:0/10:0)) | See footnote (O) | 17Q |
| 519.3459^{N} | [M+HCOO]⁻ | 147.7737 | C₂₆H₄₆N₇O₂P, C₂₇H₅₇NP₄, C₂₉H₅₁N₃OP₂, C₂₆H₄₅N₇O₄, C₂₇H₄₅L₅N₅O₅ | 1.7-14.2 | 93.3-92.8 | | | 17S |
| 529.2699 | [M-H]⁻ | 105.7854 | C₂₆14₄₃NO₈S | 1.9 | 82.7 | 3 carboxylic acid isomers (e.g. glycoursodeoxycholic acid 3-sulfate) | See footnote (P) | 17R |
| 563.3363^{N} | [M-H]⁻ | 147.7737 | C₂₆H₄₇N₉OP₂, C₂₄H₃₇N₁₇, C₂₈H₅₇NO2P₄, C₂₅H₃₇N₁₅O, C₂₇H₄₆N₇O₄P | 2.7-10.2 | 94.0-93.0 | | | 17S |
| 683.5089^{Q} | [M+CH₃COO ]⁻ | 140.4283 | C₃₇H₆₆N₉OP, C₃₉H₇₇P₄, C₃₉H₇₆NO₂P₃, C₃₄H₆₂N₁₃P, C₃₈H₆₆N₇O₂P | 0.1-14.7 | 88.7-87.9 | | | 17T |
| 697.5246^{Q} | [M+HCOO]⁻ | 140.4283 | C₃₅H₆₄N₁₃P, C₄₀H₇₉NP₄, C₃₄H₆₃N₁₅O, C₃₆H₇₄N₇P₃, C₃₅H₆₃N₁₃O₂ | 2.7-14.5 | 88.6-88.1 | | | 17T |
| 743.5300^{Q} | [M-H]⁻ | 140.4283 | C₃₇H₇₇N₇P₄, C₃₅H₆₆N₁₅OP, C₃₆H₇₆N₉OP₃, C₃₈H₈₇NP₆, C₃₇H₇₆N₇O₂P₃ | 1.6-14.7 | 88.7-88.2 | | | 17T |
| 757-5457^{Q} | [M-CH₃]⁻ | 140.4283 | C₃₉H₈₉NP₆, C₃₇H₇₈N₉OP₃, C₃₈H₇₉N₇P₄, C₃₉H₈₈NO₂P₅, C₃₂H₆₃N₂₁O | 4.8-14.5 | 88.8-88.2 | | | 17T |

| | | | | | | | | |
|---|---|---|---|---|---|---|---|---|
| **(A)** For species having multiple isomers the following nomenclature is given: # isomers found including **name of isomer** *[source (cross-listed source, if any)].* **(B)** 16 isomers found including **palmitic acid** *[LMFA 01010001 (HMDB 00220)],* **isopalmitic acid** *[LMFA 01020010],* **2,6-dimethyl-tetradecanoic acid** *[LMFA 01020038],* **2,8-dimethyl-tetradecanoic acid** *[LMFA 01020039],* **3-methyl-pentadecanoic acid** *[LMFA 01020164],* **2-propyl-tridecanoic acid** *[LMFA 01020165],* **2-hexyl-decanoic acid** *[LMFA 01020166],* **3-ethyl-3-methyl-tridecanoic acid** *[LMFA 01020167],* **2-heptyl-nonanoic acid** *[LMFA 01020168],* **6-ethyltetradecanoic acid** *[LMFA 01020169],* **2,4-dimethyl-tetradecanoic acid** *[LMFA 01020170],* **3,5-dimethyl-tetradecanoic acid** *[LMFA 01020171],* **4-hexyldecanoic acid** *[LMFA 01020172],* **2-ethyl-2-butyl-decanoic acid** *[LMFA 01020173],* **13-methyl-pentadecanoic acid** *[LMFA 01020192],* **4,8,12-trimethyltridecanoic acid** *[LMFA 01020249].* **(C)** Adduct analysis yielded multiple possible ion species for this feature. Only 1 species could be tentatively identified. **(D)** Adduct analysis yielded multiple possible ion species for this feature. Only 1 species could be tentatively identified **(E)** 12 isomers found including **stearic acid** *[HMDB 00827 (LMFA 01010018, MID 189, MMCD cq_00998)],* **10-methyl-heptadecanoic acid** *[MID 4292 (LMFA 01020013)],* **(+)-isostearic acid** *[MID 4293 (LMFA 01020014)],* **2,6-dimethyl-hexadecanoic acid** *[MID 4324 (LMFA 01020042)],* **4,8-dimethyl-hexadecanoic acid** *[MID 4325 (LMFA 01020043)],* **2,14-dimethyl-hexadecanoic acid** *[MID 4326 (LMFA 01020044)],* **4,14-dimethyl-hexadecanoic acid** *[MID 4327 (LMFA 01020045)],* **6**,**14**-**dimethyl**-**hexadecanoic acid** *[MID 4328 (LMFA 01020046)],* **lambda isostearic acid** *[MID 4493 (LMFA 01020093)],* **neostearic acid** *[MID 4620 (LMFA 01020094)],* **11,15-dimethyl-hexadecanoic acid** *[MID 34604 (LMFA 01020175)],* **15-methyl-heptadecanoic acid** *[MID 34632 (LMFA 01020205)].* **(F)** 6 isomers found including **Gln His Ala** *[MID 23091],* **Gln Ala His** *[MID 22217],* **Ala His Gln** *[MID 21229],* **Ala Gln His** *[MID 16023],* **His Gln Ala** *[MID 20595],* **His Ala Gln** *[MID 18707].* **(G)** Adduct analysis yielded multiple possible ion species for this feature. Only 1 species could be tentatively identified **(H)** 2 isomers found including **DHEA sulfate** *[HMDB 01032 (LMST 05020010)],* **testosterone sulfate** *[HMDB 02833].* **(I)** Adduct analysis yielded multiple possible ion species for this feature. All are listed as none could be matched against the databases. **(J)** Adduct analysis yielded multiple possible ion species for this feature. Only species that could be tentatively identified are listed. **(K)** Cross-listed as MMCD cq-10750 and MID 5666. **(L)** 8 isomers found including **PC(P-16:0/0:0)** *[HMDB 10407 (LMGP 01070006)],* **PC(O-16:1/0:0)** *[LMGP 01050100, 01050101, 01050102, 01050103, 01050104, 01070004, 01070005].* **(M)** 10 isomers found including **PC(10:0/4:0)** *[LMGP 01010403],* **PC(12:0/2:0)** *[LMGP 01010443],* **PC(6:0/8:0)** *[LMGP 01011233, 01011234],* **PC(7:0/7:0)** *[LMGP 01011238, 01011239, 01011240],* **PC(8:016:0)** *[LMGP 01011248, 01011249],* **PC(9:0/5:0)** *[LMGP 01011269].* **(N)** Adduct analysis yielded multiple possible ion species for this feature. All are listed as none could be matched against the databases. **(O)** 6 isomers found including **PE(9:0/10:0)[U]** *[MID 40490 (LMGP 02010091)],* **PE(10:019:0)[U]** *[MID 40669 (LMGP 02010272)],* **PC(14:012:0)** *[LMGP 01010504],* **PC(8:0/8:0)** *[LMGP 01011251, 01011252, 01011253].* **(P)** 3 isomers found including **glycoursodeoxycholic acid 3-sulfate** *[HMDB 02409 MMCD cq_17361, MID 6670)],* **glycochendeoxycholic acid 7-sulfate** *[HMDB 02496 (MMCD cq_17159, MID 6692)],* **glycochendeoxycholate-3-sulfate** *[HMDB 02497 MMCD cq_17507, MID 6702)].* **(Q)** Adduct analysis yielded multiple possible ion species for this feature. All are listed as none could be matched against the databases. | | | | | | | | |

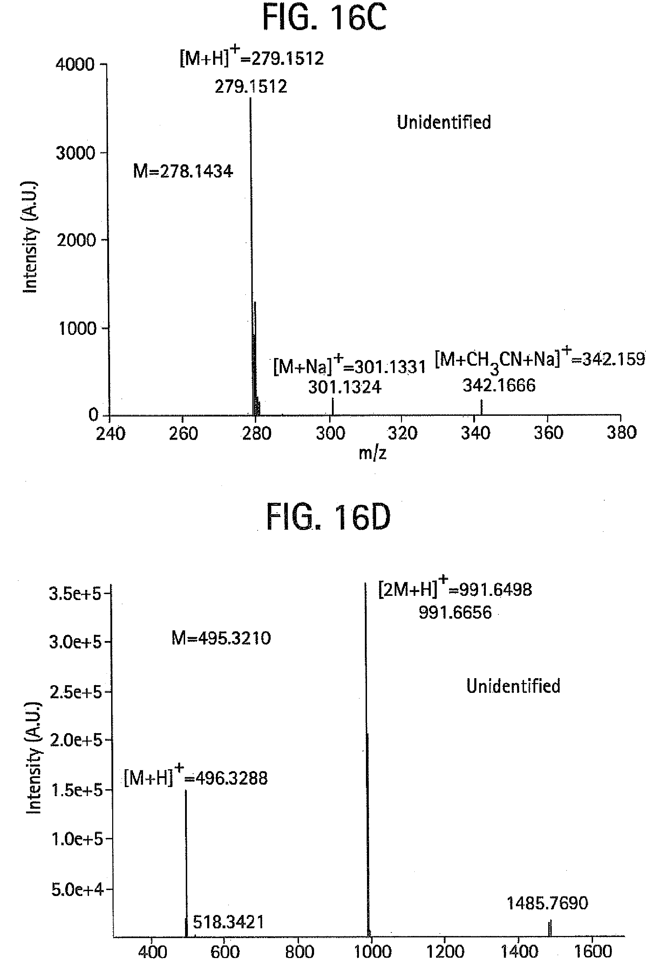

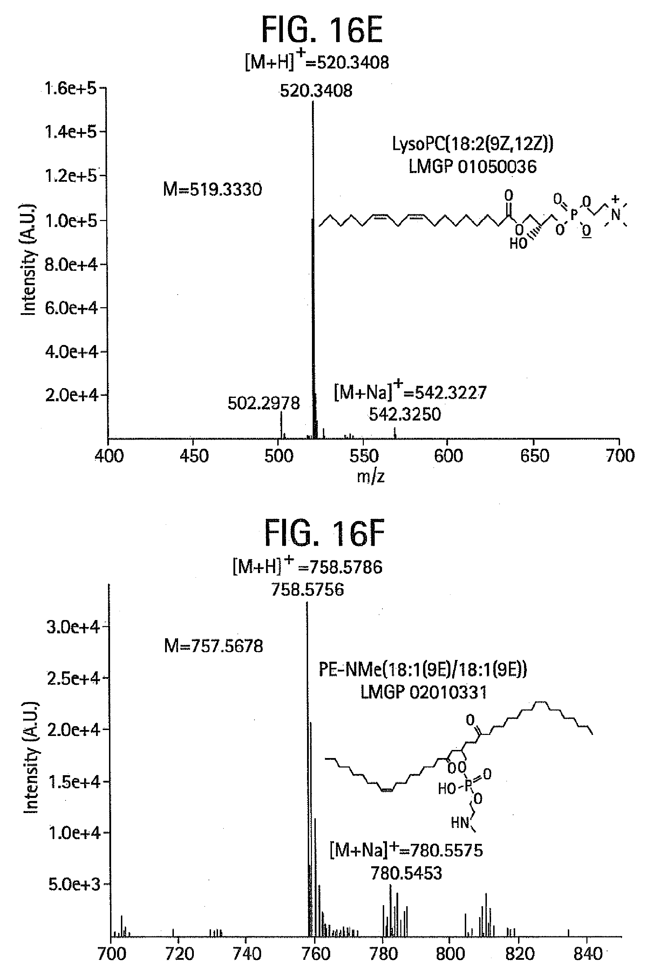

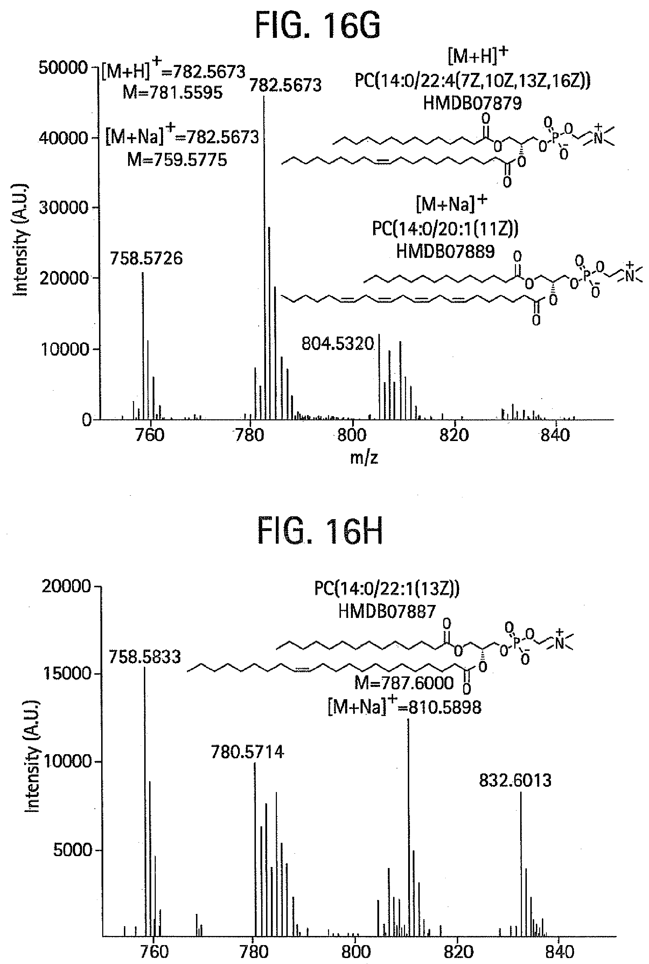

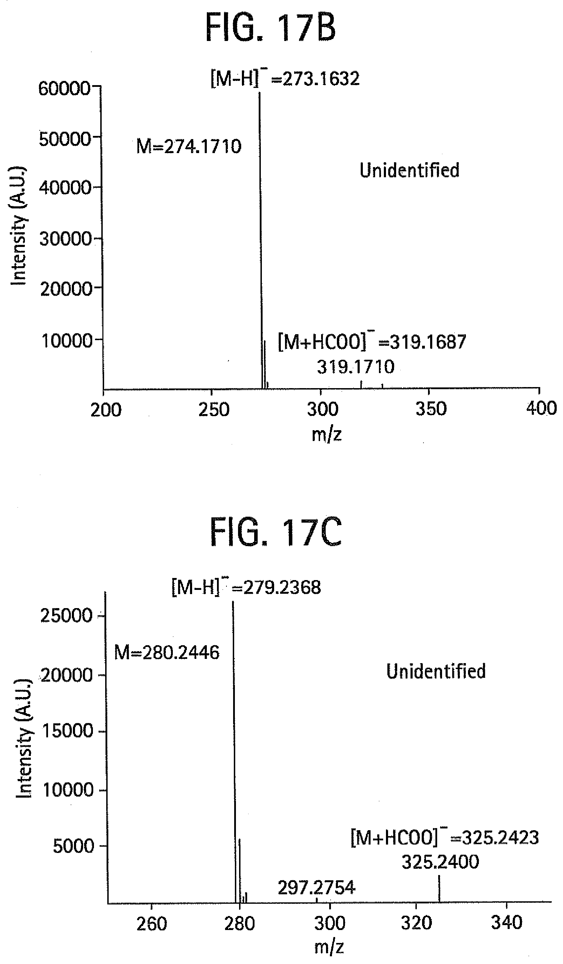

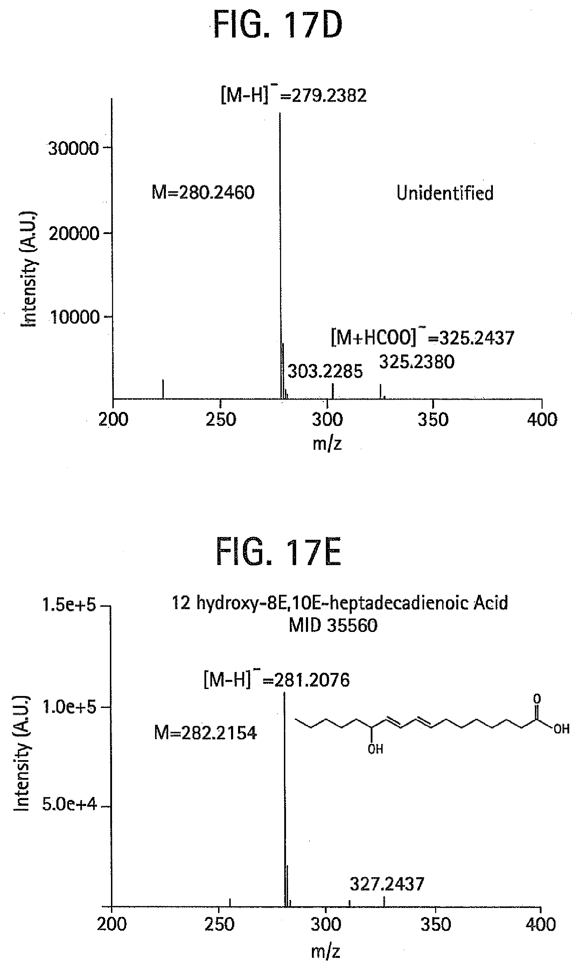

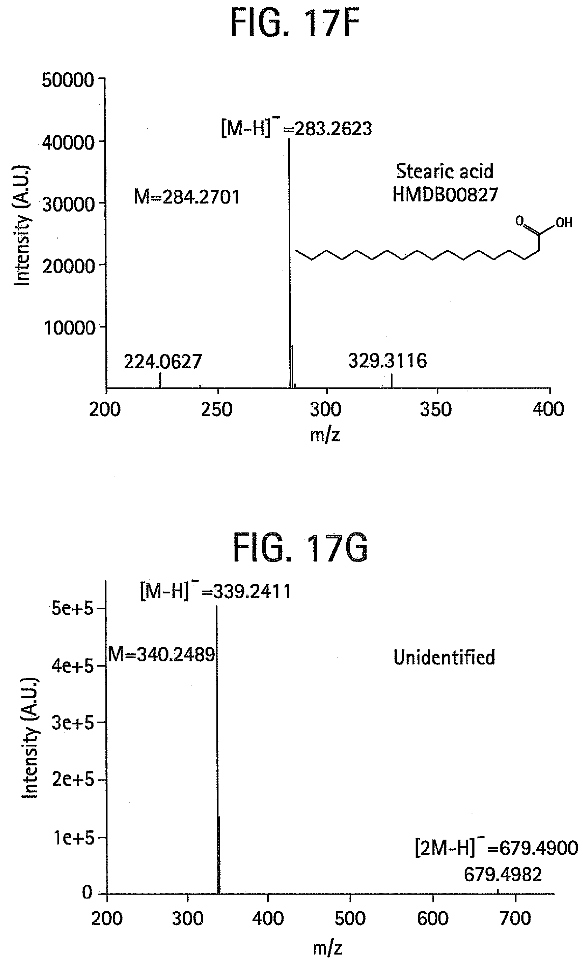

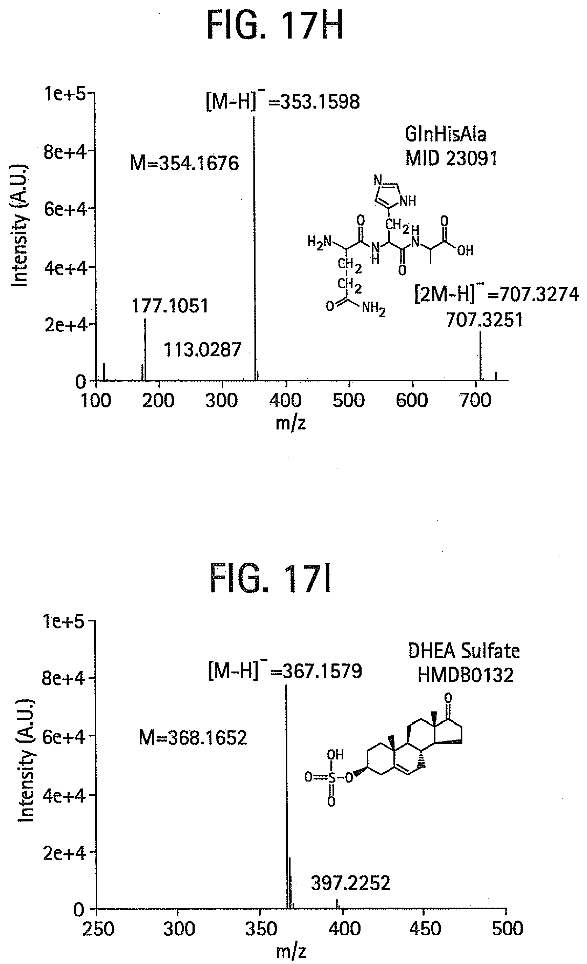

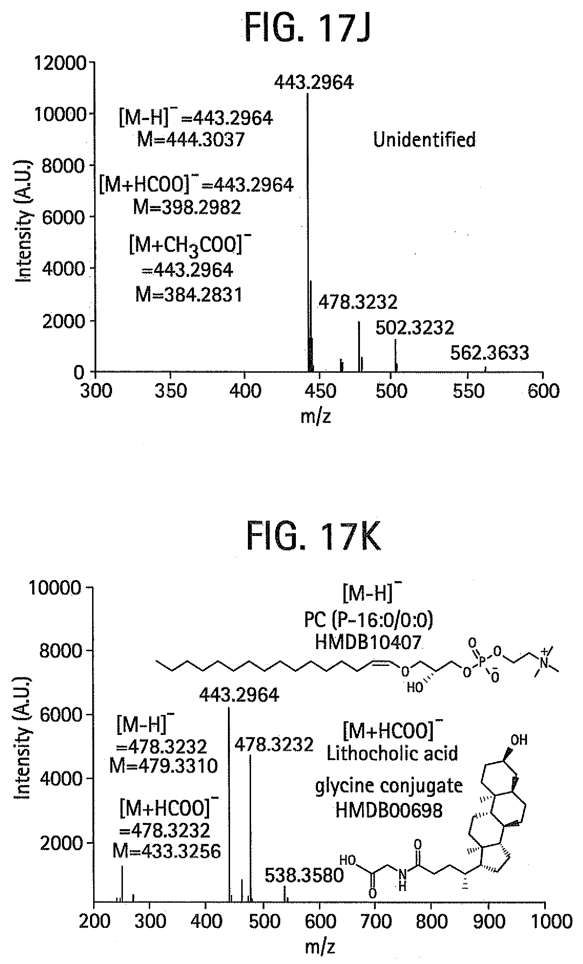

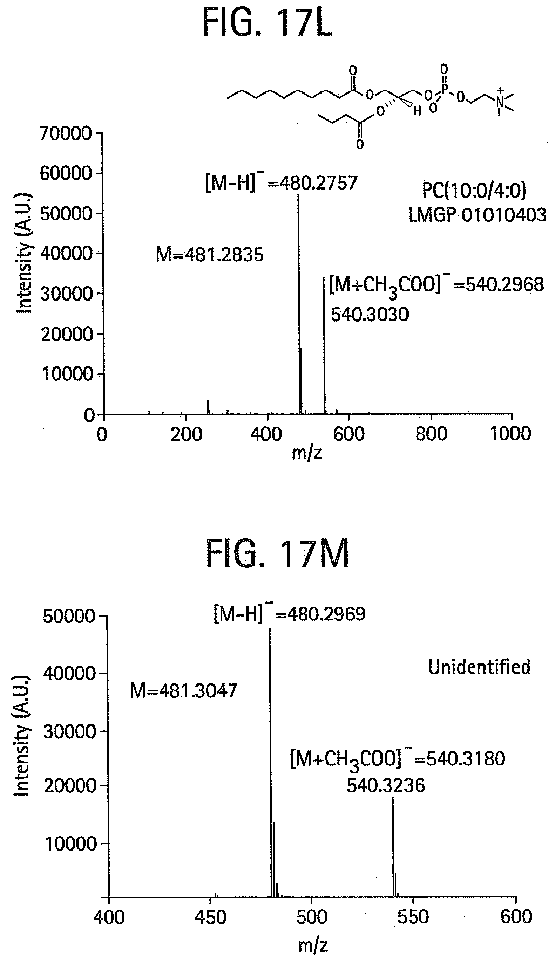

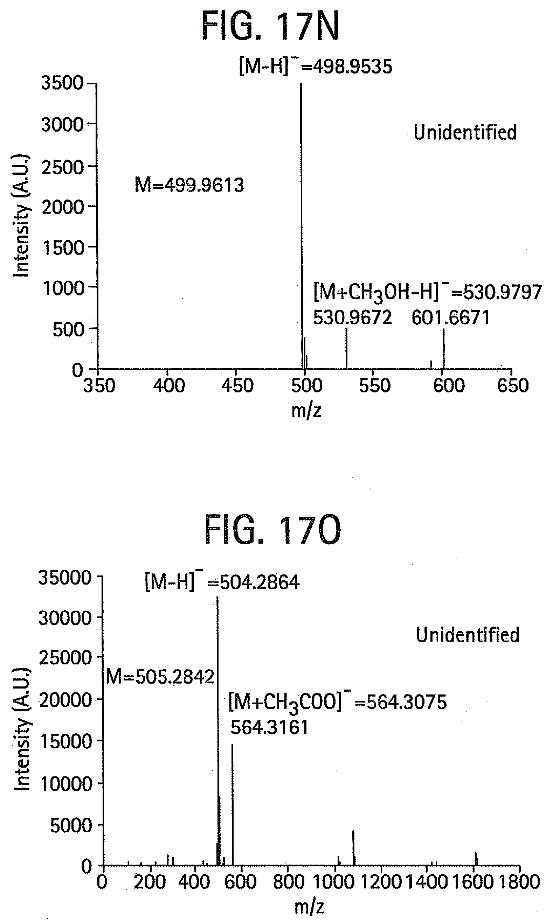

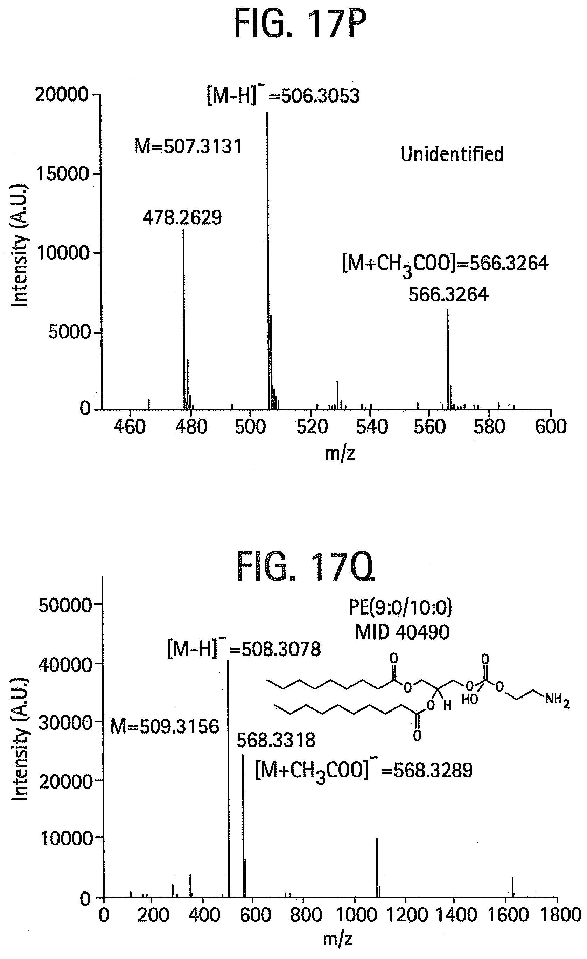

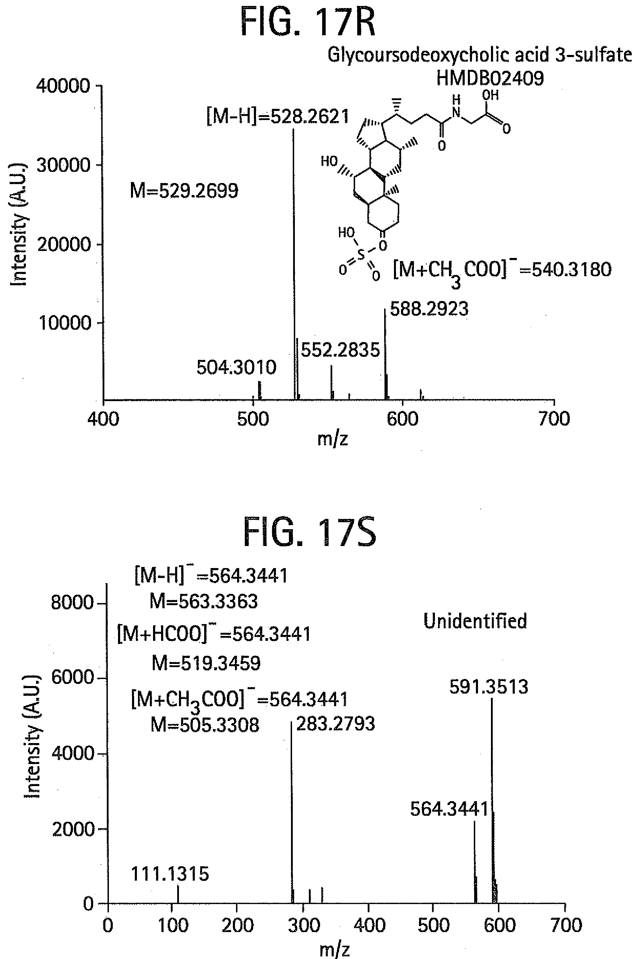

The corresponding mass spectra and structures are shown in Figures 16 and 17. Adduct analysis of the 18 and 27 features selected from positive and negative ESI modes, respectively, provided a total of 29 unique features to search against the databases as 16 features were found to be redundant.

Five of the SVMRFE_NL-selected positive ion mode ESI features from the multimode dataset were tentatively identified as glycophospholipids. Due to the inability of single stage MS analysis to distinguish between isomeric compounds (compounds having identical chemical formula but different structures), the features could not be definitively assigned to a particular glycophospholipid isomer. As such, all of the possible isomers corresponding to each feature are listed in Table 18. The chemical formulae corresponding to these five features yielded a total of 106 possible compounds with the total number of isomers attributed to each feature ranging from 3-32, mass accuracies between 0.4-11.6 ppm and matching scores between 42.6-99.0%. Examples of compounds that could be tentatively matched to the elemental formulae obtained in this investigation include LysoPC(18:2 (9Z,12Z), PE-NMe(18:1 (19E)118:1 (9E)), PCA(14:0120:1 (11Z)), PCA(14:0/22:4 (7Z,10Z,13Z,16Z)), and PC(14:0/22:1 (13Z)).

Nine of the SVMRFE_NL-selected negative ion mode ESI features were tentatively identified as endogeneous carboxylic acids, peptides, glycerophospholipids, and hormones. The total number of isomers for these nine features ranged from 1-16 yielding a total of 65 possible compounds with mass accuracies between 1.4-14.8 ppm and matching scores between 82.7-99.3%. One of the identified features could not be assigned to a single chemical formulae due to the absence of additional supporting adduct ions in the mass spectrum. This feature was attributed to either lithocholic acid glycine conjugate or any of 8 glycerophosphocholine isomers, such as PC(P-16:010:0). Potential matches for the possible identities of the selected features include palmitic acid, 12-hydroxy-8E,10E-heptadecadienoic acid, stearic acid, GlnHisAla, DHEA sulfate, PC(10:4/4:0), PE(9:0110:0) and glycoursodeoxycholic acid 3-sulfate.

Although metabolites such as lysophosphatidic acid and lipid associated sialic acid, that have been investigated as metabolic biomarkers, for ovarian cancer in literature (Baker, et al., J. Am. Med. Assoc., 287(23):3081-2 (2002); Sutphen, et al., Cancer Epidem. Biomarkers Prevention, 13(7):1185-91 (2004); Xu, et al., J. Am. Med. Assoc., 280(8):719-23 (1998); Petru, et al., Gynecol. Oncol., 38(2):181-6 (1990); Schutter, et al., Tumour Biol.: J. Int. Soc. Oncodevelopmental Biol. Med., 13(3):121 (1992); Schwartz, et al., Cancer, 60(3):353-61 (1987); Tadros, et al., Am. Coll. Obstet. Gynecol. J., 74(3):379-83 (1989); Vardi, et al., Surg. Fynecol. Obstet., 168(4):296-301 (1989)) were not pinpointed in the study, the presence of several endogenous lipids as well as other endogenous metabolites in the set of selected features suggests that this approach has merit and should be further explored.

### Example 3. Optimization of a direct analysis in real time/time-of-light mass spectrometry method for rapid serum metabolomic fingerprinting.

### Materials and Methods:

### Samples and reagents

N-trimethylsilyl-N-methyltrifluoroacetamide (MSTFA) and trimethylchlorosilane (TMCS) were obtained from Alfa Aesar (Ward Hill, MA), anhydrous pyridine, acetonitrile (ACN), acetone and isopropanol were from EMD Chemicals (Gibbstown, NJ), polyethylene glycol standard 600 (PEG 600) was from Fluka Chemical Corp. (Milwaukee, WI), healthy human serum (S7023-50mL) was from Sigma-Aldrich Corp. (St. Louis, MO), and helium (99.9% purity) was purchased from Airgas, Inc. (Atlanta, GA).

### Mass spectrometry

Serum metabolomic analysis was performed in positive ion mode *via* a DART ion source (IonSense, Saugus, MA) coupled to a JEOL AccuTOF orthogonal time-of-flight (TOF) mass spectrometer (JEOL, Japan). Derivatized serum samples were placed within the ionization region using a home-built sampling arm which secured Dip-it tips (InSense, Saugus, MA) at a fixed 3 mm distance from the ion source gas exit. Prior to DART MS analysis, 0.5 µL of derivatized serum solution were pipette-deposited onto the glass end of the Dip-tip coupled to the sampling arm, a 1.2 min data acquisition run started, and the sample allowed to air dry for 0.65 min. The sampling arm was then rapidly switched so that the dried sample was exposed to the ionizing zone of the DART ion source. After 0.9 min, the sample was removed, and a new Dip-it placed on the sample holder, while the remaining 0.3 minutes of the run were completed.

Following optimization, a DART ion source helium flow rate of 3.0 L min⁻¹ heated to 200°C was chosen. The glass tip-end was positioned 1.5 mm below the mass spectrometer inlet. A discharge needle voltage of +3600 V, and perforated and grid electrode voltages of +150 and +250 V were chosen, respectively. Accurate mass spectra were acquired in the *m*/*z* 60-1000 range with a spectral recording interval of 1.0 s. The RF ion guide peak voltage was set to 1200 V. The settings for the TOF mass spectrometer were as follows: ring lens: +8 V, orifice 1: +40 V, orifice 2: +6 V, orifice 1 temperature: 80°C, and detector voltage -2800 V. Mass drift compensation was performed after analysis of each sample using a 0.20 mM PEG 600 standard in methanol. The measured resolving power of the TOF mass spectrometer was 6000 at FWHM, with observed mass accuracies in the range 2-20 ppm, depending on the signal-to-noise ratio (S/N) of the particular peak under investigation. Metabolites were tentatively identified by matching accurate masses against a custom built database containing 2924 entries corresponding to unique endogenous human metabolites. Each entry was manually expanded to take into account the mono, di and/or tri-trimethylsilane (TMS) derivatives. Entries for families of compounds not reacting with the MSTFA/TMCS reagent mixture were not expanded. Matching of database records to experimental data was performed using the SearchFromList application part of the Mass Spec Tools suite of programs (ChemSW, Fairfield, CA) using a tolerance of 5 mmu. If no matches were found, the METLIN database was manually searched with a tolerance of 10 mmu.

### Sample preparation

Upon removal from a -80° C freezer, serum samples were immediately thawed on ice. Two-hundred µL serum aliquots were pipetted and mixed with 1 mL of freshly-prepared, chilled (-18°C) and degassed 2:1 (v/v) acetone:isopropanol mixture. The mixture was vortexed and placed in a second freezer at -18° C overnight to precipitate proteins, followed by centrifugation at 13,000 g for 5 minutes. The supernatant was transferred to a clean centrifuge tube, and the solvent was evaporated in a speed vacuum concentrator to complete dryness. The solid residue was then redissolved in 25 µL anhydrous pyridine, and shaken for one hour at room temperature for complete dissolution. Fifty µL of MSTFA containing 0.1% TMCS were added to the sample in a N₂-purged glove box. The mixture was incubated at 50°C in an inert N₂ atmosphere for half an hour, resulting in derivatization of amide, amine and hydroxyl groups. The supernatant of this derivatized mixture was subject to DART mass spectrometric analysis, each sample requiring approximately 1.2 min.

### Results:

### Effect of serum metabolite derivatization

A comparison of DART mass spectra observed for non-derivatized human serum following protein precipitation and an identical sample which was derivatized with MSTFA/TMCS is shown in Figure 18. Only a few intense signals were obtained from non-derivatized serum (Figure 18B), while more than one thousand five hundred recognizable signals were detected from derivatized serum (Figure 18A). Underivatized serum was characterized by presenting signals in a more restricted mass range *(m*/*z* 60-400), whereas for derivatized serum signals up to *m*/*z* 990 were detected due to the enhanced volatility of the TMS metabolite derivatives. Increased volatility facilitates thermal desorption prior to chemical ionization within the region between the DART ion source exit and the mass spectrometer inlet. Overall signal intensity was increased by a factor of 20 following derivatization. S/N were also dramatically improved, not only due to the higher signal intensity, but also due to a cleaner baseline. Peaks with S/N higher than 20% of the base peak (peak labeled "5") are highlighted in Figure 18A. Table 20 lists their tentative identities based on accurate mass matching.

**Table 20. Tentative matching of peaks selected from Figure 1(a) via accurate mass measurements.**

| Index | Measured Ions *(m*/*z)* | Ion Type | Experimental MW (Da) | Theoretical MW (Da) | Accuracy (ppm) | Estimated Formulae | Name | Source |
|---|---|---|---|---|---|---|---|---|
| 1 | 133.0807 | [M+TMS+H]⁺ | 60.0334 | 60.0324 | 16.6 | CH₄N₂O | Urea | HMDB00294 |
| 2 | 188.1075 | [M+TMS+H]⁺ | 115.0622 | 115.0633 | 9.6 | C₅H₉NO₂ | L-Proline | HMDB00162 |
| 3 | 274.1282 | [M+2TMS+H]⁺ | 129.0413 | 129.0426 | 10.1 | C₅H₇NO₃ | Pyroglutamic acid | HMDB00267 |
| 4 | 361.1669 | Not Identified | | | | | | |
| 5 | 369.3494 | [M+TMS+H]⁺ | 296.3020 | 296.3079 | 19.9 | C₂₀14₄₀O | 11Z-eicosen-1 -ol | MID36508 |
| 6 | 413.3421 | Not Identified | | | | | | |
| 7 | 431.3534 | [M+TMS+H]⁺, [M+2TMS+H]⁺ | 358.3060 | 358.3083 | 6.4 | C₂₁H₄₂O₄ | MG(18:0/0:0/0:0) | HMDB11131 |
| 8 | 487.2468 | [M+TMS+H]⁺ | 414.1995 | 414.2049 | 13.0 | C₁₇R₃₀N₆O₄S₁ | Lys Met His^{a} | MID23058 |
| 9 | 503.3900 | [M+TMS+H]⁺ | 430.3426 | 430.3447 | 4.9 | C₂₈H₄₆O₃ | 1α-hydroxy-25-methoxyvitamin D₃ | MID42264 |
| 10 | 540.2606 | [M+2TMS+H]⁺ | 395.1737 | 395.1693 | 11.1 | C₁₈H₂₅N₃O₇ | Thr Glu Phe^{b} | MID23502 |
| 11 | 559.2862 | [M+2TMS+H]⁺ | 414.1993 | 414.2049 | Same as Index 8 | | | |
| 12 | 568.2883 | [M+2TMS+H]⁺ | 423.2014 | 423.2006 | 1.9 | C₂₀H₂₉N₃O₇ | Tyr Leu Glu^{c} | MID22177 |
| 13 | 612.2983 | [M+3TMS+H]⁺ | 395.1713 | 395.1693 | Same as Index 10- | | | |
| 14 | 620.3029 | [M+2TMS+H]⁺ | 475.2160 | 475.2179 | 4.0 | C₂₁H₂₉N₇O₆ | Trp Arg Asp^{d} | MID20771 |
| 15 | 640.3305 | [M+2TMS+H]⁺ | 495.2436 | 495.2482 | 9.3 | C₂₆H₃₃N₅O₅ | Trp Lys Tyr^{e} | MID21781 |
| 16 | 654.3449 | Not Identified | | | | | | |

| | | | | | | | | |
|---|---|---|---|---|---|---|---|---|
| ^{a} 6 isomers found including Lys Met His: His Lys Met, Lys His Met, Met His Lys, Met Lys His and His Met Lys; ^{b} 12 isomers including Thr Glu Phe: Tyr Val Asp, Val Asp Tyr, Glu Thr Phe, Asp Tyr Val, Tyr Asp Val, Val Tyr Asp, Asp Val Tyr, Phe Thr Glu, Thr Phe Glu, Glu Phe Thr and Phe Glu Thr; ^{c} 12 isomers including Tyr Leu Glu: Tyr Glu Ile, Ile Tyr Glu, Ile Glu Tyr, Glu Tyr Leu, Leu Tyr Glu, Glu Ile Tyr, Tyr Glu Leu, Glu Tyr Ile, Leu Glu Tyr, Glu Leu Tyr and Tyr Ile Glu; ^{d} 6 isomers including Trp Arg Asp: Arg Trp Asp, Asp Arg Trp, Arg Asp Trp, Trp Asp Arg and Asp Trp Arg; ^{e} 6 isomers including Trp Lys Tyr: Lys Tyr Trp, Lys Trp Tyr, Tyr Lys Trp, Trp Tyr Lys and Tyr Trp Lys. | | | | | | | | |

Among the sixteen peaks marked as "1"-"16", thirteen of them were identified as peptides, amino acids, lipids, vitamin D₃ metabolites, fatty acid alcohols and urea. This indicates that analysis of TMS derivatized metabolites is preferable to their more hydrophilic underivatized counterparts bearing functional groups such as -COOH, -OH, -NH and -SH, in which intermolecular hydrogen bonding interactions are strong, and result in their decreased volatility. Derivatization replaces reactive hydrogen atoms in these groups by TMS, leading to a reduction in metabolite polarity.

### Effect of helium gas flow rate and temperature

Helium gas temperature and flow rate are two major parameters affecting DART ion transmission (Harris and Fernández, Anal. Chem., 81:322-329 (2009)). DART spectra for various helium gas temperatures, and the corresponding number of metabolites identified by accurate mass matching are shown in Figures 19A and 19B, respectively. As temperature was increased, the number of metabolites found was also observed to increase up to 200° C. It is important to note that temperature values refer to set values in the software, but that the local temperature where the sample is exposed to the ionizing gas stream has been measured and calculated to be lower (Harris and Fernández, Anal. Chem., 81:322-329 (2009)). To verify the effect of temperature, three randomly chosen signals with different *m*/*z* values spanning the observed mass range were selected. A plot of their S/N versus temperature is displayed in Figure 19C, showing that the optimum temperature falls in the range of 150-200° C depending on the *m*/*z* of these metabolites. High gas temperatures accelerate sample drying and analyte thermal desorption rates, thus increasing the sensitivity of detection, but too high temperature (>250° C) can cause metabolites to desorbe too quickly, resulting in signal loss if the spectral acquisition rate is not high enough. High gas temperatures also lead to partial sample charring on the glass capillary surface, leading to irreversible sample degradation.

Helium flow rates were also observed to have a strong influence on the observed DART spectra (Figure 20). The number of metabolites detected increased with increased flow rate, but high gas flows (> 3 LPM) dispersed sample particles and remaining solvent directly onto the mass spectrometer inlet, thus contaminating the orifice. Moreover, high flow gas is conducive to strong turbulence and affected the reproducibility of the experiments. The S/N plots for the ionic signals previously studied indicated an optimum helium flow rate between 2.5 and 3.0 LPM.

### Time-dependence of metabolite desorption/ionization

Although the underlying mechanisms prevailing in the DART desorption process are complicated and beyond the topic of this note, the observed temporal profiles following exposure of the derivatized serum sample to the ionizing gas stream suggest a differential thermal desorption mechanism during the first 5 s following switching of the position of the sampling arm. Mass spectra averaged every 1 s of the total ion chronogram (TIC, Figure 21 A) are shown in Figures 21B (a-h). At early times (Figure 21B (a), only a few intense signals were detected, corresponding mostly to light ions such as protonated urea-2TMS *(m*/*z* 205.12), 3-phosphoglyceraldehyde-2TMS *(m*/*z* 315.10) and the peptide Tyr-Pro-Phe-2TMS (or isomers, *m*/*z* 570.29). Examination of the mass spectra obtained between 40 and 44 s (Figure 21B (b-e)), showed that these four signals decreased in intensity with increasing exposure time until completely disappeared after 42 s (Figure 21B (d)). For spectra collected between 42 s and 44 s a large quantity of signals with medium intensities at masses between *m*/*z* 150 and 800 were observed, followed by an overall decay in signal intensity at the trailing edge of the transient TIC signal. Ions with m/z between 450 and 600 in the mass spectra shown in Figure 21B (f-h) were tentatively matched to protonated lipid 1-octadecanoyl-rac-glycerol-2TMS (*m*/*z* 503.39) and peptide Lys-Met-His (or isomers)-2TMS (*m*/*z* 559.2856). Their ionic signals lasted several seconds without obvious decrease, suggesting a relatively high concentration. Following these experiments, we determined an optimum time interval for spectrum averaging that spans regions "c" through "e" in the TIC. However, it must be noted that this interval may vary depending on the type of sample holder used, mass range of the metabolites of interest, and He flow rate and temperature.

### Repeatability

Highly repeatable measurements are critical in serum metabolomic fingerprinting since potential biomarkers of stress or disease are down-selected based on significance tests or multivariate analysis of intensity information directly obtained from mass spectra. Repeatability experiments based on ten separate runs of a control serum sample are presented in Figure 21C and 21D. A CV of 4.5% was obtained for the TIC peak heights shown in Figure 21C. Relative signal intensities also showed good reproducibility across all spectra (Figure 21D), with an average CV of 18.9% and 16.7% for the two peaks marked with asterisks, respectively.

### Example 4. Rapid mass spectrometric metabolic profiling of blood sera detects ovarian cancer with high accuracy.

### Materials and Methods:

### Sample collection

Serum samples were obtained from the Ovarian Cancer Institute (OCI, Atlanta, GA) after approval by the Institutional Review Board from Northside Hospital and Georgia Institute of Technology, Atlanta, GA (HO5002 John McDonald PI). All donors were required to fast and to avoid medicine and alcohol for 12 h prior to sampling, except for certain allowable medications, for instance, diabetics were allowed insulin. Following informed consent by donors, 5 mL of whole blood are collected by venipuncture into evacuated blood collection tubes that contained no anticoagulant. Blood was drawn and centrifuged within an hour of serum collection, 200 µL aliquots of each serum sample was stored into 1.5 mL Safe-Lock Eppendorf micro test tubes at -80° C until ready to use.

### Sample preparation

Prior to analysis, 200 µL of each serum sample was thawed on ice and mixed with 1 mL of freshly-prepared, chilled (-18°C) and degassed 2:1 (v/v) acetone:isopropanol mixture. The mixture was vortexed and proteins allowed to precipitate at -18° C overnight followed by centrifugation at 13,000 g for 5 minutes. The supernatant was transferred to a new centrifuge tube, and the solvent was evaporated in a speed vac. The solid residue was re-dissolved in 25 µL anhydrous pyridine (EMD Chemicals, Gibbstown, NJ), and shaken for one hour at room temperature for complete dissolution. Fifty µL of N-trimethylsilyl-N-methyltrifluoroacetamide (MSTFA, Alfa Aesar, Ward Hill, MA) containing 0.1% trimethylchlorosilane (TMCS, Alfa Aesar) was added to the sample in a N₂-purged glove box. The mixture was then incubated at 50° C in an inert N₂ atmosphere for half an hour, resulting in TMS-derivatization of amide, amine and hydroxyl groups. The final derivatized mixture was subject to DART-MS analysis.

### DART-TOF MS

Serum mass spectrometric analysis was performed using a DART ion source (IonSense Inc., Saugus, MA) coupled to a JEOL AccuTOF orthogonal time-of-flight (TOF) mass spectrometer (JEOL Inc., Japan). Derivatized serum samples (0.5 µl) were pipette-deposited onto the glass end of a Dip-tip® applicator (IonSense, Inc.), allowed to air dry for 0.65 minutes in a fume hood and exposed to the ionizing protonated water cluster reagent ions of the DART ion source. Each sample was run in triplicate, requiring a total of analysis time of 4.0 minutes.

The DART ion source was operated in positive ion mode with a helium gas flow rate of 3.0 L min⁻¹ heated to 200° C. The glass tip-end was positioned 1.5 mm below the mass spectrometer inlet. The discharge needle voltage of the DART source was set to +3600 V, and the perforated, and grid electrode voltages set to +150 and +250 V, respectively. Accurate mass spectra were acquired within the range of *m*/*z* 60-1000 with a spectral recording interval of 1.0 s, and an RF ion guide peak voltage of 1200 V. The settings for the TOF mass spectrometer were as follows: ring lens: +8 V, orifice 1 : +40 V, orifice 2: +6 V, orifice 1 temperature: 80° C, and detector voltage -2800 V. Mass drift compensation was performed after analysis of each sample using a 0,20 mM polyethylene glycol standard 600 standard (PEG 600, Fluka Chemical Corp., Milwaukee, WI) in methanol. The measured resolving power of the TOF MS detector was 6000 at FWHM, with observed mass accuracies in the range 2-20 ppm, depending on signal-to-noise ratios (S/N) of the particular peak investigated.

### Data preprocessing

All profile mass spectra were obtained by time-averaging of the total ion chronogram between 0.73 and 0.76 minutes after each injection. Following DART-TOF MS data collection, mass drift compensation was performed using PEG 600 as the reference spectrum. The background spectrum was subtracted; profile spectral data was exported in JEOL-DX format and converted to a comma-separated format prior to importing in MATLAB 7.6.0 (R2008a, MathWorks). The data were normalized to a relative intensity scale and re-sampled to a total of 20,000 points between *m*/*z* 60 and 990 using the *msresample* function in the Matlab Bioinformatics Toolbox. The three replicate DART spectra were then averaged.

### Multivariate classification

SVM and PLSDA analysis of averaged spectra were performed in MATLAB 7.6.0. PLSDA is performed using the PLS Toolbox (Version 4.1, Eigenvector Research) for MATLAB.

### Description of fSVM classification method

Support Vector Machines (SVM) (Vapnik, The Nature of Statistical Learning Theory, (Springer, New York, 2000)) have been successfully used in many scientific applications, as they generally achieve state-of-the-art classification performance, particularly versus older methods and in high-dimensional settings. Though computationally intensive, they are efficient enough to handle problems of the size considered here. Given a dataset $S = {\left\{{x}_{i}, {y}_{i}\right\}}_{i = 1}^{M}$ (*xᵢ* ∈ *R^{N}* is the feature vector of *i*^{th} instance and *yᵢ* is the corresponding label), for two-class classification problems, the standard linear SVM solves the following convex optimization: ${min}_{w , ξ} \frac{1}{2} {‖ w ‖}^{2} + C {\sum }_{i = 1}^{M} {ξ}_{i} s . t . { y}_{i} \left(w⋅{x}_{i}+b\right) + {ξ}_{i} \geq 1 , {ξ}_{i} \geq 0 , i = 1 , ⋯ , M$

In the case of nonlinear SVMs, the feature vectors *xᵢ ∈ R^{N}* are mapped into high dimensional Euclidean space, H, through a mapping function Φ(·) : *R^{N}* → *H*. The optimization problem becomes: ${min}_{w , ξ} \frac{1}{2} {‖ w ‖}^{2} + C {\sum }_{i = 1}^{M} {ξ}_{i} s . t . { y}_{i} \left(w⋅Φ\left({x}_{i}\right)+b\right) + {ξ}_{i} \geq 1 , {ξ}_{i} \geq 0 , i = 1 , ⋯ , M$

The kernel function is defined as *K*(*xᵢ*, *xⱼ*) = Φ(x*ᵢ*) · Φ(x*ⱼ*) -- for example, for a polynomial kernel of degree 2, *K*(*xᵢ*,*xⱼ*) = (*gxᵢ* · *xⱼ* + *r*)², where g, r are kernel parameters. The linear kernel function is defined as *K*(*xᵢ*, *xⱼ*) *= xᵢ* · *xⱼ*. Tools such as libSVM (http://www.csie.ntu.edu.tw/cjlin/libsvm) can efficiently solve the dual formation of the following problem: ${min}_{α} \frac{1}{2} {\sum }_{i = 1}^{M} {y}_{i} {y}_{j} {α}_{i} {α}_{j} K \left({x}_{i}, {x}_{j}\right) - {\sum }_{i = 1}^{M} {α}_{i} s . t . {\sum }_{i = 1}^{M} {y}_{i} {α}_{i} = 0 , 0 \leq {α}_{i} \leq C , i = 1 , ⋯ , M$ where α*ᵢ* is the Lagrange multiplier corresponding to the *i*^{th} inequality in the primal form. The solution is $w = {\sum }_{i = 1}^{M} {α}_{i} {y}_{i} Φ \left({x}_{i}\right)$ (in the case of linear SVM, $w = {\sum }_{i = 1}^{M} {α}_{i} {y}_{i} {x}_{i} ) .$ The optimal decision function for an input vector x is *f*(*x*) = *w* · Φ(*x*) + *b*, that is, $f \left(x\right) = {\sum }_{i = 1}^{M} {α}_{i} {y}_{i} K \left({x}_{i}x\right) ,$ where the predicted class is +1 if *f(x) > 0* and -1 otherwise.

In functional classification problems, the input data instances *Xᵢ* are random variables that take values in an infinite dimensional Hilbert space H, the space of functions. The goal of classification (Biau, et al., IEEE Transactions on Information Theory, 51:2163-2172 (2005)) is to predict the label y of an observation X given training data $\left(S={\left\{{X}_{i}, {y}_{i}\right\}}_{i = 1}^{M} , { X}_{i} ∈ H\right) .$

In practice, the functions that describe the input data instances *X₁*,··· , *X_{M}* are never perfectly known. Often, n discretization points have been chosen in *t₁*,···,*t_{N}* ∈ *R*, and each functional data instance *Xᵢ* is described by a vector in *R^{N}*, (*Xᵢ* (*t*₁),···, *Xᵢ*(*t_{N}*))*.* Sometimes, the functional data instances are badly sampled and the number and the location of discretization points are different between different functional data instances. A usual solution under this context is to construct an approximation (such as B-spline interpolation) for each input functional data instance *Xᵢ* based on its observation values, and then apply sampling uniformly to the reconstructed functional data (Visintin, et al., Clin. Cancer Res., 14:1065-1072 (2008); Greene, et al., Clin. Cancer Res., 14: 7574-7575 (2008)). Therefore, a simple solution would be to apply the standard SVM to the vector representation of the functional data.

However, in some application domains such as chemometrics, it is well known that the shape of a spectrum is sometimes more important than its actual mean value. Therefore, it is beneficial to design SVMs specifically for functional classification, by introducing functional transformations and function kernels (Williams, et al., J. Proteome Res., 6:2936-2962 (2007); Anderson, and Anderson, Mol. Cell Proteomics, 1:845-867 (2002).
3. Apply functional transformation, projection *P_{V^{N}},* on each observation *Xᵢ* as *P_{V^{N}}* (*Xᵢ*) = *xᵢ* = (*x*_{*i*1},···*, x_{iN}*) with *Xᵢ* approximated by ${\sum }_{k = 1}^{N} {x}_{ik} {Ψ}_{k} ,$ where {Ψₖ}_{*k*≥1} is a complete orthonormal basis of the functional space H
4. Build a standard SVM on the coefficients *xᵢ* ∈ *R^{N}* for all *i* = 1,···, *M*.

This procedure is equivalent to working with a functional kernel, *K_{N}* (*xᵢ*, *xⱼ*) defined as *K*(*P_{V^{N}}* (*Xᵢ*), *P_{V^{N}}* (*Xⱼ*)), where *P_{V^{N}}* denotes the projection onto the N-dimensional subspace *V^{N}* ∈ *H* spanned by {Ψₖ}_{*k*=1},···,*N*, and *K denotes* any standard SVM kernel.

Good candidates for the basis functions include the Fourier basis and wavelet bases. If the functional data are known to be nonstationary, a - wavelet basis might yield better results than the Fourier basis. Other good choices include B-spline bases, which generally perform well in practice (Rossi and Villa, Neurocomputing, 69:730-742 (2006).

### Metabolite identification

Metabolites in the fSVM model utilizing 1:7:20,000 subsampled features were tentatively identified by finding the closest mass spectral peak matching the selected model features in the 103-714 *m*/*z* range. This *m*/*z* range is fully covered by the TOF calibration function thus providing the most reliable accurate mass matches. No attempt was made to identify SVM model features outside this range. Accurate masses of mass spectral peaks closest to the model features were matched against a custom built database containing 2924 entries corresponding to endogenous human metabolites in the HMDB database. Each entry was manually expanded to take into account the mono, di and/or tri-trimethylsilane (TMS) derivatives. Entries for families of compounds not reacting with the MSTFA/TMCS reagent mixture were not expanded. Matching of database records to experimental DART-TOF MS data was performed using the SearchFromList application part of the Mass Spec Tools suite of programs (ChemSW, Fairfield, CA) using a tolerance of 10 mmu. If no matches were found, the next closest match within 20 mmu was selected.

### Results:

The approach used here circumvents chromatographic separation, making use of *non-contact direct ionization* with minimum sample preparation and no matrix addition. The assay is based on Direct Analysis in Real Time (DART) MS (Cody, et al., Anal. Chem., 77:2297-2302 (2005)), an innovative technique where a stream of excited metastables is used to desorb and chemically ionize a dried drop of metabalite mixture solution extracted from serum. A mass spectrometer is used to evaluate the relative abundances of these metabolites. The method displays no memory effects, as it is performed in a non-contact fashion. This increases the reproducibility of the metabolic fingerprints, enabling the detection of differences between disease states. Moreover, DART is able to ionize a broad range of metabolites with varying polarities (Cody, Anal. Chem., 81:1101-1107 (2009)), enabling the simultaneous interrogation of multiple species.

The results from the application of a rapid methodology to the detection of metabolic changes associated with ovarian cancer are presented here. This study was approved by the Institutional Review Boards of Georgia Institute of Technology and Northside Hospital, (Atlanta) from which the patient blood samples (Table 21) were obtained.

**Table 21. Patient cohort characteristics.**

| **Characteristics** | **Stages I-II** | **Stages III -IV** | **Controls^{a}** | **Total** |
|---|---|---|---|---|
| mean age | 60 | 61 | 52 | 56 |
| papillary serous carcinoma | 5 | 39 | 0 | 44 |
| controls | 0 | 0 | 50 | 50 |

| | | | | |
|---|---|---|---|---|
| ^{a} Controls refer to patients with histology within normal limits (NWL). | | | | |

Peripheral blood was drawn from ovarian cancer and control patients using standardized procedures. Samples were subsequently processed and stored in 200 µl aliquots at -80° C in the tissue bank of the Ovarian Cancer Institute (Atlanta). Following protein precipitation, derivatized metabolites were subject in triplicate to DART mass spectrometric analysis using a time-of-flight (TOF) mass spectrometer (Figure 22). A typical DART-TOP MS metabolic profile displays a multitude of signals corresponding to metabolites rapidly desorbed and ionized in a time-dependent fashion (Figure 22.c.x).

A customized functional Support Vector Machine (fSVM) classification algorithm for the classification of the metabolic profiles for developed for this study. The fSVM operates as follows: 1) The data are collapsed along the desorption time dimension by using the average value within the time range of interest for each mass; 2) The resulting vector is smoothed using B-splines (Eubank, Nonpararnetric Regression and Spline Smoothing, (Marcel Dekker, New York (1988)) to create the functional representation; 3) The vector of spline coefficients is classified by a SVM (Ramsay, and Silverman, Functional Data Analysis, (Springer, New York, (2005)), i.e., using a kernel between a pair of smooth functions. In order to deal with the very large number of features (over 20,000 *m*/*z* values per sample run), a variety of approaches were tested, including simple subsampling, ANOVA feature selection, and recursive feature elimination.

The efficacy of the classifiers was evaluated by leave-one-out cross-validation (LOOCV). Feature selection was performed on each training set. The results of the fSVN analyses (one-way ANOVA with p = 0.05; one-way ANOVA with p = 0.01; selection of 1 from every 7 peaks consecutively across al 20,000 peaks) are presented in Table 22.

**Table 22. Ovarian cancer detection using fSVMs.**

| **Classifier type** | **Feature selection method** | **Number of Features** | **SENS(%)** | **SPEC(%)** | **ACC(%)** |
|---|---|---|---|---|---|
| fSVM | 1:7:20,000 subsampling | 2,858 | **100.0** | **98.0** | **98.9** |
| FSVM_NL | | | 100.0 | 92.0 | 95.7 |
| fSVM | One-way ANOVA (p=0.05) | 4,390^{a} | **100.0** | **98.0** | **98.9** |
| fSVM_NL | | | 100.0 | 96.0 | 97.9 |
| fSVM | One-way ANOVA (p=0.01) | 2,084^{a} | **97.7** | **100.0** | **98.9** |
| fSVM_NL | | | 97.7 | 98.0 | 97.9 |

| | | | | | |
|---|---|---|---|---|---|
| ^{a} Average number of features selected during each CV. | | | | | |

The classifers were evaluated and optimized using LOOCV. ANOVA feature selection in combination with fSVM was first applied only to the training dataset and then the test set predicted using the selected features subset. The sensitivity (SENS), specificity (SPEC) and accuracy (ACC) were determined by true positive (TP) / positive (P), true negative (TN) / negative (N) and (TP+TN) / (P+N), respectively. The best accuracies obtained are shown in bold. fSVM_NL = functional support vector machine with nonlinear (NL) degree 2 polynomial kernel. In each case, the fSVMs yielded an average of only one misclassification in all LOOCV resulting in an accuracy of 98.9 %.

Table 23 presents a summary of analytical results using standard SVMs and partial least-squares discriminant analysis (PLSDA) (Barker and Rayens, J. Chemom., 17:166-173 (2003)), two of the most frequently employed data analysis methods in bioinformatics and chemometrics.

**Table 23. Ovarian cancer detection using standard SVMs.**

| **Classifier type** | **Feature selection method** | **Number of Features** | **SENS(%)** | **SPEC(%)** | **ACC(%)** |
|---|---|---|---|---|---|
| SVM | No | 20,000 | 90.9 | 92 | 91.5 |
| SVM_NL | | | **95.5** | **100** | **97.9** |
| PLSDA_(8LV) | | | 97.7 | 96 | 96.8 |
| SVM | RFE | 15^{a} | 97.7 | 94 | 95.7 |
| SVM | L1SVM | 14^{a} | 97.7 | 96 | 96.8 |
| SVM | SVMRW | 18^{a} | **100** | **96** | **97.9** |
| SVM NL | RFE | 35^{a} | 95.5 | 84 | 89.4 |
| SVM | 1:7:20,000 subsampling | 2,858 | 95.5 | 92.0 | 93.6 |
| SVM NL | | | 93.2 | 92.0 | 92.6 |
| PLSDA (8LV) | | | 93.2 | 90.0 | 91.5 |
| SVM | One-way ANOVA (p=0.05) | 4,390^{a} | 97.7 | 94.0 | 95.7 |
| SVM NL | | | 95.5 | 94.0 | 94.7 |
| PLSDA (8LV) | | | **97.7** | **98.0** | **97.9** |
| SVM | One-way ANOVA (p=0.01) | 2,084^{a} | **97.7** | **98.0** | **97.9** |
| SVM NL | | | 97.7 | 88.0 | 92.6 |
| PLSDA (8LV) | | | 93.2 | 92.0 | 92.6 |

| | | | | | |
|---|---|---|---|---|---|
| ^{a} Average number of features selected during each CV. | | | | | |

Classifiers were evaluated and optimized using LOOCV. Feature selection methods in combination with SVM or PLSDA were applied only to the training dataset and then the test set predicted using the selected features subset. The best prediction accuracies obtained are bolded. SVM_NL = SVM with nonlinear degree 2 polynomial kernel, PLSDA (8LV) = partial least squares discriminant analysis with 8 latent variables, RFE = recursive feature elimination, L1 SVM = L1-norm SVM, SVMRW = SVM following Weston's feature selection.

All methods performed well, owing to the inherent discriminative power of the data but the highest accuracy was obtained using the fSVM approach. In a second set of experiments, a training set of 64 patients was used with 30 held out as a test set. fSVM achieved 100% accuracy, though the LOOCV estimate should be regarded as more reliable. A list of features selected by L1-norm, RFE, 7-element subsampling and ANOVA that fall within the TOF mass spectrometer calibration range, and their tentative identifications is provided in Tables 24-26.

**Table 24. Identification of elemental formulae and metabolites matches in the m/z range 103∼714 derived from features used by the fSVM model with 1:7:20,000 subsampling.**

| **Feature Index in fSVM Model** | **Feature *m*/*z* in Model** | **Closest Peak Matched (*m*/*z)*** | **Ion Type** | **Experimental MW (Da)** | **Theoretical MW (Da)** | **Δm (mmu)** | **Estimated Elemental Formulae** | **Possible Match in Metabolome Databases** | **Source** |
|---|---|---|---|---|---|---|---|---|---|
| 1037 | 108.1764 | 108.0928 | *Not Identified* | | | | | | |
| 1058 | 109.1530 | 109.0994 | *Not Identified* | | | | | | |
| 1079 | 110.1295 | 110.0704 | *Not Identified* | | | | | | |
| 1100 | 111.1061 | 111.056 | *Not Identified* | | | | | | |
| 1121 | 112.0826 | 112.0896 | [M+H]⁺ | 111.0818 | 111.0796 | -2.2 | C₅H₉N₃ | Histamine | MID68 |
| 1142 | 113.0592 | 113.1013 | *Not Identified* | | | | | | |
| 1163 | 114.0357 | 114.0732 | *Not Identified* | | | | | | |
| 1184 | 115.0123 | 115.0967 | *Not Identified* | | | | | | |
| 1212 | 116.3143 | 116.0777 | [M+H]⁺ | 115.0699 | 115.0633 | -6.6 | C₅H₉NO₂ | D-Proline | HML3B00162 |
| 1275 | 119.2440 | 119.0927 | [M+TMS+H]⁺ | 46.0454 | 46.0418 | -3.6 | C₂H₆O | Ethanol | HMDB00108 |
| 1359 | 123.1502 | 123.1186 | *Not Identified* | | | | | | |
| 1380 | 124.1267 | 124.0865 | *Not Identified* | | | | | | |
| 1401 | 125.1033 | 125.1333 | *Not Identified* | | | | | | |
| 1422 | 126.0798 | 126.096 | *Not Identified* | | | | | | |
| 1443 | 127.0564 | 127.1301 | *Not Identified* | | | | | | |
| 1464 | 128.0329 | 128.0456 | *Not Identified* | | | | | | |
| 1555 | 132.2646 | 132.1007 | [M+TMS+H]⁻ | 59.0534 | 59.0484 | -5.0 | CH₅N₃ | Guanidine | HMDB01842 |
| 1576 | 133.2412 | 133.0813 | [M+TMS+H]⁺ | 60.0340 | 60.0324 | -1.6 | CH₄N₂O | Urea | HMDB00294 |
| 1702 | 139.1005 | 139.1499 | *Not Identified* | | | | | | |
| 1723 | 140.0770 | 140.0754 | *Not Identified* | | | | | | |
| 1744 | 141.0536 | 141.1415 | *Not Identified* | | | | | | |
| 1765 | 142.0301 | 142.0894 | *Not Identified* | | | | | | |
| 1814 | 144.3087 | 144.1093 | [M+TMS+H⁺ | 71.0620 | 71.0609 | -1.1 | C₃H₇N₂ | beta-Aminopropionitrile | MID7017 |
| 1856 | 146.2618 | 146.0839 | [M+TMS+H]⁺ | 73.0366 | 73.0528 | 16.2 | C₃H₇NO | 3-aminopropanal | HMDB01106 |
| 1877 | 147.2384 | 147.114 | *Not Identified* | | | | | | |
| 1940 | 150.1680 | 150.1007 | *Not Identified* | | | | | | |
| 1961 | 151.1446 | 151.1414 | *Not Identified* | | | | | | |
| 1982 | 152.1211 | 152.0889 | [M+TMS+H]⁺ | 79.0416 | 79.0422 | 0.6 | C₅H₅N | Pyridine | HMDB00926 |
| 2066 | 156.0273 | 156.0852 | *Not Identified* | | | | | | |
| 2115 | 158.3059 | 158.1132 | *Not Identified* | | | | | | |
| 2178 | 161.2356 | 161.1288 | *Not Identified* | | | | | | |
| 2199 | 162.2121 | 162.0944 | [M+TMS+H]⁺ | 89.0470 | 89.0477 | 0.7 | C₃H₇NO₂ | L-Alanine | HMDB00161 |
| 2304 | 167.0949 | 167.0805 | *Not Identified* | | | | | | |
| 2325 | 168.0714 | 168.094 | *Not Identified* | | | | | | |
| 2367 | 170.0245 | 170.1123 | *Not Identified* | | | | | | |
| 2416 | 172.3031 | 172.1059 | [M+TMS+H]⁺ | 99.0586 | 99.0684 | 9.8 | C₅H₉NO | 2-Piperidinone | HMDB1749 |
| 2458 | 174.2562 | 174.1174 | *Not Identified* | | | | | | |
| 2479 | 175.2328 | 175.1408 | *Not Identified* | | | | | | |
| 2500 | 176.2093 | 176.1053 | [M+TMS+H]⁺, [M+2TMS+H]⁺ | 103.0650 | 103.0633 | -1.7 | C₄H₉NO₂ | L-a-aminobutyric acid | HMDB00452 |
| 2542 | 178.1624 | 178.0987 | [M+TMS+H]⁺, [M+2TMS+H]⁺ | 105.0514 | 105.0426 | -8.8 | C₃H₇NO₃ | L-Serine | HMDB00187 |
| 2584 | 180.1155 | 180.1106 | *Not Identified* | | | | | | |
| 2605 | 181.0921 | 181.1112 | [M+TMS+H]⁺ | 108.0638 | 108.0575 | -6.3 | C₇H₈O | p-Cresol | HMDB01858 |
| 2647 | 183.0452 | 183.0854 | [M+TMS+H]⁺ | 110.0380 | 110.0480 | 10.0 | C₅H₆N₂O | Imidazole-4-acetaldehyde | HMDB03905 |
| 2668 | 184.0217 | 184.1321 | [M+TMS+H]⁺ | 111.0848 | 111.0796 | -5.2 | C₅H₉N₃ | Histamine | HMDB00870 |
| 2696 | 185.3238 | 185.1208 | *Not Identified* | | | | | | |
| 2717 | 186.3003 | 186.1425 | *Not Identified* | | | | | | |
| 2738 | 187.2769 | 187.1185 | [M+TMS+H]⁺ | 114.0712 | 114.0681 | -3.1 | C₆H₁₀O₂ | trans-Hex-2-enoic acid | HMDB0719 |
| 2759 | 188.2534 | 188.1084 | [M+TMS+H]⁺, [M+2TMS+H]⁺ | 115.0610 | 115.0633 | 2.3 | C₅H₉NO₂ | L-Proline | HMDB00162 |
| 2864 | 193.1362 | 193.1822 | *Not Identified* | | | | | | |
| 2885 | 194.1127 | 194.1087 | [M+TMS+H]⁺ | 121.0614 | 121.0528 | -8.6 | C₇H₇NO | Benzamide | HMDB04461 |
| 2969 | 198.0189 | 198.127 | [M+TMS+H]⁺ | 125.0796 | 125.0953 | 15.7 | C₆H₁₁N₃ | 1-Methylhistamine | HMDB00898 |
| 3018 | 200.2975 | 200.112 | [M+TMS+H]⁺ | 127.0646 | 127.0633 | -1.3 | C₆H₉NO₂ | D-1-Piperideine-2-carboxylic acid | HMDB1084 |
| 3060 | 202.2506 | 202.0905 | [M+TMS+H]⁺, [M+2TMS+H]⁺ | 129.0432 | 129.0426 | -0.6 | C₅H₇NO₃ | Pyroglutamic acid | HMDB00267 |
| 3102 | 204.2037 | 204.1398 | [M+TMS+H]⁺ | 131.0924 | 131.0946 | 2.2 | C₆H₁₃NO₂ | L-Isoleucine | HMDB00172 |
| 3186 | 208.1099 | 208.1152 | [M+TMS+H]⁺ | 135.0679 | 135.4684 | 0.5 | C₈H₉NO | 2-Phenylacetamide | HMDB10715 |
| 3207 | 209.0865 | 209.1359 | [M+TMS+H]⁺ | 136.0886 | 136.0749 | -13.7 | C₆H₈N₄ | Tetrahydropteridine | HMDB1216 |
| 3228 | 210.0630 | 210.1228 | [M+TMS+H]⁺ | 137.0754 | 137.0841 | 8.7 | C₈H₁₁NO | Tyramine | HMDB00306 |
| 3249 | 211.0396 | 211.1304 | *Not Identified* | | | | | | |
| 3270 | 212.0161 | 212.1096 | *Not Identified* | | | | | | |
| 3319 | 214.2947 | 214.1424 | [M+TMS+H]⁺ | 141.0950 | 141.0902 | -4.8 | C₆H₁₁N₃O | L-Histidinol | HMDB03431 |
| 3361 | 216.2478 | 216.1269 | [M+TMS+H]⁺ | 143.0796 | 143.0946 | 15.0 | C₇H₁₃NO₂ | Proline betaine | HMDB04827 |
| 3487 | 222.1071 | 222.1132 | [M+TMS+H]⁺ | 149.0658 | 149.0701 | 4.3 | C₆H₇N₅ | 6-Methyladenine | HMDB02099 |
| 3550 | 225.0368 | 225.111 | [M+TMS+H]⁺ | 152.0636 | 152.0685 | 4.9 | C₅H₁₂O₅ | D-Arabitol | HMDB00568 |
| 3620 | 228.2919 | 228.2617 | *Not Identified* | | | | | | |
| 3641 | 229.2585 | 229.1891 | *Not Identified* | | | | | | |
| 3662 | 230.2450 | 230.153 | *Not Identified* | | | | | | |
| 3704 | 232.1981 | 232.1383 | [M+TMS+H]⁺ | 159.0910 | 159.0895 | -1.5 | C₇H₁₃NO₃ | 2-Methyl- butyrylglycine | HMDB00339 |
| 3767 | 235.1278 | 235.1697 | *Not Identified* | | | | | | |
| 3830 | 238.0574 | 238.1238 | [M+TMS+H]⁺ | 165.0764 | 165.0651 | -11.3 | C₆H₇N₅O | 7-Methylguanine | HMDB00897 |
| 3900 | 241.3126 | 241.1302 | [M+TMS+H]⁺ | 168.0828 | 168.0899 | 7.1 | C₈H₁₂N₂O₂ | Pyridoxamine | HMDB1431 |
| 3921 | 242.2891 | 242.1356 | [M+TMS+H]⁺ | 169.0882 | 169.0851 | -3.1 | C₇H₁₁N₃O₂ | 1-Methylhistidine | HMDBD0001 |
| 3942 | 243.2657 | 243.2024 | *Not Identified* | | | | | | |
| 3963 | 244.2422 | 244.1403 | [M+TMS+H]⁺ | 171.0929 | 171.0895 | -3.4 | C₈H₁₃NO₃ | N-butanoyl-Ihonnoserine lactone | MID36732 |
| 4005 | 246.1953 | 246.1479 | [M+TMS+H]⁺ | 173.1006 | 173.1052 | 4.6 | C₈H₁₅NO₃ | Hexanoylglycine | HMDB00701 |
| 4047 | 248.1484 | 248.1361 | [M+TMS+H]⁺ | 175.0888 | 175.0957 | 6.9 | C₆H₁₃N₃O₃ | Citrulline | HMDB00904 |
| 4089 | 250.1015 | 250.1414 | [M+TMS+H]⁺ | 177.0940 | 177.0790 | -15.0 | C₁₀H₁₁NO₂ | 5-Hydroxytryptophol | HMDB01855 |
| 4131 | 252.0546 | 252.1394 | [M+TMS+H]⁺ | 179.0920 | 179.0946 | 2.6 | C₁₀H₁₃NO₂ | 2(N)-Methyl-norsalsolinol | HMDB01189 |
| 4173 | 254.0077 | 254.1522 | [M+TMS+H]⁺ | 181.1048 | 181.0964 | -8.4 | C₇H₁₁N₅O | 6-methyl-tetrahydropterin | HMDB02249 |
| 4243 | 257.2629 | 257.2311 | [M+TMS+H]⁺ | 184.1838 | 184.1827 | -1.1 | C₁₂H₂₄O | 11-dodecen-1-ol | MID36478 |
| 4264 | 258.2394 | 258.2817 | *Not Identified* | | | | | | |
| 4285 | 259.2160 | 259.1428 | [M+TMS+H]⁺ | 186.0954 | 186.1004 | 5.0 | C₈H₁₄N₂O₃ | Ala Pro | MID23860 |
| 4306 | 260.1925 | 260.1541 | [M+2TMS+H]⁺ | 115.0672 | 115.0633 | -3.9 | C₅H₉NO₂ | Proline | MID29 |
| 4369 | 263.1222 | 263.2296 | *Not Identified* | | | | | | |
| 4390 | 264.0987 | 264.196 | *Not Identified* | | | | | | |
| 4432 | 266.0518 | 266.147 | [M+TMS+H]⁺ | 193.0997 | 193.1103 | 10.6 | C₁₁H₁₅NO₂ | (R)-N-Methylsalsolinol | HMDB03626 |
| 4496 | 268.0284 | 267.267 | *Not Identified* | | | | | | |
| 4474 | 268.0049 | 268.1692 | *Not Identified* | | | | | | |
| 4502 | 269.3070 | 269.1688 | *Not Identified* | | | | | | |
| 4523 | 270.2835 | 270.1698 | [M+2TMS+H]⁺ | 125.0829 | 125.0953 | 12.4 | C₆H₁₁N₃ | 1-Methylhistamine | HMDB00898 |
| 4544 | 271.2601 | 271.1195 | [M+2TMS+H]⁺ | 126.0326 | 126.0429 | 10.3 | C₅H₆N₂O₂ | Thymine | HMDB00262 |
| 4565 | 272.2366 | 272.1781 | *Not Identified* | | | | | | |
| 4607 | 274.1897 | 274.13 | [M+2TMS+H]⁺ | 129.0431 | 129.0426 | -0.5 | C₅H₇NO₃ | Pyroglutamic acid | MID3251 |
| 4691 | 278.0959 | 278.1682 | *Not Identified* | | | | | | |
| 4712 | 279.0725 | 279.1551 | [M+2TMS+H]⁺ | 134.0682 | 134.0579 | -10.3 | C₅H₁₀O₄ | Deoxyribose | HMDB03224 |
| 4733 | 280.0490 | 280.1564 | [M+2TMS+H]⁺ | 135.0695 | 135.0684 | -1.1 | C₈H₉NO | 2-Phenylacetamide | HMDB10715 |
| 4754 | 281.0256 | 281.2894 | *Not Identified* | | | | | | |
| 4775 | 282.0021 | 282.2802 | *Not Identified* | | | | | | |
| 4803 | 283.3042 | 283.2658 | *Not Identified* | | | | | | |
| 4824 | 284.2807 | 284.1606 | [M+2TMS+H]⁺ | 139.0737 | 139.0746 | 0.9 | C₆H₉N₃O | Histidinal | HMDB12234 |
| 4845 | 285.2573 | 285.2806 | *Not Identified* | | | | | | |
| 4908 | 288.1869 | 288.1624 | [M+TMS+H]⁺ | 215.1150 | 215.1157 | 0.7 | C₁₀H₁₇NO₄ | 2-amino-8-oxo-9,10-epoxy-decanoic acid | MID35859 |
| 4992 | 292.0931 | 292.1655 | [M+3TMS+H]⁺ | 75.0391 | 75.0320 | -7.1 | C₂H₅NO₂ | Glycine | HMDB00123 |
| 5013 | 293.0697 | 293.1588 | [M+2TMS+H]⁺ | 148.0719 | 148.0736 | 1.7 | C₆H₁₂O₄ | Mevalonic acid | HMDB00227 |
| 5034 | 294.0462 | 294.1537 | [M+2TMS+H]⁺ | 149.0668 | 149.0510 | -15.8 | C₅H₁₁NO₂S | L-Methionine | HMDB00696 |
| 5055 | 295.0228 | 295.1787 | *Not Identified* | | | | | | |
| 5083 | 296.3248 | 297.2538 | [M+TMS+H]⁺ | 224.2065 | 224.2140 | 7.5 | C₁₅H₂₈O | 10-pentadecenal | MID36604 |
| 5125 | 298.2779 | 298.1833 | [M+2TMS+H]⁺ | 153.0964 | 153.0790 | -17.4 | C₈H₁₁NO₂ | Dopamine | HMDB00073 |
| 5146 | 299.2545 | 299.2597 | [M+TMS+H]⁺ | 226.2124 | 226.1933 | -19.1 | C₁₄H₂₆O₂ | 5-Tetradecenoic acid | HMDB00499 |
| 5167 | 300.2310 | 300.1662 | [M+2TMS+H]⁺ | 155.0793 | 155.0695 | -9.8 | C₆H₉N₃O₂ | L-Histidine | HMDB00177 |
| 5188 | 301.2076 | 301.1874 | [M+TMS+H]⁺ | 228.1401 | 228.1474 | 7.3 | C₁₁H₂₀N₂O₃ | L-isoleucyl-L-proline | HMDB11174 |
| 5209 | 302.1841 | 302.1712 | [M+2TMS+H]⁺ | 157.0843 | 157.0739 | -10.4 | C₇H₁₁NO₃ | 3-Methyl- crotonylglycine | HMDB00459 |
| 5230 | 303.1607 | 303.2969 | *Not Identified* | | | | | | |
| 5251 | 304.1372 | 304.171 | [M+2TMS+H]⁺ | 159.0841 | 159.0895 | 5.4 | C₇H₁₃NO₃ | 2-Methyl- butyrylglycine | HMDB00339 |
| 5293 | 306.0903 | 306.1762 | [M+3TMS+H]⁺ | 89.0498 | 89.0477 | -2.1 | C₃H₇NO₂ | Beta-Alanine | HMDB00056 |
| 5335 | 308.0434 | 308.1673 | [M+2TMS+H]⁺ | 163.0804 | 163.0633 | -17.1 | C₉H₉NO₂ | 3-Methyldioxyindole | HMDB04186 |
| 5356 | 309.0200 | 309.1566 | [M+TMS+H]⁺ | 236.1092 | 236.1017 | -7.5 | C₉H₂₀N₂0S₂ | S-aminomethyl-dihydrolipoamide | HMDB06239 |
| 5447 | 313.2517 | 313.2913 | [M+TMS+H]⁺ | 240.2440 | 240.2453 | 1.3 | C₁₆H₃₂O | 9-hexadecen-1-ot | MID36487 |
| 5489 | 315.2048 | 315.1044 | [M+2TMS+H]⁺ | 170.0175 | 169.9980 | -19.5 | C₃H₇O₆P | D-Glyceraldehyde 3-phosphate | HMDB01112 |
| 5552 | 318.1344 | 318.1817 | [M+2TMS+H]⁺ | 173.0948 | 173.1052 | 10.4 | C₈H₁₅NO₃ | Hexanoylglycine | HMDB00701 |
| 5594 | 320.0875 | 320.1781 | [M+2TMS+H]⁺ | 175.0912 | 175.0957 | 4.5 | C₆H₁₃N₃O₃ | Citrulline | HMDB00904 |
| 5657 | 323.0172 | 323.1745 | *Not Identified* | | | | | | |
| 5685 | 324.3192 | 324.1645 | [M+TMS+H]⁺ | 251.1172 | 251.1018 | -15.4 | C₁₀H₁₃N₅O₃ | Deoxyadenosine | HMDB04101 |
| 5706 | 325.2958 | 325.1855 | [M+2TMS+H]⁺ | 180.0986 | 180.0899 | -8.7 | C₉H₁₂N₂O₂ | 5-Hydroxy-kynurenamine | HMDB04fl76 |
| 5727 | 326.2723 | 326.1599 | [M+2TMS+H]⁺ | 181.0730 | 181.0739 | 0.9 | C₉H₁₁NO₃ | L-Tyrosine | HMDB00158 |
| 5748 | 327.2489 | 327.2764 | [M+TMS+H]⁺ | 254.2291 | 254.2246 | -4.5 | C₁₆H₃₀O₂ | Hypogeic acid | HMDB02186 |
| 5790 | 329.2020 | 329.2859 | [M+TMS+H]⁺ | 256.2386 | 256.2402 | 1.6 | C₁₆H₃₂O₂ | Palmitic acid | HMDB00220 |
| 5832 | 331.1551 | 331.2722 | [M+2TMS+H]⁺ | 258.2249 | 258.2195 | -5.4 | C₁₅H₃₀O₃ | 2-hydroxy-pentadecanoic acid | MID35423 |
| 5853 | 332.1316 | 332.1598 | [M+TMS+H]⁺ | 259.1125 | 259.1168 | 4.3 | C₁₀H₁₇N₃O₅ | Ser Pro Gly | MID33557 |
| 5937 | 336.0378 | 336.235 | *Not Identified* | | | | | | |
| 5986 | 338.3164 | 338.1905 | *Not Identified* | | | | | | |
| 6112 | 344.1757 | 344.3206 | *Not Identified* | | | | | | |
| 6049 | 341.2461 | 341.3034 | *Not Identified* | | | | | | |
| 6133 | 345.1523 | 345.2206 | [M+TMS+H]⁺ | 272.1733 | 272.1776 | 4.3 | C₃₈H₂₄O₂ | Estradiol | HMDB00151 |
| 6154 | 346.1288 | 346.1878 | [M+TMS+H]⁺ | 273.1405 | 273.1325 | -8.0 | C₁₁H₁₉N₃O₅ | Gly Pro Thr | MID22941 |
| 6175 | 347.1054 | 347.2285 | [M+2TMS+H]⁺ | 202.1416 | 202.1430 | 1.4 | C₈H₁₈N₄O₂ | Dimethyl-L-arginine | HMDB01539 |
| 6287 | 352.3136 | 352.2091 | *Not Identified* | | | | | | |
| 6308 | 353.2902 | 353.2908 | [M+TMS+H]⁺ | 280.2435 | 280.2402 | -3.3 | C₁₈H₃₂O₂ | Bovinic acid | HMDB03797 |
| 6350 | 355.2433 | 355.3029 | [M+TMS+H]⁺ | 282.2556 | 282.2559 | 0.3 | C₁₈H₃₄O₂ | Vaccenic acid | HMDB03231 |
| 6392 | 357.1964 | 357.3194 | [M+TMS+H]⁺ | 284.2720 | 284.2715 | -0.5 . | C₁₈H₃₆O₂ | Stearic acid | HMDB00827 |
| 6434 | 359.1495 | 359.3168 | *Not Identified* | | | | | | |
| 6455 | 360.1260 | 360.3305 | [M+TMS+H]⁺ | 287.2832 | 287.2824 | -0.8 | C₁₇H₃₁NO₂ | C17 Sphinganine | MID41558 |
| 6476 | 361.1026 | 361.3344 | *Not Identified* | | | | | | |
| 6539 | 364.0322 | 364.1823 | [M+TMS+H]⁺ | 291.1350 | 291.1327 | -2.3 | C₁₃H₂₅NO₂S₂ | S-(3-Methylbutanoyl)-dihydrolipoamide-E | HMDB06867 |
| 6588 | 366.3108 | 367.3389 | *Not Identified* | | | | | | |
| 6651 | 369.2405 | 369.3507 | [M+TMS+H]⁺ | 296.3034 | 296.3079 | 4.5 | C₂₀H₄₀O | 11Z-eicosen-1-ol | MID36508 |
| 6693 | 371.1936 | 371.3576 | *Not Identified* | | | | | | |
| 6756 | 374.1232 | 374.3349 | [M+TMS+H]⁺ | 301.2876 | 301.2981 | 10.5 | C₁₈H₃₉NO₂ | Sphinganine | HMDB00269 |
| 6798 | 376.0763 | 376.2339 | [M+2TMS+H]⁺ | 231.1470 | 231.1583 | 11.3 | C₁₀H₂₁N₃O₃ | Gamma-Aminobutyryl-lysine | HMDB01959 |
| 6840 | 378.0294 | 378.2119 | [M+3TMS+H]⁺ | 161.0855 | 161.0688 | -16.7 | C₆H₁₁NO₄ | Aminoadipic acid | HMDB00510 |
| 6861 | 379.0060 | 379.1802 | [M+2TMS+H]⁺ | 234.0933 | 234.0852 | -8.1 | C₈H₁₄N₂O₆ | L-beta-aspartyl-L-threonine | HMDB11169 |
| 6952 | 383.2377 | 383.3388 | [M+TMS+H]⁺ | 310.2915 | 310.2872 | -4.3 | C₂₀H₃₈O₂ | 14Z-eicosenoic acid | MID34768 |
| 6994 | 385.1908 | 385.3174 | [M+TMS+H]⁺ | 312.2700 | 312.2664 | -3.6 | C₁₉H₃₆O₃ | 10-oxo-nonadecanoic acid | MID35818 |
| 7036 | 387.1439 | 387.1435 | *Not Identified* | | | | | | |
| 7057 | 388.1204 | 388.3615 | *Not Identified* | | | | | | |
| 7099 | 390.0735 | 390.3692 | *Not Identified* | | | | | | |
| 7120 | 391.0501 | 391.2645 | [M+TMS+H]⁺ | 318.2172 | 318.2195 | 2.3 | C₂₀H₃₀O₃ | 5-HEPE | HMDB05081 |
| 7141 | 392.0266 | 392.229 | [M+3TMS+H]⁺ | 175.1026 | 175.0957 | -6.9 | C₆H₁₃N₃O₃ | Argininic acid | HMDB03148 |
| 7190 | 394.3052 | 394.2083 | [M+3TMS+H]⁺ | 177.0819 | 177.0790 | -2.9 | C₁₀H₁₁NO₂ | 5-Hydroxytryptophol | HMDB1855 |
| 7232 | 396.2583 | 396.2009 | [M+3TMS+H]⁺ | 179.0745 | 179.0794 | 4.9 | C₆H₁₃NO₅ | Fructosamine | HMDB02030 |
| 7253 | 397.2349 | 397.2051 | [M+2TMS+H]⁺ | 180.0787 | 180.0634 | -15.3 | C₆H₁₂O₆ | D-Glucose | HMDB00122 |
| 7295 ' | 399.1880 | 399.3415 | [M+TMS+H]⁺ | 326.2942 | 326.2821 | -12.1 | C₂₀H₃₈O₃ | 19-oxo-eicosanoic acid | MID35822 |
| 7316 | 400.1645 | 400.3961 | *Not Identified* | | | | | | |
| 7337 | 401.1411 | 401.3334 | [M+TMS+H]⁺ | 328.2861 | 328.2977 | 11.6 | C₂₀H₄₀O₃ | 2-hydroxy-eicosanoic acid | MID35451 |
| 7358 | 402.1176 | 402.368 | Not Identified | | | | | | |
| 7379 | 403.0942 | 403.3303 | [M+TMS+H]⁺ | 330.2830 | 330.2770 | -6.0 | C₁₉H₃₈O₄ | MG(0:0/16:0/0:0) | HMDB11533 |
| 7400 | 404.0707 | 404.2066 | [M+TMS+H]⁺ [M+2TMS+H]⁺ | 259.1197 | 259.1168 | -2.9 | C₁₀H₁₇N₃O₅ | Ser Pro Gly | MID22557 |
| 7442 | 406.0238 | 406.2184 | [M+TMS+H]⁺, [M+2TMS+H]⁺ | 261.1315 | 261.1325 | 1.0 | C₁₀H₁₉N₃O₅ | Ser Gly Val | MID23067 |
| 7491 | 408.3024 | 408.2776 | *Not Identified* | | | | | | |
| 7533 | 410.2555 | 410.2265 | [M+TMS+H]⁺ | 337.1792 | 337.1750 | -4.2 | C₁₅H₂₃N₅O₄ | Kyotorphin | HMDB05768 |
| 7596 | 413.1852 | 413.3419 | [M+TMS+H]⁺ | 340.2946 | 340.2977 | 3.1 | C₂₁H₄₀O₃ | 2-oxo-heneicosanoic acid | MID35825 |
| 7659 | 416.1148 | 416.2254 | [M+2TMS+H]⁺ | 271.1385 | 271.1406 | 2.1 | C₁₁H₁₉N₄O₄ | 2-(3-Carboxy-3-(methylammonio)propyl)-L-histidine | HMDB11654 |
| 7701 | 418.0679 | 418.3526 | [M+TMS+H]⁺ | 345.3053 | 345.3032 | -2.1 | C₂₃H₃₉NO | N-propyl arachidonoyl amine | MID36681 |
| 7722 | 419.0445 | 419.2884 | [M+3TMS+H]⁺ | 202.1620 | 202.1430 | -19.0 | C₈H₁₈N₄O₂ | Dimethyl-L-arginine | HMDB01539 |
| 7792 | 422.2996 | 422.2203 | [M+2TMS+H]⁺ | 277.1334 | 277.1175 | -15.9 | C₁₂H₁₅N₅O₃ | Queuine | HMDB01495 |
| 7813 | 423.2762 | 423.2556 | [M+TMS+H]⁺ | 350.2083 | 350.2093 | 1.0 | C₂₀H₃₀O₅ | 8-iso-15-keto-PGE2 | HMDB02341 |
| 7834 | 424.2527 | 424.2178 | [M+3TMS+H]⁺ | 207.0914 | 207.0752 | -16.2 | C₈H₁₇NOS₂ | Dihydrolipoamide | HMDB00985 |
| 7855 | 425.2293 | 425.3162 | [M+TMS+H]⁺ | 352.2689 | 352.2614 | -7.5 | C₂₁H₃₆O₄ | MG(0:0/18:3(6Z,9Z12Z)/0:0) | HMDB11539 |
| 7918 | 428.1589 | 428.3949 | [M+TMS+H]⁺ | 355.3476 | 355.3450 | -2.6 | C₂₂H₄₅N0₂ | N-(2-hydroxyethyl)icosanamide | MID3723 |
| 7939 | 429.1355 | 429.3694 | [M+TMS+H]⁺ | 356.3221 | 356.3290 | 6.9 | C₂₂H₄₄O₃ | 2-hydroxy behenic | MID35454 |
| 7981 | 431.0886 | 431.3533 | [M+TMS+H]⁺, [M+2TMS+H]⁺ | 358.3060 | 358.3083 | 2.3 | C₂₁H₄₂O₄ | MG(18:0/0:0/0:0) | HMDB11131 |
| 8072 | 435.3203 | 435.3824 | [M+TMS+H]⁺ | 362.3351 | 362.3185 | -16.6 | C₂₄H₄₂O₂ | 5beta-Cholane-3alpha,24-diol | MID42895 |
| 8114 | 437.2734 | 437.3135 | [M+2TMS+H]⁺ | 292.2266 | 292.2402 | 13.6 | C₁₉H₃₂O₂ | 3b,17b-Dihydroxyetiocholane | HMDB00369 |
| 8163 | 439.5520 | 439.2287 | [M+TMS+H]⁺ | 366.1814 | 366.1652 | -16.2 | C₃₅H₂₂N₆O₅ | Pro His Asn | MID23382 |
| 8240 | 443.1327 | 443.2628 | [M+TMS+H]⁺ | 370.2155 | 370.2329 | 17.4 | C₁₆H₃₀N₆O₄ | Val Arg Pro | MID23376 |
| 8282 | 445.0858 | 445.2388 | [M+3TMS+H]⁺ | 228.1124 | 228.1110 | -1.4 | C₁₀H₁₆N₂O₄ | Prolylhydroxyproline | HMDB06695 |
| 8324 | 447.0389 | 447.3446 | [M+2TMS+H]⁺ | 302.2577 | 302.2457 | -12.0 | C₁₇H₃₄O₄ | MG(0:0/14:0/0:0) | HMDB11530 |
| 8345 | 448.4154 | 448.3935 | *Not Identified* | | | | | | |
| 8394 | 450.2940 | 450.2371 | [M+3TMS+H]⁺ | 233.1107 | 233.0916 | -19.1 | C₁₀H₁₁N₅O₂ | Dihydroxycoprostanoic acid | HMDB01974 |
| 8415 | 41.2706 | 451.2253 | [M+3TMS+H]⁺ | 234.0989 | 234.1004 | 1.5 | C₁₂H₁₄N₂O₃ | 5-Methoxytryptophan | HMDB02339 |
| 8604 | 460.0595 | 460.4028 | [M+TMS+H]⁺ | 387.3555 | 387.3501 | -5.4 | C₂₆H₄₅NO | 25-Azacholesterol | HMDB01028 |
| 8695 | 464.2912 | 464.2809 | [M+3TMS+H]⁺ | 247.1545 | 247.1532 | -1.3 | C₁₀H₂₁N₃O₄ | Lys Thr | MID23652 |
| 8779 | 468.1974 | 468.23 77 | [M+2TMS+H]⁺ | 251.1113 | 251.1018 | -9.5 | C₁₀H₅₃N₅O₃ | Deoxyadenosine | HMDB00101 |
| 8842 | 471.1271 | 471.3956 | [M+TMS+H]⁺ | 398.3483 | 398.3548 | 6.5 | C₂₈H₄₆O | 4a-Methylzymosterol | HMDB01217 |
| 8884 | 473.0802 | 473.3847 | [M+TMS+H]⁺ | 400.3374 | 400.3341 | -3.3 | C₂₇H₄₄O₂ | 7-Ketocholesterol | HMDB10501 |
| 8926 | 475.0333 | 475.3655 | [M+2TMS+H]⁺ | 330.2786 | 330.2770 | -1.6 | C₁₉H₃₈O₄ | MG(0:0/16:0/0:0) | HMDB11533 |
| 8996 | 478.2884 | 478.2522 | [M+3TMS+H]⁺ | 261.1258 | 261.1325 | 6.7 | C₁₀H₁₉N₃O₅ | Ser Gly Val | MID23067 |
| 9080 | 482.1946 | 482.26 | [M+2TMS+H]⁺ | 337.1731 | 337.1750 | 1.9 | C₁₅H₂₃N₅O₄ | Kyotorphin | HMDB05768 |
| 9143 | 485.1243 | 485.3228 | *Not Identified* | | | | | | |
| 9185 | 487.0774 | 487.2499 | [M+TMS+H]⁺ | 414.2026 | 414.2049 | 2.3 | C₁₇H₃₀N₆O₄S₁ | Lys Met His | MID23058 |
| 9248 | 490.0070 | 490.2768 | [M+TMS+H]⁺, [M+2TMS+H]⁺ | 345.1899 | 345.1900 | 0.1 | C₁₅H₂₇N₃O₆ | Val Glu Val | MID22736 |
| 9297 | 492.2856 | 492.2743 | [M+3TMS+H]⁺ | 275.1479 | 275.1481 | 0.2 | C₁₁H₂₁N₃O₅ | Epsilon-(gamma-Glutamyl)-lysine | HMDB03869 |
| 9339 | 494.2387 | 494.2575 | [M+3TMS+H]⁺ | 277.1311 | 277.1175 | -13.6 | C₁₂H₁₅N₅O₃ | Queuine | HMDB01495 |
| 9381 | 496.1918 | 496.2643 | [M+2TMS+H]⁺ | 351.1774 | 351.1794 | 2.0 | C₁₇H₂₅N₃O₅ | Val Tyr Ala | MID22964 |
| 9465 | 500.0980 | 500.4358 | [M+2TMS+H]⁺ | 355.3489 | 355.3450 | -3.9 | C₂₂H₄₅NO₂ | N-(2-hydroxyethyl) icosanamide | MID3723 |
| 9507 | 502.0511 | 502.4386 | *Not Identified* | | | | | | |
| 9528 | 503.0277 | 503.39 | [M+TMS+H]⁺ | 430.3426 | 430.3447 | 2.1 | C₂₈H₄₆O₃ | 1α-hydroxy-25-methoxyvitamin D3 | MID42264 |
| 9619 | 507.2594 | 507.5022 | *Not Identified* | | | | | | |
| 9640 | 508.2359 | 508.2806 | [M+3TMS+H]⁺ | 291.1542 | 291.1430 | -11.2 | C₁₁H₂₁N₃O₆ | Ala Thr Thr | MID22878 |
| 9682 | 510.1890 | 510.2765 | [M+2TMS+H]⁺ | 365.1896 | 365.1951 | 5.5 | C₁₈H₂₇N₃O₅ | Ser Phe Ile | MID22773 |
| 9808 | 516.0483 | 516.45 | *Not Identified* | | | | | | |
| 9829 | 517.0249 | 518.2985 | [M+2TMS+H]⁺ [M+3TMS+H]⁺ | 301.1721 | 301.1638 | -8.3 | C₁₃H₂₃N₃O₅ | Pro Ser Val | MID23420 |
| 9850 | 518.0014 | 518.4984 | *Not Identified* | | | | | | |
| 9899 | 520.2800 | 520.5065 | *Not Identified* | | | | | | |
| 9941 | 522.2331 | 522.2826 | [M+TMS+H]⁺ | 449.2352 | 449.2387 | 3.5 | C₂₀H₃₁N₇O₅ | Gln Arg Phe | MID22049 |
| 10025 | 526.1393 | 526.2566 | [M+3TMS+H]⁺ | 309.1302 | 309.1325 | 2.3 | C₁₄H₁₉N₅O₅ | Tyr Gly Ala | MID23104 |
| 10109 | 530.0455 | 530.4306 | [M+TMS+H]⁺ | 457.3833 | 457.3920 | 8.7 | C₃₀H₅₁NO₂ | 3'-O-Aminopropyl-25-hydroxyvitamin D3 | MID42610 |
| 10130 | 531.0221 | 531.2916 | [M+TMS+H]⁺, [M+2TMS+H]⁺ | 458.2442 | 458.2338 | -10.4 | C₂₃H₃₈O₇S | 3-Sulfodeoxycholic acid | HMDB02Sfl4 |
| 10158 | 532.3241 | 532.3106 | [M+TMS+H]⁺ | 459.2632 | 459.2554 | -7.8 | C₁₇H₃₃N₉O₆ | Arg Arg Glu | MID23106 |
| 10200 | 534.2772 | 534.5247 | *Not Identified* | | | | | | |
| 10326 | 540.1365 | 540.2642 | [M+2TMS+H]⁺ [M+3TMS+H]⁺ | 323.1378 | 323.1481 | 10.3 | C₁₅H₂₁N₃O₅ | Tyr Ala Ala | MID22475 |
| 10501 | 548.2744 | 548.2722 | [M+TMS+H]⁺ | 475.2248 | 475.2179 | -6.9 | C₂₁H₂₉N₇O₆ | Trp Asp Arg | MID22780 |
| 10543 | 550.2275 | 550.2827 | [M+2TMS+H]⁺, [M+2TMS+H]⁺ | 333.1563 | 333.1536 | -2.7 | C₁₃H₂₃N₃O₇ | Asp Val Thr | MID23209 |
| 10732 _{:} | 559.0165 | 559.2889 | [M+2TMS+H]⁺ | 414.2020 | 414.2049 | 2.9 | C₁₇H₃₀N₆O₄S₁ | Lys Met His | MID23058 |
| 10886 | 566.1778 | 566.2841 | [M+2TMS+H]⁺ , [M+3TMS+H]⁺ | 349.1577 | 349.1485 | -9.2 | C₁₃H₂₃N₃O₈ | Glu Thr Thr | MID21841 |
| 10928 | 568.1309 | 568.293 | [M+TMS+H]⁺ | 495.2456 | 495.2482 | 2.6 | C₂₆H₃₃N₅O₅ | Trp Lys Tyr | MID21781 |
| 11103 | 576.2688 | 576.4439 | [M+TMS+H]⁺ | 503.3965 | 503.4008 | 4.3 | C₂₈H₅₇NO₄S | 2-hexacosanamido-ethanesulfonic acid | MID3740 |
| 11145 | 578.2219 | 578.543 | *Not Identified* | | | | | | |
| 11229 | 582.1281 | 582.3106 | [M+2TMS+H]⁺ [M+3TMS+H]⁺ | 365.1842 | 365.1951 | 10.9 | C₁₈H₂₇N₃O₅ | Ser Phe Ile | MID22773 |
| 11313 | 586.0343 | 586.3148 | [M+TMS+H]⁺ | 513.2674 | 513.2760 | 8.6 | C₂₆H₄₃NO₇S | Sulfolithocholylglycine | HMDB02639 |
| 11446 | 592.2191 | 592.5467 | | | | *Not* | *Identified* | | |
| 11572 | 598.0784 | 598.295 | [M+3TMS+H]⁺ | 381.1686 | 381.1536 | -15.0 | C₁₇H₂₃N₃O₇ | Phe Ser Glu | MID23135 |
| 11663 | 602.3101 | 602.3189 | [M+TMS+H]⁺, [M+2TMS+H]⁺ | 529.2716 | 529.2709 | -0.7 | C₂₆H₄₃NO₈S | N-[(3a,5b,7b)-7-hydroxy-24-oxo-3 -(sulfooxy)chalau-24-yl]-Glycine | HMDB02409 |
| 11684 | 603.2867 | 603.3334 | [M+2TMS+H]⁺ | 458.2465 | 458.2390 | -7.5 | C₂₁H₃₀N₈o₄ | Arg Phe His | MID21269 |
| 11705 | 604.2632 | 604.3471 | [M+2TMS+H]⁺, [M+3TMS+H]⁺ | 387.2207 | 387.2230 | 2.3 | C₁₇H₃₃N₉O₆ | Arg Arg Glu | MID23106 |
| 11831 | 610.1225 | 610.3368 | [M+2TMS+H]⁺ [M+3TMS+H]⁺ | 393.2104 | 393.2264 | 16.0 | C₂₀H₃₁N₃O₅ | He Val Tyr | MID23584 |
| 11873 | 612.0756 | 612.2989 | [M+3TMS+H]⁺ | 395.1725 | 395.1693 | -3.2 | C₁₈H₂₅N₃O₇ | Thr Glu Phe | MID23502 |
| 12027 | 619.237 | 619.3249 | [M+TMS+H]⁺ | 546.2776 | 546.2703 | -7.3 | C₂₈H₃₄N₈O₄ | Arg Trp Trp | MID19915 |
| 12048 | 620.2135 | 620.3034 | [M+2TMS+H]⁺, [M+3TMS+H]⁺ | 403.1815 | 403.1770 | -4.5 | C₁₄H₂₅N₇O₇ | Asn Arg Asp | MID22139 |
| 12216 | 628.0259 | 628.3128 | [M+TMS+H]⁺ | 555.2693 | 555.2654 | -3.9 | C₂₈H₃₇N₅O₇ | Leucine Enkephalin | MID24069 |
| 12265 | 630.3045 _{'} | 630.3436 | *Not Identified* | | | | | | |
| 12307 | 632.2576 | 632.371 | [M+3TMS+H]⁺ | 415.2446 | 415.2543 | 9.7 | C₁₇H₃₃N₇O₅ | Ile Arg Gln | MID22784 |
| 12391 | 636.1638 | 636.3449 | [M+2TMS+H]⁺ | 419.2185 | 419.2169 | -1.6 | C₂₀H₂₉N₅O₅ | Trp Ser Lys | MID22695 |
| 12433 | 638.1169 | 638.3212 | [M+3TMS+H]⁺ | 421.1961 | 421.1948 | -1.3 | C₁₉H₂₇N₅O₆ | Gin Phe Gin | MID22749 |
| 12475 | 640.07 | 640.328 | [M+2TMS+H]⁺ [M+2TMS+H]⁺ | 423.2016 | 423.2006 | -1.0 | C₂₀H₂₉N₃O₇ | Tyr Ile Glu | MID22318 |
| 12650 | 648.2079 | 648.3302 | [M+2TMS+H]⁺ [M+3TMS+H]⁺ | 431.2038 | 431.2128 | 9.0 | C₁₆H₂₉N₇O₇ | Gln Glu Arg | MID21914 |
| 12692 | 650.161 | 650.3447 | [M+3TMS+H]⁺ | 433.2183 | 433.2220 | 3.7 | C₁₅H₃₁N₉O₄S₁ | Arg Cys Arg | MID21431 |
| 12776 | 654.0672 | 654.3428 | [M+3TMS+H]⁺ | 437.2164 | 437.2274 | 11.0 | C₂₀H₃₁N₅O₆ | Tyr Lys Gin | MID22135 |
| 12909 | 660.252 | 660.3563 | [M+2TMS+H]⁺ | 515.2694 | 515.2917 | 22.3 | C₂₆H₄₅NO₇S | Taurocholic Acid | MID34542 |
| 12993 | 664.1582 | 664.4789 | *Not Identified* | | | | | | |
| 13119 | 670.0175 | 670.3398 | *Not Identified* | | | | | | |
| 13210 | 674.2492 | 674.3549 | [M+2TMS+H]⁺ | 529.2680 | 529.2709 | 2.9 | C₂₆H₄₃NO₈S | N-[(3a,5b,7b)-7-hydroxy-24-oxo-3-(sulfooxy)cholan-24-yl]-Glycine | MID6670 |
| 13469 | 686.2933 | 686.3726 | [M+3TMS+H]⁺ | 469.2462 | 469.2438 | -2.4 | C₂₃H₃₁N₇O₄ | Lys His Trp | MID22014 |
| 13511 | 688.2464 | 688.352 | *Not Identified* | | | | | | |
| 13574 | 691.1761 | 691.3389 | [M+2TMS+H]⁺, [M+3TMS+H]⁺ | 474.2125 | 474.2339 | 21.4 | C₂₁H₃₀N₈O₅ | His Tyr Arg | MID22969 |
| 13749 | 699.314 | 699.3891 | [M+3TMS+H]⁺ | 482.2627 | 482.2516 | -11.1 | C₂₅H₃₈O₉ | 11-beta-hydroxy-androsterone-3-glucuronide | HMDB10351 |
| 13770 | 700.2905 | 700.323 | *Not Identified* | | | | | | |
| 13812 | 702.2436 | 702.3626 | *Not Identified* | | | | | | |
| 14071 | 714.2877 | 714.3688 | [M+3TMS+H]⁺ | 497.2424 | 497.2499 | 7.5 | C₂₃H₃₁N₉O₄ | Arg His Trp | MID20604 |

**Table 25. Identification of elemental formulae and metabolites matches from features used by the L1SVM model.**

| **Feature Index In L1SVM Model** | **Feature *mlz* in Model** | **Closest Peak Matched *(m*/*z)*** | **Ion Type** | **Experimen. MW (Da)** | **Theoretic. MW (Da)** | **Δm (mmu)** | **Estimated Elemental Formula** | **Possible Match in Metabolome Databases** | **Source** |
|---|---|---|---|---|---|---|---|---|---|
| 3011 | 199.9720 | 200.1189 | [M+TMS+H]⁺ | 127.0716 | 127.0633 | 8.3 | C₆H₉NO₂ | D-1-Piperideine-2-carboxylic acid | HMDB01084 |
| 3197 | 208.6214 | 208.1158 | [M+TMS+H]⁺ | 135.0685 | 135.0684 | 0.1 | C₈H₉NO | 2-Phenylacetamide | HMDB0715 |
| 5546 | 317.8554 | 315.1034 | [M+2TMS+H]⁺ | 170.0165 | 169.9980 | 18.5 | C₃H₇O₆P | D-Glyceraldehyde 3-phosphate | HMDB01112 |
| 8438 | 452.3401 | 451.2253 | [M+3TMS+H]⁺ | 234.0989 | 234.1004 | -1.5 | C₁₂H₁₄N₂O₃ | 5-Methoxytryptophan | HMDB02339 |
| 9476 | 500.6095 | 500.4358 | [M+2TMS+H]⁺ | 355.3489 | 355.3450 | 3.9 | C₂₂H₄₅NO₂ | N-(2-hydroxyethyl) icosanamide | MID3723 |
| 9675 | 509.8635 | 508.2806 | [M+2TMS+H]⁺ | 363.1937 | 363.1906 | 3.1 | C₁₇H₂₅N₅O₄ | Isopentenyladenine -9-N-glucoside | HMDB12240 |
| 10613 | 553.4827 | 550.2862 | [M+3TMS+H]⁺ | 333.1598 | 333.1536 | 6.2 | C₁₃H₂₃N₃O₇ | Asp Val Thr | MID23209 |
| 12083 | 621.8411 | 520.5821 | *Not Identified* | | | | | | |
| 13411 | 683.5962 | 683.4615 | [M+3TMS+H]⁺ | 466.3351 | 466.3535 | -18.4 | C₂₃H₅₁N₂O₅P | LysoSM(d18:0) | HMDB12082 |
| 13571 | 691.0366 | 691.3587 | [M+3TMS+H]⁺ | 474.2323 | 474.2339 | -1.6 | C₂₁H₃₀N₈O₅ | His Tyr Arg | MID22969 |
| 14335 | 726.5643 | 726.3855 | *Not Identified* | | | | | | |
| 15640 | 787.2499 | 786.3686 | *Not Identified* | | | | | | |
| 15641 | 787.2964 | | | | | | | | |
| 15642 | 787.3429 | | | | | | | | |

**Table 26. Identification of elemental formulae and metabolites matches from features used by the SVMRFE_NL model.**

| **Feature Index In L1SVM Model** | **Feature *m*/*z* in Model** | **Closest Peak Matched *(m*/*z)*** | **Ion Type** | **Experimen. MW** (**Da**) | **Theoretic MW (Da)** | **Δm (mmu)** | **Estimated Elemental Formula** | **Possible Match in Metabolome Databases** | **Source** |
|---|---|---|---|---|---|---|---|---|---|
| 5546 | 317.8554 | 315.1034 | [M+2TMS+H]⁺ | 170.0165 | 169.9980 | 18.5 | C₃H₇O₆P | D-Glyceraldehyde 3-phosphate | HMDB01112 |
| 8438 | 452.3401 | 451.2253 | [M+3TMS+H]⁺ | 234.0989 | 234.1004 | -1.5 | C₁₂H₁₄N₂O₃ | 5-Methoxytryptophan | HMDB02339 |
| 9675 | 509.8635 | 508.2806 | [M+2TMS+H]⁺ | 363.1937 | 363.1906 | 3.1 | C₁₇H₂₅N₅O₄ | Isopentenyladenine -9-N-glucoside | HMDB12240 |
| 10613 | 553.4827 | 550.2862 | [M+3TMS+H]⁺ | 333.1598 | 333.1536 | 6.2 | C₁₃H₂₃N₃O₇ | Asp Val Thr | MID23209 |
| 10614 | 553.5292 | 553.5526 | *Not Identified* | | | | | | |
| 12388 | 636.0243 | 636.3296 | [M+3TMS+H]⁺ | 419.2032 | 419.1951 | 8.1 | C₁₅H₂₉N₇O₅S₁ | Asn Met Arg | MID23124 |
| 12389 | 636.0708 | 636.5844 | [M+TMS+H]⁺ | 563.5371 | 563.5278 | 9.3 | C₃₆H₆₉NO₃ | Ceramide (d18:1/9Z-18:1) | HMDB04948 |
| 13069 | 667.6924 | 667.3536 | *Not Identified* | | | | | | |
| 13571 | 691.0366 | 691.3587 | [M+3TMS+H]⁺ | 474.2323 | 474.2339 | 1.6 | C₂₁H₃₀N₈O₅ | His Tyr Arg | MID22969 |
| 15640 | 787.2499 | 786.3686 | *Not Identified* | | | | | | |
| 15641 | 787.2964 | | | | | | | | |
| 15642 | 787.3429 | | | | | | | | |

There is general consensus among the ovarian cancer community that to be of clinical significance, a screening test for ovarian cancer in the general population must have a minimum positive predictive value (PPV) of ∼10% (Schwartz and Taylor, Ann. Med., 27:519-528 (1995)). Because the prevalence of ovarian cancer in the general population is low (∼0.04%), the required specificity of any potential screening test must be ≥ 99%. The results presented here suggest the potential of this method as an ovarian cancer diagnostic of significant clinical value.

## Claims

1. A computer-implemented method for selecting a subject for treatment of ovarian cancer comprising:
(i) inputting expression data of a panel of serum metabolic biomarkers in a serum sample obtained from the subject; and
(ii) determining whether expression of the metabolic biomarkers in the serum sample obtained from the subject is indicative of ovarian cancer using a computer system programmed with a trained machine learning classifier for distinguishing subjects with ovarian cancer and without ovarian cancer; wherein the machine learning classifier has been trained using expression data of a panel of serum metabolic biomarkers obtained from patients having ovarian cancer and from control subjects that do not have ovarian cancer; and
(iii) selecting the subject wherein the expression data of the panel of serum metabolic biomarkers in the serum sample obtained from the subject is correlated by the computer system to be indicative of ovarian cancer, and wherein the diagnostic accuracy is at least 80%, and wherein the panel of serum metabolic biomarkers comprises each of D-1-Piperideine-2-carboxylic acid, 2-Phenylacetamide, D-Glyceraldehyde 3-phosphate, 5-Methoxytryptophan, N-(2-hydroxyethyl) icosanamide, Isopentenyladenine-9-N-glucoside, Asp-Val-Thr, LysoSM(d18:0) and His-Tyr-Arg.

2. The method of claim 1, wherein the expression of data of the panel of serum metabolic biomarkers is determined using a mass spectrometry method.

3. The method of claim 2, wherein the mass spectrometry method is direct analysis in real time (DART) mass spectrometry.

4. The method of claim 1 wherein the trained machine learning classifier is a support vector machine (SVM).

5. A computer program adapted to perform a method according to claim 1 when executed on a computer comprising:
(i) a means adapted for receiving expression data of the metabolic biomarkers recited in claim 1;
(ii) a module adapted for performing the determination recited in claim 1;
(iii) a means for indicating whether the subject is selected as recited in claim 1.

## Patentansprüche

1. Computerimplementiertes Verfahren zur Auswahl eines Subjekts zur Behandlung von Eierstockkrebs, umfassend:
(i) Eingeben von Expressionsdaten einer Gruppe von Serum-Stoffwechsel-Biomarkern in einer aus dem Subjekt erhaltenen Serumprobe; und
(ii) Bestimmen dessen, ob Expression der Stoffwechsel-Biomarker in der aus dem Subjekt erhaltenen Serumprobe für Eierstockkrebs indikativ ist, mithilfe eines mit einem trainierten Maschinen-Lern-Klassifikator zum Unterscheiden der Subjekte mit Eierstockkrebs und ohne Eierstockkrebs programmierten Computersystems; worin der Maschinen-Lern-Klassifikator mithilfe von Expressionsdaten einer Gruppe von Serum-Stoffwechsel-Biomarkern trainiert wurde, die aus Patientinnen mit Eierstockkrebs und aus Kontrollsubjekten, die keinen Eierstockkrebs haben, erhalten wurden; und
(iii) Auswählen des Subjekts, worin die Expressionsdaten der Gruppe von Serum-Stoffwechsel-Biomarkern in der aus dem Subjekt erhaltenen Serumprobe vom Computersystem als für Eierstockkrebs indikativ korreliert werden, und worin die diagnostische Genauigkeit mindestens 80 % beträgt, und worin die Gruppe von Serum-Stoffwechsel-Biomarkern jede/jedes von D-1-Piperidin-2-Carboxylsäure, 2-Phenylacetamid, D-Glyceraldehyd-3-phosphat, 5-Methoxytryptophan, N-(2-Hydroxyethyl)icosanamid, Isopentenyladenin-9-N-glucosid, Asp-Val-Thr, LysoSM(d18:0) und His-Tyr-Arg umfasst.

2. Verfahren nach Anspruch 1, worin die Expression von Daten der Gruppe von Serum-Stoffwechsel-Biomarkern mithilfe eines Massenspektrometrieverfahrens bestimmt wird.

3. Verfahren nach Anspruch 2, worin das Massenspektrometrieverfahren die DART-(Direktanalyse in Echtzeit) Massenspektrometrie ist.

4. Verfahren nach Anspruch 1, worin der trainierte Maschinen-Lern-Klassifikator eine Stützvektormaschine (SVM) ist.

5. Computerprogramm, das dafür ausgelegt ist, ein Verfahren nach Anspruch 1 bei Ausführung auf einem Computer durchzuführen, umfassend:
(i) ein Mittel, das dafür ausgelegt ist, Expressionsdaten der Stoffwechsel-Biomarker gemäß Anspruch 1 entgegenzunehmen;
(ii) ein Modul, das dafür ausgelegt ist, die Bestimmung gemäß Anspruch 1 durchzuführen;
(iii) ein Mittel zum Anzeigen dessen, ob das Subjekt gemäß Anspruch 1 ausgewählt ist.

## Revendications

1. Procédé mis en oeuvre par ordinateur permettant de sélectionner un sujet pour un traitement d'un cancer de l'ovaire, ledit procédé consistant à :
(i) saisir des données d'expression d'un panel de biomarqueurs métaboliques de sérum dans un échantillon de sérum obtenu chez le sujet; et
(ii) déterminer si l'expression des biomarqueurs métaboliques dans l'échantillon de sérum obtenu chez le sujet indique un cancer de l'ovaire au moyen d'un système informatique programmé avec un classificateur d'apprentissage automatique entraîné pour distinguer des sujets atteints d'un cancer de l'ovaire et des sujets non atteints d'un cancer de l'ovaire; dans lequel le classificateur d'apprentissage automatique a été entraîné au moyen de données d'expression d'un panel de biomarqueurs métaboliques de sérum obtenus de patientes atteintes d'un cancer de l'ovaire et de sujets témoins non atteints d'un cancer de l'ovaire; et
(iii) sélectionner le sujet, les données d'expression du panel de biomarqueurs métaboliques de sérum dans l'échantillon de sérum obtenu chez le sujet étant corrélées par le système informatique pour indiquer un cancer de l'ovaire, et la précision du diagnostic étant d'au moins 80 %, et le panel de biomarqueurs métaboliques de sérum comprenant de l'acide D-1-pipéridéine-2-carboxylique, du 2-phénylacétamide, du D-glycéraldéhyde-3-phosphate, du 5-méthoxytryptophane, du N-(2-hydroxyéthyl)icosanamide, de l'isopentényladénine-9-N-glucoside, de l'Asp-Val-Thr, du LysoSM(d18:0) et du His-Tyr-Arg.

2. Procédé selon la revendication 1, dans lequel l'expression de données du panel de biomarqueurs métaboliques de sérum est déterminée au moyen d'un procédé de spectrométrie de masse.

3. Procédé selon la revendication 2, dans lequel le procédé de spectrométrie de masse est une spectrométrie de masse par analyse directe en temps réel (DART).

4. Procédé selon la revendication 1, dans lequel le classificateur d'apprentissage machine entraîné est une machine à vecteur de support (SVM).

5. Programme informatique conçu pour effectuer un procédé selon la revendication 1 lorsqu'il est exécuté sur un ordinateur comprenant :
(i) un moyen conçu pour recevoir des données d'expression des biomarqueurs métaboliques comme énoncé dans la revendication 1;
(ii) un module conçu pour effectuer la détermination comme énoncé dans la revendication 1;
(iii) un moyen permettant d'indiquer si le sujet est sélectionné comme énoncé dans la revendication 1.
